# EUROPÄISCHE PATENTSCHRIFT

(11) **EP 3 898 612 B1**
(45) Veröffentlichungstag und Bekanntmachung des Hinweises auf die Patenterteilung: **02.08.2023**
(21) Anmeldenummer: 19817301.5
(22) Anmeldetag: 11.12.2019
(51) Int. Cl.: C07D 401/12, C07D 413/12, A01N 43/56, A01N 43/80

(54) **SUBSTITUIERTE PYRIDINYLOXYBENZOLE SOWIE DEREN SALZE UND IHRE VERWENDUNG ALS HERBIZIDE WIRKSTOFFE**
SUBSTITUTED PYRIDINYLOXYBENZENES, THEIR SALTS AND USE OF SAID COMPOUNDS AS HERBICIDAL AGENTS
PYRIDINYLOXY-BENZÈNES SUBSTITUÉES AINSI QUE LEURS SELS ET LEUR UTILISATION EN TANT QUE SUBSTANCES ACTIVES HERBICIDES

(30) Priorität: 18.12.2018 EP 18213444
(43) Veröffentlichungstag der Anmeldung: 27.10.2021
(73) Patentinhaber: Bayer Aktiengesellschaft, 51373 Leverkusen (DE)
(72) Erfinder: MCLEOD, Michael, Charles, 60594 Frankfurt (DE); BRAUN, Ralf, 76857 Ramberg (DE); BARBER, David, Michael, 60486 Frankfurt am Main (DE); JAKOBI, Harald, 60598 Frankfurt (DE); REY, Jullien, 68510 Sierentz (FR); SCHMUTZLER, Dirk, 65795 Hattersheim (DE); MACHETTIRA, Anu, Bheemaiah, 60326 Frankfurt am Main (DE); ASMUS, Elisabeth, 63768 Hösbach (DE); ROSINGER, Christopher, Hugh, 65719 Hofheim (DE); GATZWEILER, Elmar, 61231 Bad Nauheim (DE); MANOS-TURVEY, Alexandra, 53721 Siegburg (DE)
(74) Vertreter: BIP Patents
(86) Internationale Anmeldenummer: PCT/EP2019/084671
(87) Internationale Veröffentlichungsnummer: WO 2020/126746

(56) Entgegenhaltungen:
- WO-A1-2020/094524
- JP-A- S61 236 766
- US-A1- 2016 333 000
- DATABASE PubChem Compound [Online] 11. Februar 2016 (2016-02-11), XP002790523, Database accession no. 117073802
- KOYANAGI T ET AL: "BIOISOSTERISM IN AGROCHEMICALS", WATER-SOLUBLE POLYMERS: SYNTHESIS, SOLUTION PROPERTIES AND APPLICATIONS, AMERICAN CHEMICAL SOCIETY, WASHINGTON, DC, US, Bd. 584, 1. Januar 1995 (1995-01-01), Seiten 15-24, XP000578692, ISBN: 978-0-541-23408-9

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte Pyridinyloxybenzole sowie deren Salze, Verfahren zu ihrer Herstellung und ihre Verwendung als Herbizide.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

In JP 61236766 sind verschiedene Pyridinyloxybenzole als Herbizide beschrieben, die in der 2-Position des Benzols einen Benzolring oder einen verbrückten Benzolring tragen. XP-002790523 beschreibt ein spezifisch substituiertes Pyridinyloxyphenylpyridin (2-chloro-4-(2-pyridin-2-yloxyphenyl)pyridin), für das an dieser Stelle aber keinerlei Funktions-/Wirkungsbeziehungen genannt werden. Daneben werden in den Schriften WO 1994/017059, WO 2015/089003, WO 2015/108779 (US2016/333000), WO 2016/010731, WO 2016/196606, WO 2017/011288 verschiedene Pyrimidinyloxybenzole als Herbizide beschrieben. Bestimmte Pyridinyloxybenzole und deren Salze, die in der 2-Position des Benzols einen Heteroarylring tragen und eine herbizide Wirkung aufweisen, sind dagegen noch nicht beschrieben.

Überraschenderweise wurde nun gefunden, dass ausgewählte substituierte Pyridinyloxybenzole und/oder deren Salze, die an der 2-Position anstelle des in JP61236766 beschriebenen Benzolrests einen Heteroarylring tragen, als herbizide Wirkstoffe besonders gut geeignet sind und daneben, v.a. in Bezug auf vergleichbare Pyrimidinyloxybenzole (u.a. bekannt aus US2016/333000), auch noch eine deutlich verbesserte Kulturpflanzenverträglichkeit aufweisen.

Gegenstand der vorliegenden Erfindung sind damit substituierte Pyridinyloxybenzole der allgemeinen Formel (I) oder deren Salze 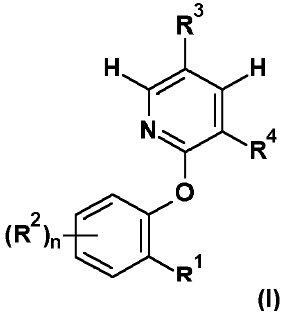 worin
- R¹: für ein gegebenenfalls substituiertes Pyridin oder Pyrimidin steht, das optional mit bis zu 4 Substituenten, unabhängig voneinander ausgewählt aus der Gruppe R⁵, substituiert ist,
- R²: unabhängig voneinander für Halogen, Hydroxy, Amino, Cyano, Nitro, Formyl, Formamid, (C₁-C₄)-Alkyl, (C₁-C₄)-Haloalkyl, (C₃-C₆)-Cycloalkyl, (C₂-C₄)-Alkenyl, (C₂-C₄)-Alkinyl, (C₂-C₄)-Haloalkenyl, (C₂-C₄)-Haloalkinyl, (C₁-C₄)-Alkoxy-(C₁-C₄)-alkyl, (C₁-C₄)-Haloalkoxy-(C₁-C₄)-alkyl, (C₁-C₄)-Alkylthio-(C₁-C₄)-alkyl, (C₁-C₄)-Alkylsulfinyl-(C₁-C₄)-alkyl, (C₁-C₄)-Alkylsulfonyl-(C₁-C₄)-alkyl, (C₁-C₄)-Alkylcarbonyl, (C₁-C₄)-Haloalkylcarbonyl, (C₃-C₆)-Cycloalkylcarbonyl, Carboxyl, (C₁-C₄)-Alkoxycarbonyl, (C₁-C₄)-Haloalkoxycarbonyl, (C₃-C₆)-Cycloalkoxycarbonyl, (C₁-C₄)-Alkylaminocarbonyl, (C₂-C₆)-Dialkylaminocarbonyl, (C₃-C₆)-Cycloalkylaminocarbonyl, (C₁-C₄)-Alkylcarbonylamino, (C₁-C₄)-Haloalkylcarbonylamino, (C₂-C₆)-Cycloalkylcarbonylamino, (C₁-C₄)-Alkoxycarbonylamino, (C₁-C₄)-Alkylaminocarbonylamino, (C₂-C₆)-Dialkylaminocarbonylamino, Carboxy-(C₁-C₄)-alkyl, (C₁-C₄)-Alkoxycarbonyl-(C₁-C₄)-alkyl, (C₁-C₄)-Haloalkoxycarbonyl-(C₁-C₄)-alkyl, (C₃-C₆)-Cycloalkoxycarbonyl-(C₁-C₄)-alkyl, (C₁-C₄)-Alkoxy, (C₁-C₄)-Haloalkoxy, (C₃-C₆)-Cycloalkoxy, (C₁-C₄)-Alkenyloxy, (C₁-C₄)-Alkinyloxy, (C₁-C₄)-Alkylthio, (C₁-C₄)-Haloalkylthio, (C₃-C₆)-Cycloalkylthio, (C₁-C₄)-Alkylsulfinyl, (C₁-C₄)-Haloalkylsulfinyl, (C₃-C₆)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl, (C₂-C₆)-Dialkylaminosulfonyl oder (C₃-C₆)-Trialkylsilyl steht,
- n: ist gleich 0, 1, 2, 3, oder 4,
- R³: für Wasserstoff, Halogen, Cyano, (C₁-C₄)-Alkyl oder (C₁-C₄)-Haloalkyl steht,
- R⁴: für Wasserstoff oder Halogen steht,
- und R⁵: für Wasserstoff, Halogen, Hydroxy, Amino, Cyano, Formyl, (C₁-C₄)-Alkyl, (C₁-C₄)-Haloalkyl, (C₁-C₄)-Alkoxy, (C₁-C₄)-Haloalkoxy, (C₁-C₄)-Alkoxycarbonyl oder (C₁-C₄)-Alkoxythiocarbonyl steht,
ausgenommen 2-Chlor-4-(2-pyridin-2-yloxyphenyl)pyridin.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der azide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Arylalkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die erfindungsgemäß substituierten Pyridinyloxybenzole der allgemeinen Formel (I) können in Abhängigkeit von äußeren Bedingungen, wie pH-Wert, Lösungsmittel und Temperatur eventuell in verschiedenen tautomeren Strukturen vorliegen, die alle von der allgemeinen Formel (I) umfasst sind. Im Folgenden werden die erfindungsgemäß verwendeten Verbindungen der Formel (I) und ihre Salze als "Verbindungen der allgemeinen Formel (I)" bezeichnet.

Bevorzugter Erfindungsgegenstand sind Verbindungen der allgemeinen Formel (I), worin
- R¹: für ein gegebenenfalls substituiertes Pyridin oder Pyrimidin steht, das optional mit bis zu 4 Substituenten, unabhängig voneinander ausgewählt aus der Gruppe R⁵, substituiert ist,
- R²: unabhängig voneinander für Halogen, Hydroxy, Amino, Cyano, Nitro, Formyl, Formamid, (C₁-C₄)-Alkyl, (C₁-C₄)-Haloalkyl, (C₃-C₆)-Cycloalkyl, (C₂-C₄)-Alkenyl, (C₂-C₄)-Alkinyl, (C₁-C₄)-Alkylcarbonyl, (C₁-C₄)-Haloalkylcarbonyl, (C₃-C₆)-Cycloalkylcarbonyl, Carboxyl, (C₁-C₄)-Alkoxycarbonyl, (C₁-C₄)-Haloalkoxycarbonyl, (C₁-C₄)-Alkylaminocarbonyl, (C₂-C₆)-Dialkylaminocarbonyl, (C₁-C₄)-Alkylcarbonylamino, (C₁-C₄)-Haloalkylcarbonylamino, Carboxy-(C₁-C₄)-alkyl, (C₁-C₄)-Alkoxycarbonyl-(C₁-C₄)-alkyl, (C₁-C₄)-Haloalkoxycarbonyl-(C₁-C₄)-alkyl, (C₁-C₄)-Alkoxy, (C₁-C₄)-Haloalkoxy, (C₃-C₆)-Cycloalkoxy, (C₁-C₄)-Alkenyloxy, (C₁-C₄)-Alkinyloxy, (C₁-C₄)-Alkylthio, (C₁-C₄)-Haloalkylthio, (C₃-C₆)-Cycloalkylthio, (C₁-C₄)-Alkylsulfinyl, (C₁-C₄)-Haloalkylsulfinyl, (C₃-C₆)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl, (C₂-C₆)-Dialkylaminosulfonyl oder (C₃-C₆)-Trialkylsilyl steht,
- n: ist gleich 0, 1, 2, oder 3,
- R³: für Wasserstoff, Halogen, Cyano, (C₁-C₄)-Alkyl oder (C₁-C₄)-Haloalkyl steht,
- R⁴: für Wasserstoff oder Halogen steht,
- und R⁵: für Wasserstoff, Halogen, Cyano, Formyl, (C₁-C₄)-Alkyl, (C₁-C₄)-Haloalkyl, (C₁-C₄)-Alkoxy, (C₁-C₄)-Haloalkoxy, (C₁-C₄)-Alkoxycarbonyl oder (C₁-C₄)-Alkoxythiocarbonyl steht,
ausgenommen 2-Chlor-4-(2-pyridin-2-yloxyphenyl)pyridin.

Besonders bevorzugter Erfindungsgegenstand sind Verbindungen der allgemeinen Formel (I), worin
- R¹: für ein gegebenenfalls substituiertes Pyridin oder Pyrimidin steht, das optional mit bis zu 4 Substituenten, unabhängig voneinander ausgewählt aus der Gruppe R⁵, substituiert ist,
- R²: unabhängig voneinander für Halogen, Hydroxy, Amino, Cyano, Nitro, Formyl, (C₁-C₄)-Alkyl, (C₁-C₄)-Haloalkyl, (C₃-C₆)-Cycloalkyl, Carboxyl, (C₁-C₄)-Alkoxycarbonyl, (C₁-C₄)-Haloalkoxycarbonyl, (C₁-C₄)-Alkylaminocarbonyl, (C₂-C₆)-Dialkylaminocarbonyl, (C₁-C₄)-Alkylcarbonylamino, Carboxy-(C₁-C₄)-alkyl, (C₁-C₄)-Alkoxycarbonyl-(C₁-C₄)-alkyl, (C₁-C₄)-Alkoxy, (C₁-C₄)-Haloalkoxy, (C₁-C₄)-Alkenyloxy, (C₁-C₄)-Alkinyloxy oder (C₁-C₄)-Alkylthio steht,
- n: ist gleich 0, 1, 2, oder 3,
- R³: für Wasserstoff, Halogen, Cyano, (C₁-C₄)-Alkyl oder (C₁-C₄)-Haloalkyl steht,
- R⁴: für Wasserstoff oder Halogen steht,
- und R⁵: für Wasserstoff, Halogen, Formyl, (C₁-C₄)-Alkyl, (C₁-C₄)-Haloalkyl, (C₁-C₄)-Alkoxy, (C₁-C₄)-Haloalkoxy, (C₁-C₄)-Alkoxycarbonyl oder (C₁-C₄)-Alkoxythiocarbonyl steht,
ausgenommen 2-Chlor-4-(2-pyridin-2-yloxyphenyl)pyridin.

Ganz besonders bevorzugter Erfindungsgegenstand sind Verbindungen der allgemeinen Formel (I), worin
- R¹: für ein gegebenenfalls substituiertes Pyridin oder Pyrimidin steht, das optional mit bis zu 4 Substituenten, unabhängig voneinander ausgewählt aus der Gruppe R⁵, substituiert ist,
- R²: unabhängig voneinander für Fluor, Chlor, Brom, Amino, Hydroxy, Cyano, Carboxyl, Nitro, Methyl, Ethyl, Cyclopropyl, Trifluormethyl, Difluormethyl, Methoxy, Ethoxy, Allyloxy, But-2-ynoxy, Trifluormethoxy oder Methylthio steht,
- n: ist gleich 0, 1, 2, oder 3,
- R³: für Wasserstoff, Fluor, Chlor, Brom, Cyano, Methyl, Ethyl, Trifluormethyl, oder Difluormethyl steht,
- R⁴: für Wasserstoff, Fluor oder Chlor steht,
- und R⁵: für Wasserstoff, Fluor, Chlor, Brom, Formyl, Methyl, Trifluormethyl, Difluormethyl, Chlorfluormethyl, Chlordifluormethyl, Methoxycarbonyl oder Methoxythiocarbonyl steht,
ausgenommen 2-Chlor-4-(2-pyridin-2-yloxyphenyl)pyridin.

Äußerst bevorzugter Erfindungsgegenstand sind Verbindungen der allgemeinen Formel (I), worin
- R¹: 4-Pyridin oder 4-Pyrimidin für ein gegebenenfalls substituiertes 2-Pyridin, 3-Pyridin, steht, das optional mit bis zu 3 Substituenten, unabhängig voneinander ausgewählt aus der Gruppe R⁵, substituiert ist,
- R²: unabhängig voneinander für Fluor, Chlor, Brom, Amino, Hydoxy, Nitro, Cyano, Carboxyl, Methyl, Ethyl, Cyclopropyl, Trifluormethyl, Methoxy, Ethoxy, Allyloxy, But-2-ynoxy oder Methylthio steht,
- n: ist gleich 0, 1 oder 2,
- R³: für Wasserstoff, Fluor, Chlor, Brom, Cyano, Methyl, Ethyl, oder Trifluormethyl steht,
- R⁴: für Wasserstoff, Fluor oder Chlor steht,
- und R⁵: für Wasserstoff, Chlor, Formyl, Methyl, Difluormethyl, Trifluormethyl, Chlorfluormethyl, Chlordifluormethyl, Methoxycarbonyl oder Methoxythiocarbonyl steht,
ausgenommen 2-Chlor-4-(2-pyridin-2-yloxyphenyl)pyridin.

Die oben aufgeführten allgemeinen oder in Vorzugsbereichen aufgeführten Restedefinitionen gelten sowohl für die Endprodukte der allgemeinenFormel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Vor allem aus den Gründen der höheren herbiziden Wirkung, besseren Selektivität und/oder besseren Herstellbarkeit sind erfindungsgemäße Verbindungen der genannten allgemeinen Formel (I) oder deren Salze bzw. deren erfindungsgemäße Verwendung von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₁-C₄)-Alkoxy über das Sauerstoffatom, und im Falle von Carboxy-(C₁-C₄)-alkyl oder (C₁-C₄)-Alkoxy-(C₁-C₄)-alkyl jeweils über das C-Atom der Alkylgruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 4 Kohlenstoffatomen, z.B. (aber nicht beschränkt auf) (C₁-C₄)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 4 Kohlenstoffatomen, wie (C₁-C₄)-Alkylthio, z.B. (aber nicht beschränkt auf) (C₁-C₄)-Alkylthio wie Methylthio, Ethylthio, Propylthio, 1-Methylethylthio, Butylthio, 1-Methylpropylthio, 2-Methylpropylthio, 1,1-Dimethylethylthio.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₄)-Alkylsulfinyl, z. B. (aber nicht beschränkt auf) (C₁-C₄)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl.

"Alkoxy" bedeutet ein über ein Sauerstoffatom gebundenen Alkylrest, z. B. (aber nicht beschränkt auf) (C₁-C₄)-Alkoxy wie Methoxy, Ethoxy, Propoxy, 1-Methylethoxy, Butoxy, 1-Methylpropoxy, 2-Methylpropoxy, 1,1-Dimethylethoxy.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₄)-Alkylcarbonyl. Die Anzahl der C-Atome bezieht sich dabei auf den Alkylrest in der Alkylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₄)-Alkoxycarbonyl. Die Anzahl der C-Atome bezieht sich dabei auf den Alkylrest in der Alkoxycarbonylgruppe.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z.B. CCl₃, CClF₂, CFCl₂,CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Haloalkoxy" ist z.B. OCF₃, OCHF₂, OCH₂F, OCF₂CF₃, OCH₂CF₃ und OCH₂CH₂Cl; entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl) und 1,2-Butadienyl. Alkenyl bedeutet z.B. Vinyl, welches ggf. durch weitere Alkylreste substituiert sein kann, z B. (aber nicht beschränkt auf) (C₂-C₄)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl. (C₂-C₄)-Alkinyl bedeutet z.B. Ethinyl, 1-Propinyl, 2-Propinyl, 1-Butinyl, 2-Butinyl, 3-Butinyl, 1-Methyl-2-propinyl.

Der Begriff "Cycloalkyl" bedeutet ein carbocyclisches, gesättigtes Ringsystem mit vorzugsweise 3-6 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₆)-Cycloalkyl" bedeutet eine Kurzschreibweise für Cycloalkyl mit drei bis 6 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-6 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅. Cycloalkyliden bedeutet ein carbocyclischer Rest, der über eine Zweifachbindung gebunden ist.

"Arylalkyl" steht für einen über eine Alkylgruppe gebundenen Arylrest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 4 Kohlenstoffatomen, wie (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

"Halocycloalkyl" bedeutet durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl , z.B. 1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl.

Erfindungsgemäß steht "Trialkylsilyl" - in Alleinstellung oder als Bestandteil einer chemischen Gruppe - für geradkettiges oder verzweigtes Si-Alkyl, vorzugsweise mit 1 bis 6 Kohlenstoffatomen, wie Tri-[(C₁-C₂)-alkyl]silyl, z.B. (aber nicht beschränkt auf) Trimethylsilyl, Triethylsilyl.

Wenn die Verbindungen durch Wasserstoffverschiebung Tautomere bilden können, welche strukturell formal nicht durch die allgemeine Formel (I) erfasst würden, so sind diese Tautomere gleichwohl von der Definition der erfindungsgemäßen Verbindungen der allgemeinen Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der allgemeinen Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der allgmeinen Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der allgemeinen Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

Die vorliegende Erfindung beansprucht auch Verfahren zur Herstellung der erfindungsgemäßen Verbindungen der allgemeinen Formel (I).

Die erfindungsgemäßen Verbindungen der allgemeinen Formel (I) können unter anderem ausgehend von bekannten Verfahren hergestellt werden. Die eingesetzten und untersuchten Syntheserouten gehen dabei von kommerziell erhältlichen oder leicht herstellbaren Bausteinen aus. Die Gruppierungen R¹, R², R³, R⁴ und n der allgemeinen Formel (I) haben in den nachfolgenden Schemata die zuvor definierten Bedeutungen, sofern nicht beispielhafte, aber nicht einschränkende, Definitionen erfolgen.

Erfindungsgemäße Verbindungen können beispielsweise nach der in Schema 1 angegebenen Methode hergestellt werden.

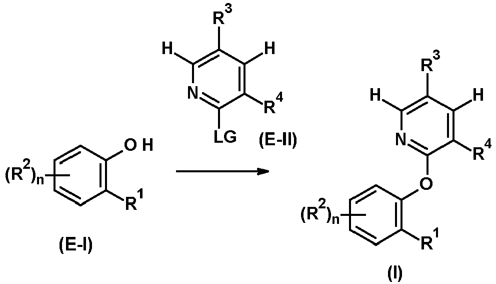

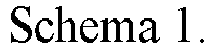

Die Pyridine der allgemeinen Formel (I) können über eine Alkylierung der Benzole (E-I) in Gegenwart von Basen mit dem Pyridin (E-II), wobei LG eine Abgangsgruppe ist, hergestellt werden. Die Base kann ein Carbonat-Salz von einem Alkali-Metall (wie zum Beispiel Natrium, Kalium oder Cäsium) sein. Die Reaktionen werden im Allgemeinen in einem organischen Lösungsmittel, wie zum Beispiel Acetonitril, Dimethylformamid, oder 1-Methyl-2-pyrrolidon, bei Temperaturen zwischen 0°C und dem Siedepunkt des Lösemittels, durchgeführt. Die in Schema 1 genannten Reste R¹, R², R³ und R⁴ sowie der Index n entsprechen den zuvor genannten Definitionen.

Phenole der allgemeinen Formel (E-I) sind literaturbekannt und können beispielsweise gemäß der in WO 2015/108779 und ähnlich beschriebenen Methoden hergestellt werden.

Die erfindungsgemäßen Verbindungen werden in den Ansprüchen definiert und weisen als Rest R¹ einen Pyridin- oder Pyrimidinring auf. Die im folgenden gezeigten Verbindungen, welche als R¹ einen 5-gliedrigen Heteroarylrest aufweisen, sind Vergleichsverbindungen. Synthesebeispiel: 5 -[2-[(5 -Chlor-2-pyridyl)oxy] -6-methyl-phenyl] -3 -(difluormethyl)isoxazol (Tabellenbeispiel Nr. 1-18):

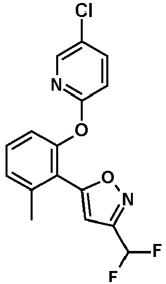

Eine Mischung von 170 mg (0.76 mmol) 2-[3-(Difluormethyl)isoxazol-5-yl]-3-methyl-phenol, 130 mg (0.86 mmol) 2,5-Dichlorpyridin und 220 mg (1.6 mmol) K₂CO₃ in 2 mL 1-Methyl-2-pyrrolidon wurden bei 180 °C für 16 h erhitzt. Durch anschließende säulenchromatographische Reinigung des resultierenden Rohproduktes konnte ein farbloses Öl erhalten werden.

Die Ausbeute beträgt 190 mg (76% der Theorie).

In Analogie zu den oben angeführten und an entsprechender Stelle rezitierten Herstellungsbeispielen erhält man die nachfolgend genannten und in Tabelle 1 dargestellten Verbindungen der allgemeinen

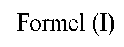

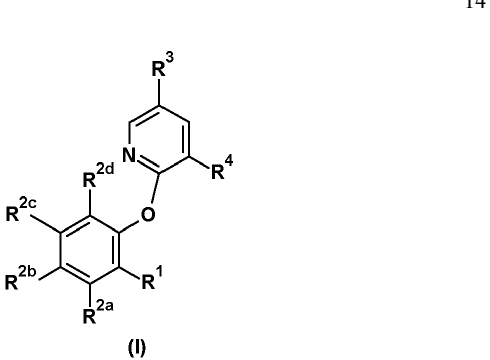

**Tabelle 1**

| Beispielnummer | R¹ | R^{2a} | R^{2b} | R^{2c} | R^{2d} | R³ | R⁴ |
|---|---|---|---|---|---|---|---|
| 1-1 | 5-CF₃-3-Isoxazolyl | F | H | H | H | H | H |
| 1-2 | 5-CF₃-3-Isoxazolyl | F | H | H | H | Cl | F |
| 1-3 | 5-CF₃-3-Isoxazolyl | F | H | H | H | CN | H |
| 1-4 | 5-CF₃-3-Isoxazolyl | F | H | H | H | Cl | Cl |
| 1-5 | 5-CF₃-3-Isoxazolyl | F | H | H | H | CF₃ | Cl |
| 1-6 | 5-CF₃-3-Isoxazolyl | F | H | H | H | H | F |
| 1-7 | 5-CF₃-3-Isoxazolyl | F | H | H | H | CF₃ | H |
| 1-8 | 5-CHF₂-3-Isoxazolyl | F | H | H | H | H | H |
| 1-9 | 5-CHF₂-3-Isoxazolyl | F | H | H | H | Cl | H |
| 1-10 | 5-CHF₂-3-Isoxazolyl | Me | H | H | H | Cl | F |
| 1-11 | 5-CHF₂-3-Isoxazolyl | F | H | H | H | CN | H |
| 1-12 | 5-CHF₂-3-Isoxazolyl | F | H | H | H | Cl | Cl |
| 1-13 | 5-CHF₂-3-Isoxazolyl | F | H | H | H | CF₃ | Cl |
| 1-14 | 5-CHF₂-3-Isoxazolyl | F | H | H | H | H | F |
| 1-15 | 5-CHF₂-3-Isoxazolyl | F | H | H | H | CF₃ | H |
| 1-16 | 3-CHF₂-5-Isoxazolyl | F | H | H | H | H | H |
| 1-17 | 3-CHF₂-5-Isoxazolyl | F | H | H | H | Cl | H |
| 1-18 | 3-CHF₂-5-Isoxazolyl | Me | H | H | H | Cl | H |
| 1-19 | 3-CHF₂-5-Isoxazolyl | F | H | H | H | Cl | F |
| 1-20 | 3-CHF₂-5-Isoxazolyl | Me | H | H | H | Cl | F |
| 1-21 | 3-CHF₂-5-Isoxazolyl | F | H | H | H | Cl | Cl |
| 1-22 | 3-CHF₂-5-Isoxazolyl | F | H | H | H | CF₃ | Cl |
| 1-23 | 3-CHF₂-5-Isoxazolyl | F | H | H | H | H | F |
| 1-24 | 3-CHF₂-5-Isoxazolyl | F | H | H | H | CF₃ | H |
| 1-25 | 3-CHF₂-5-Isoxazolyl | H | H | Me | H | H | H |
| 1-26 | 3-CHF₂-5-Isoxazolyl | H | H | Me | H | Cl | H |
| 1-27 | 3-CHF₂-5-Isoxazolyl | H | H | Me | H | Cl | F |
| 1-28 | 3-CHF₂-5-Isoxazolyl | H | H | Me | H | Cl | Cl |
| 1-29 | 5-CHF₂-3-Isoxazolyl | H | F | H | H | H | H |
| 1-30 | 5-CHF₂-3-Isoxazolyl | H | F | H | H | Cl | H |
| 1-31 | 5-CHF₂-3-Isoxazolyl | H | F | H | H | Cl | F |
| 1-32 | 1-Me-3-CHF₂-1*H*-pyrazol-5-yl | H | F | H | H | Cl | H |
| 1-33 | 3-CHF₂-5-Isoxazolyl | H | H | Me | H | H | F |
| 1-34 | 1-Me-3-CHO-5-Pyrazolyl | F | H | H | H | Cl | H |
| 1-35 | 3-CHF₂-5-Isoxazolyl | H | H | H | H | Cl | H |
| 1-36 | 3-CHF₂-5-Isoxazolyl | H | H | H | H | Cl | F |
| 1-37 | 3-CHF₂-5-Isoxazolyl | H | H | OMe | H | Cl | F |
| 1-38 | 3-CHF₂-5-Isoxazolyl | H | H | H | H | H | H |
| 1-39 | 3-CHF₂-5-Isoxazolyl | H | H | OMe | H | Cl | H |
| 1-40 | 3-CHF₂-5-Isoxazolyl | F | H | H | H | Br | H |
| 1-41 | 3-CHF₂-5-Isoxazolyl | F | H | H | H | F | H |
| 1-42 | 3-CHF₂-5-Isoxazolyl | H | H | OH | H | Cl | H |
| 1-43 | 3-CHF₂-5-Isoxazolyl | H | H | but-2-ynoxy | H | Cl | F |
| 1-44 | 3-CHF₂-5-Isoxazolyl | H | H | OAllyl | H | Cl | F |
| 1-45 | 3-CHF₂-5-Isoxazolyl | H | H | OEt | H | Cl | H |
| 1-46 | 3-CHF₂-5-Isoxazolyl | H | H | OEt | H | Cl | F |
| 1-47 | 3-CHF₂-5-Isoxazolyl | H | H | H | H | H | F |
| 1-48 | 6-CF₃-2-Pyridinyl | H | H | H | H | Cl | F |
| 1-49 | 6-CF₃-2-Pyridinyl | H | H | H | H | Cl | H |
| 1-50 | 6-CF₃-2-Pyridinyl | H | H | H | H | H | H |
| 1-51 | 6-CF₃-2-Pyridinyl | H | H | H | H | Me | H |
| 1-52 | 5-Cl-2-Pyridinyl | H | H | H | H | Me | H |
| 1-53 | 3-CHF₂-5-Isoxazolyl | Br | H | H | H | Cl | H |
| 1-54 | 5-CHF₂-3-Isoxazolyl | H | H | Br | H | Cl | H |
| 1-55 | 3-CHF₂-5-Isoxazolyl | CN | H | H | H | Cl | H |
| 1-56 | 3-CHF₂-5-Isoxazolyl | SMe | H | H | H | Cl | H |
| 1-57 | 5-CHF₂-3-Isoxazolyl | H | H | H | H | Cl | H |
| 1-58 | 5-CHF₂-3-Isoxazolyl | H | H | COzH | H | Cl | H |
| 1-59 | 3-CHF₂-5-Isoxazolyl | Et | H | H | H | Cl | H |
| 1-60 | 3-CHF₂-5-Isoxazolyl | Et | H | H | H | Et | H |
| 1-61 | 1-Me-5-CHF₂-3-Pyrazolyl | F | H | H | H | Cl | F |
| 1-62 | 3-CHF₂-5-Isoxazolyl | H | H | Br | H | Cl | H |
| 1-63 | 5-CHF₂-3-Isoxazolyl | H | H | SMe | H | Cl | H |
| 1-64 | 1-Me-5-CHF₂-3-Pyrazolyl | F | H | H | H | Cl | H |
| 1-65 | 1-Me-5-CHF₂-3-Pyrazolyl | H | F | H | H | Cl | H |
| 1-66 | 5-CF₃-2-Pyridinyl | H | H | H | H | Cl | F |
| 1-67 | 5-CF₃-2-Pyridinyl | H | H | H | H | Cl | H |
| 1-68 | 5-CF₃-2-Pyridinyl | H | H | H | H | H | H |
| 1-69 | 4-CF₃-2-Pyridinyl | H | H | H | H | Cl | F |
| 1-70 | 4-CF₃-2-Pyridinyl | H | H | H | H | Cl | H |
| 1-71 | 4-CF₃-2-Pyridinyl | H | H | H | H | H | H |
| 1-72 | 5-CF₃-3-Pyridinyl | H | H | H | H | Cl | F |
| 1-73 | 5-CF₃-3-Pyridinyl | H | H | H | H | Cl | H |
| 1-74 | 5-Cl-2-Pyridinyl | H | H | H | H | Cl | F |
| 1-75 | 5-Cl-2-Pyridinyl | H | H | H | H | Cl | H |
| 1-76 | 5-Cl-2-Pyridinyl | H | H | H | H | H | H |
| 1-77 | 3-CHF₂-5-Isoxazolyl | cPr | H | H | H | Cl | H |
| 1-78 | 3-CHF₂-5-Isoxazolyl | Me | H | Me | H | Cl | H |
| 1-79 | 3-CHF₂-5-Isoxazolyl | Me | H | Me | H | Cl | F |
| 1-80 | 5-Me-2-Pyridinyl | H | H | H | H | Cl | H |
| 1-81 | 4-Me-2-Pyridinyl | H | H | H | H | Cl | H |
| 1-82 | 1-Me-3-CHF₂-5-Pyrazolyl | F | H | H | H | Cl | F |
| 1-83 | 1-Me-5-CHF₂-3-Pyrazolyl | F | H | H | H | H | H |
| 1-84 | 1-Me-5-CHF₂-3-Pyrazolyl | F | H | H | H | H | F |
| 1-85 | 3-CHF₂-5-Isoxazolyl | H | H | Br | H | Cl | F |
| 1-86 | 3-CHF₂-5-Isoxazolyl | Me | H | Me | H | H | H |
| 1-87 | 5-CHF₂-3-Isoxazolyl | F | H | Me | H | Cl | F |
| 1-88 | 3-CHF₂-5-Isoxazolyl | F | H | Me | H | Cl | H |
| 1-89 | 3-CHF₂-5-Isoxazolyl | F | H | Me | H | Cl | F |
| 1-90 | 5-CHF₂-3-Isoxazolyl | F | H | Me | H | Cl | H |
| 1-91 | 3-CHF₂-5-Isoxazolyl | F | H | Me | H | H | H |
| 1-92 | 3-CHFCl-5-Isoxazolyl | F | H | H | H | Cl | F |
| 1-93 | 3-CO₂Me-5-Isoxazolyl | H | H | H | H | Cl | F |
| 1-94 | 3-CF₂Cl-5-Isoxazolyl | F | H | H | H | Cl | F |
| 1-95 | 3-CHF₂-5-Isoxazolyl | F | H | H | H | Me | H |
| 1-96 | 3-C(=S)OMe-5-Isoxazolyl | H | H | H | H | Cl | F |
| 1-97 | 2-CF₃-4-Pyridinyl | H | H | H | H | Cl | F |
| 1-98 | 2-CF₃-4-Pyridinyl | H | H | H | H | Cl | H |
| 1-99 | 2-CF₃-4-Pyridinyl | H | Me | H | H | Cl | F |
| 1-100 | 2-CF₃-4-Pyridinyl | H | Me | H | H | Cl | H |
| 1-101 | 3-CHF₂-5-Isoxazolyl | H | Me | H | H | Cl | H |
| 1-102 | 3-CHF₂-5-Isoxazolyl | H | Me | H | H | H | F |
| 1-103 | 3-CHF₂-5-Isoxazolyl | H | Me | H | H | H | H |
| 1-104 | 2-CF₃-4-Pyridinyl | H | H | H | H | F | F |
| 1-105 | 2-CF₃-4-Pyridinyl | H | Me | H | H | F | F |
| 1-106 | 5-Me-2-Pyridinyl | H | H | H | H | Cl | F |
| 1-107 | 4-Me-2-Pyridinyl | H | H | H | H | H | H |
| 1-108 | 6-Me-4-Pyrimidinyl | H | H | H | H | Cl | H |
| 1-109 | 6-Me-4-Pyrimidinyl | H | H | H | H | Cl | F |
| 1-110 | 6-Me-4-Pyrimidinyl | H | H | H | H | H | H |
| 1-111 | 6-Me-4-Pyrimidinyl | H | H | H | H | H | F |
| 1-112 | 3-CF₃-5-Isoxazolyl | H | H | H | H | Cl | F |
| 1-113 | 3-CF₃-5-Isoxazolyl | H | H | H | H | Cl | H |
| 1-114 | 3-CHF₂-5-Isoxazolyl | H | Me | H | H | Cl | F |
| 1-115 | 5-CHF₂-3-Isoxazolyl | H | Me | H | H | Cl | F |
| 1-116 | 3-Me-5-Isoxazolyl | F | H | H | H | Cl | F |
| 1-117 | 3-Me-5-Isoxazolyl | F | H | H | H | Cl | H |
| 1-118 | 5-CF₃-3-Isoxazolyl | H | H | H | H | Cl | H |
| 1-119 | 5-CF₃-3-Isoxazolyl | H | H | H | H | Cl | F |

### NMR-Daten ausgewählter Beispiele

Ausgewählte detaillierte Synthesebeispiele für die erfindungsgemäßen Verbindungen der allgemeinen Formeln (I) sind im Folgenden aufgeführt. Die ¹H-NMR-spektroskopischen Daten, die für die in den nachfolgenden Abschnitten beschriebenen chemischen Beispiele angegeben sind, (400 MHz bei ¹H-NMR, Lösungsmittel CDCl₃ oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben.

Die nachfolgend aufgeführten spektroskopischen Daten ausgewählter Tabellenbeispiele wurden über klassische ¹H-NMR-Interpretation ausgewertet.

Klassische ¹H-NMR-Interpretation
Beispiel Nr. 1-30:
   ¹H-NMR (400 MHz, d₆-DMSO δ, ppm) 8.05 (d, 1H), 7.97 (dd, 1H), 7.87 (dd, 1H), 7.70 (dd, 1H), 7.57-7.52 (m, 1H), 7.47 (dd, 1H), 7.19 (d, 1H), 7.17 (t, J = 50 Hz, 1H).
Beispiel Nr. 1-31:
   ¹H-NMR (400 MHz, CDCl₃ δ, ppm) 7.90-7.87 (m, 1H), 7.73 (d, 1H), 7.53 (dd, 1H), 7.33-7.32 (m, 1H), 7.30-7.27 (m, 2H), 6.63 (t, J = 50 Hz, 1H).
Beispiel Nr. 1-41:
   ¹H-NMR (400 MHz, d₆-DMSO δ, ppm) 8.11 (d, 1H), 7.79-7.75 (m, 1H), 7.69-7.65 (m, 1H), 7.38-7.18 (m, 4H), 7.11 (s, 1H).
Beispiel Nr. 1-55:
   ¹H-NMR (400 MHz, d₆-DMSO δ, ppm) 8.17 (d, 1H), 8.02-8.00 (m, 2H), 7.85 (t, 1H), 7.78 (dd, 1H), 7.32 (t, 1H), 7.26-7.25 (m, 2H),
Beispiel Nr. 1-60:
   ¹H-NMR (400 MHz, d₆-DMSO δ, ppm) 7.94 (d, 1H), 7.66 (dd, 1H), 7.54 (t, 1H), 7.29 (d, 1H), 7.25 (t, 1H), 7.07 (d, 1H), 6.86-6.85 (m, 2H), 2.57-2.53 (m, 4H), 1.14 (t, 3H), 1.09 (t, 3H).
Beispiel Nr. 1-83:
   ¹H-NMR (400 MHz, CDCl₃ δ, ppm) 8.15-8.13 (m, 1H), 7.67-7.63 (m, 1H), 7.38-7.33 (m, 1H), 7.08-6.94 (m, 3H), 6.87-6.84 (m, 1H), 6.66 (t, 1H), 6.63 (dd, 1H), 3.92 (s, 3H).
Beispiel Nr. 1-84:
   ¹H-NMR (400 MHz, CDCl₃ δ, ppm) 7.83 (dd, 1H), 7.43-7.34 (m, 2H), 7.10-7.06 (m, 2H), 6.94-6.90 (m, 1H), 6.68-6.66 (m, 1H), 6.67 (t, 1H), 3.90 (s, 3H).

### NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁⁾; δ₂ (Intensität₂);........; δᵢ (Intensitätᵢ⁾;......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| |
|---|
| 1-1: ¹H-NMR(400.0 MHz, CDCl3): |
| δ= 8.0688 (3.8); 8.0639 (4.0); 8.0617 (3.4); 8.0575 (2.5); 8.0545 (4.4); 8.0517 (3.6); 8.0494 (3.5); 7.7136 (3.4); 7.7086 (3.4); 7.6955 (3.9); 7.6929 (4.4); 7.6892 (4.7); 7.6749 (3.8); 7.6699 (3.7); 7.5746 (2.9); 7.5593 (3.1); 7.5537 (5.9); 7.5383 (6.3); 7.5327 (3.7); 7.5174 (3.6); 7.4859 (3.2); 7.4822 (8.1); 7.4786 (7.9); 7.4750 (3.0); 7.2592 (49.7); 7.1511 (3.1); 7.1486 (4.2); 7.1361 (3.3); 7.1333 (8.3); 7.1302 (6.4); 7.1269 (7.6); 7.1241 (3.3); 7.1152 (3.3); 7.1124 (7.4); 7.1097 (3.6); 7.1057 (4.2); 7.1031 (2.8); 6.9914 (3.3); 6.9891 (4.9); 6.9806 (5.6); 6.9777 (16.0); 6.9733 (4.1); 6.9710 (4.6); 6.9580 (10.8); 5.2978 (1.8); 1.5370 (3.7); 1.2560 (1.5); 0.0080 (2.0); -0.0002 (63.7); -0.0085 (2.2) |
| 1-2: ¹H-NMR(400.0 MHz, CDCl3): |
| δ= 7.7824 (12.1); 7.7770 (12.4); 7.6109 (3.1); 7.5957 (3.2); 7.5898 (6.1); 7.5748 (6.2); 7.5689 (3.7); 7.5538 (3.6); 7.5292 (7.1); 7.5238 (6.9); 7.5065 (16.0); 7.5018 (13.7); 7.2613 (22.2); 7.2182 (4.4); 7.1966 (7.1); 7.1716 (7.7); 7.1688 (8.3); 7.1480 (7.0); 1.5571 (3.0); 1.2575 (0.8); -0.0002 (25.4) |
| 1-3: ¹H-NMR(400.0 MHz, CDCl3): |
| δ= 11.6440 (0.5); 8.3416 (6.1); 8.3397 (6.6); 8.3358 (6.9); 8.3339 (6.6); 7.9538 (6.4); 7.9480 (6.1); 7.9323 (6.6); 7.9264 (6.7); 7.6288 (2.5); 7.6138 (2.5); 7.6078 (4.8); 7.5929 (5.0); 7.5869 (3.2); 7.5720 (3.0); 7.5550 (0.6); 7.5180 (0.8); 7.4793 (2.8); 7.4756 (7.1); 7.4720 (7.3); 7.4684 (3.0); 7.2591 (132.2); 7.2473 (4.6); 7.2447 (4.6); 7.2261 (3.6); 7.2233 (6.0); 7.2206 (4.2); 7.2019 (3.1); 7.1993 (3.2); 7.1465 (3.5); 7.1437 (6.0); 7.1411 (3.5); 7.1259 (3.3); 7.1231 (5.5); 7.1205 (3.3); 7.0975 (7.2); 7.0956 (7.4); 7.0759 (7.1); 7.0740 (7.2); 7.0545 (0.6); 6.9951 (0.8); 5.2983 (1.2); 1.5291 (16.0); 1.2843 (0.8); 1.2557 (4.2); 0.8802 (0.7); 0.0079 (4.5); -0.0002 (157.4); -0.0085 (7.3); -0.1496 (0.6) |
| 1-4: ¹H-NMR(400.0 MHz, CDCl3): |
| δ= 7.8608 (11.7); 7.8559 (16.0); 7.7667 (13.0); 7.7617 (16.0); 7.6070 (2.6); 7.5864 (6.9); 7.5708 (6.4); 7.5659 (6.2); 7.5503 (3.7); 7.4982 (13.2); 7.4954 (14.2); 7.2591 (24.9); 7.2132 (4.8); 7.1914 (8.9); 7.1678 (4.8); 7.1546 (9.8); 7.1340 (8.9); 1.5350 (7.3); 1.2578 (1.7); 0.8818 (0.8); -0.0002 (26.2) |
| 1-5: ¹H-NMR(400.0 MHz, CDCl3): |
| δ= 8.1746 (3.4); 8.1720 (4.2); 8.1692 (4.5); 8.1667 (3.9); 7.9853 (4.4); 7.9841 (4.7); 7.9799 (4.7); 7.9787 (4.6); 7.6379 (2.0); 7.6229 (2.1); 7.6169 (4.0); 7.6020 (4.0); 7.5959 (2.6); 7.5810 (2.5); 7.4834 (2.0); 7.4798 (5.5); 7.4761 (5.7); 7.4726 (2.3); 7.2587 (65.0); 7.2366 (2.8); 7.2338 (4.8); 7.2310 (3.4); 7.2123 (2.4); 7.2097 (2.6); 7.1890 (2.9); 7.1862 (4.9); 7.1835 (2.9); 7.1683 (2.6); 7.1655 (4.5); 7.1629 (2.6); 1.5267 (16.0); 0.0079 (2.0); -0.0002 (73.9); - 0.0085 (3.3) |
| 1-6: ¹H-NMR(400.0 MHz, CDCl3): |
| δ= 7.8111 (7.6); 7.8073 (7.8); 7.7988 (8.0); 7.7950 (7.9); 7.6004 (4.1); 7.5852 (4.4); 7.5794 (8.0); 7.5642 (8.8); 7.5584 (5.1); 7.5433 (4.9); 7.4892 (9.3); 7.4855 (16.0); 7.4820 (11.8); 7.4785 (4.6); 7.4693 (5.6); 7.4651 (8.3); 7.4609 (5.0); 7.4448 (5.1); 7.4410 (4.9); 7.2590 (60.9); 7.1957 (4.3); 7.1931 (6.6); 7.1876 (5.5); 7.1848 (11.3); 7.1820 (4.6); 7.1744 (5.1); 7.1715 (10.4); 7.1685 (6.0); 7.1669 (6.2); 7.1640 (10.4); 7.1613 (4.5); 7.1501 (5.2); 7.1475 (4.1); 6.9998 (5.0); 6.9917 (5.4); 6.9876 (5.2); 6.9797 (9.1); 6.9719 (4.9); 6.9677 (4.8); 6.9596 (4.4); 1.5351 (5.4); 1.2843 (0.6); 1.2564 (3.3); 0.8801 (0.6); 0.0079 (2.0); -0.0002 (67.6); -0.0085 (2.4) |
| 1-7: ¹H-NMR(400.0 MHz, CDCl3): |
| δ= 8.3202 (6.9); 8.3182 (9.6); 8.3160 (9.5); 8.3142 (9.7); 8.3120 (9.9); 8.3100 (7.2); 7.9339 (6.4); 7.9328 (6.5); 7.9276 (6.5); 7.9266 (6.3); 7.9123 (6.8); 7.9112 (6.8); 7.9060 (6.9); 7.9050 (6.6); 7.6169 (5.6); 7.6017 (5.9); 7.5959 (11.2); 7.5808 (11.1); 7.5750 (6.9); 7.5598 (6.5); 7.4837 (6.2); 7.4801 (16.0); 7.4765 (15.7); 7.4730 (6.1); 7.2594 (34.4); 7.2221 (6.8); 7.2195 (7.3); 7.2008 (7.1); 7.1981 (12.5); 7.1954 (8.1); 7.1767 (6.2); 7.1741 (6.4); 7.1551 (8.3); 7.1524 (13.5); 7.1498 (7.4); 7.1344 (7.6); 7.1317 (12.4); 7.1291 (6.6); 7.0882 (11.6); 7.0665 (11.0); 1.5517 (1.0); 0.0079 (1.3); -0.0002 (44.4); -0.0085 (1.6) |
| 1-8: ¹H-NMR(400.0 MHz, CDCl3): |
| δ= 8.0779 (4.1); 8.0732 (4.2); 8.0708 (3.6); 8.0669 (2.5); 8.0637 (4.6); 8.0609 (3.8); 8.0586 (3.7); 7.7064 (3.5); 7.7014 (3.5); 7.6883 (4.1); 7.6857 (4.6); 7.6820 (5.4); 7.6677 (4.0); 7.6627 (3.9); 7.5492 (3.0); 7.5338 (3.2); 7.5282 (6.2); 7.5179 (1.7); 7.5129 (6.5); 7.5073 (3.8); 7.4919 (3.6); 7.3745 (4.0); 7.3683 (7.4); 7.3627 (4.1); 7.3095 (1.6); 7.2591 (228.1); 7.1365 (3.4); 7.1339 (4.2); 7.1152 (12.0); 7.1122 (11.3); 7.0971 (3.2); 7.0943 (8.2); 7.0912 (7.2); 7.0885 (2.8); 6.9950 (1.4); 6.9858 (3.4); 6.9836 (5.0); 6.9754 (6.0); 6.9723 (16.0); 6.9678 (4.2); 6.9655 (4.8); 6.9528 (12.6); 6.7498 (4.0); 6.7487 (4.1); 6.6150 (8.2); 6.6139 (8.4); 6.4802 (4.1); 6.4791 (4.1); 3.8853 (0.8); 1.5381 (6.1); 1.2843 (0.8); 1.2563 (2.0); 0.8802 (0.6); 0.0502 (0.8); 0.0079 (3.8); -0.0002 (123.4); -0.0085 (4.4) |
| 1-9: ¹H-NMR(400.0 MHz, CDCl3): |
| δ= 8.0006 (13.1); 7.9991 (13.5); 7.9939 (14.3); 7.9925 (13.8); 7.6590 (13.5); 7.6524 (13.1); 7.6372 (14.2); 7.6306 (14.0); 7.5584 (5.4); 7.5431 (5.6); 7.5374 (11.1); 7.5222 (11.0); 7.5165 (6.8); 7.5012 (6.3); 7.3875 (6.9); 7.3818 (13.1); 7.3756 (7.2); 7.2596 (105.9); 7.1604 (6.5); 7.1578 (7.2); 7.1391 (6.7); 7.1365 (12.0); 7.1337 (8.1); 7.1151 (6.0); 7.1125 (6.6); 7.1030 (8.1); 7.1004 (13.1); 7.0978 (7.4); 7.0823 (7.3); 7.0796 (12.0); 7.0771 (6.6); 6.9956 (0.6); 6.9438 (16.0); 6.9424 (15.9); 6.9220 (15.1); 6.9205 (14.9); 6.7788 (7.3); 6.7776 (7.4); 6.6442 (15.2); 6.6430 (14.9); 6.5096 (7.6); 6.5084 (7.5); 5.2971 (0.6); 1.5498 (6.1); 1.2843 (0.6); 1.2582 (1.7); 0.8800 (0.5); 0.0080 (1.7); -0.0002 (61.4); -0.0085 (2.4) |
| 1-10: ¹H-NMR(400.0 MHz, CDCl3): |
| δ= 7.8111 (4.1); 7.8056 (4.1); 7.4802 (1.6); 7.4641 (2.5); 7.4593 (4.2); 7.4412 (3.9); 7.4362 (2.3); 7.3701 (1.4); 7.3638 (2.6); 7.3575 (1.3); 7.2594 (7.9); 7.2482 (2.1); 7.2290 (1.7); 7.1469 (2.0); 7.1265 (1.8); 6.7617 (1.6); 6.6271 (3.4); 6.4925 (1.7); 2.4695 (16.0); 2.4233 (0.7); 1.5560 (0.6); -0.0002 (10.2) |
| 1-11: ¹H-NMR(400.0 MHz, CDCl3): |
| δ= 8.3375 (5.4); 8.3357 (5.7); 8.3317 (5.9); 8.3299 (5.6); 7.9414 (5.5); 7.9356 (5.3); 7.9198 (5.6); 7.9140 (5.6); 7.6041 (2.1); 7.5891 (2.2); 7.5832 (4.2); 7.5682 (4.2); 7.5622 (2.6); 7.5472 (2.5); 7.5186 (0.8); 7.3653 (0.8); 7.3541 (3.0); 7.3481 (5.6); 7.3423 (3.3); 7.2597 (137.8); 7.2322 (2.6); 7.2296 (2.8); 7.2110 (2.7); 7.2082 (4.8); 7.2054 (3.1); 7.1867 (2.4); 7.1841 (2.4); 7.1354 (3.2); 7.1327 (5.3); 7.1300 (3.0); 7.1147 (2.9); 7.1120 (4.9); 7.1094 (2.7); 7.0938 (6.3); 7.0919 (6.2); 7.0722 (6.0); 7.0703 (6.0); 6.9956 (0.8); 6.7660 (3.0); 6.6327 (6.3); 6.6317 (6.3); 6.4984 (3.1); 6.4974 (3.1); 5.2983 (1.0); 3.8858 (0.5); 3.7075 (0.6); 1.5376 (16.0); 1.2844 (0.7); 1.2576 (1.5); 0.0080 (2.3); -0.0002 (76.8); -0.0085 (2.7) |
| 1-12: ¹H-NMR(400.0 MHz, CDCl3): |
| δ= 7.8585 (13.2); 7.8527 (14.6); 7.7582 (16.0); 7.7524 (14.9); 7.5826 (2.9); 7.5675 (3.1); 7.5616 (5.9); 7.5466 (5.9); 7.5407 (3.7); 7.5256 (3.5); 7.3825 (3.9); 7.3772 (7.4); 7.3709 (4.0); 7.2594 (59.6); 7.2023 (3.6); 7.1997 (4.0); 7.1810 (3.8); 7.1783 (6.5); 7.1755 (4.5); 7.1568 (3.4); 7.1542 (3.6); 7.1474 (4.7); 7.1447 (7.3); 7.1420 (3.9); 7.1267 (4.2); 7.1240 (6.6); 7.1213 (3.5); 6.7836 (4.1); 6.7824 (4.0); 6.6489 (8.4); 6.6477 (8.3); 6.5143 (4.2); 6.5131 (4.1); 1.5459 (2.7); 0.0080 (1.1); -0.0002 (32.7); -0.0085 (1.1) |
| 1-13: ¹H-NMR(400.0 MHz, CDCl3): |
| δ= 11.5388 (0.6); 8.1739 (4.5); 8.1716 (10.2); 8.1691 (12.5); 8.1663 (12.7); 8.1638 (10.5); 7.9769 (13.4); 7.9758 (13.7); 7.9715 (13.3); 7.9705 (12.8); 7.6124 (5.5); 7.5974 (5.8); 7.5915 (11.0); 7.5765 (10.9); 7.5705 (6.8); 7.5556 (6.5); 7.5182 (0.7); 7.4930 (0.5); 7.3610 (7.4); 7.3553 (14.0); 7.3492 (7.7); 7.3363 (0.5); 7.2593 (116.9); 7.2435 (7.0); 7.2409 (7.5); 7.2222 (7.1); 7.2194 (12.2); 7.2165 (8.3); 7.1979 (6.2); 7.1952 (6.5); 7.1787 (8.4); 7.1760 (13.8); 7.1733 (7.5); 7.1581 (7.6); 7.1553 (12.5); 7.1526 (6.7); 6.9953 (0.7); 6.9079 (0.5); 6.8292 (0.6); 6.7679 (7.7); 6.7668 (7.7); 6.6946 (0.5); 6.6335 (16.0); 6.6323 (15.5); 6.4990 (7.9); 6.4978 (7.9); 1.5406 (13.8); 1.2581 (0.5); 0.0080 (2.1); - 0.0002 (66.4); -0.0085 (2.3) |
| 1-14: ¹H-NMR(400.0 MHz, CDCl3): |
| δ= 7.8131 (7.4); 7.8092 (7.6); 7.8009 (7.8); 7.7970 (7.7); 7.5750 (4.0); 7.5598 (4.3); 7.5540 (7.8); 7.5388 (8.8); 7.5331 (5.0); 7.5179 (5.3); 7.4820 (4.7); 7.4781 (4.7); 7.4621 (5.4); 7.4579 (7.8); 7.4535 (4.8); 7.4376 (5.2); 7.4337 (4.9); 7.3726 (5.1); 7.3672 (9.6); 7.3609 (5.3); 7.2591 (86.2); 7.1814 (4.2); 7.1788 (6.6); 7.1730 (5.3); 7.1701 (11.2); 7.1672 (4.7); 7.1601 (5.0); 7.1571 (10.1); 7.1541 (5.7); 7.1522 (5.9); 7.1493 (10.4); 7.1466 (4.5); 7.1357 (5.3); 7.1331 (4.1); 6.9951 (0.7); 6.9904 (5.2); 6.9823 (5.5); 6.9781 (5.2); 6.9703 (9.2); 6.9625 (5.0); 6.9583 (4.8); 6.9502 (4.6); 6.7669 (5.5); 6.7657 (5.3); 6.6322 (11.3); 6.6309 (11.1); 6.4974 (5.6); 6.4961 (5.5); 1.5341 (16.0); 1.3333 (0.6); 1.2844 (0.9); 1.2563 (1.4); 0.0080 (3.5); -0.0002 (112.1); -0.0085 (3.8) |
| 1-15: ¹H-NMR(400.0 MHz, CDCl3): |
| δ= 8.3262 (7.1); 8.3241 (9.8); 8.3220 (9.7); 8.3202 (9.8); 8.3180 (10.0); 8.3159 (7.2); 7.9283 (6.6); 7.9272 (6.7); 7.9220 (6.6); 7.9209 (6.5); 7.9065 (6.9); 7.9055 (7.0); 7.9003 (6.7); 7.5972 (5.5); 7.5821 (5.9); 7.5763 (11.2); 7.5611 (11.2); 7.5553 (6.8); 7.5402 (6.5); 7.3874 (7.6); 7.3818 (14.3); 7.3756 (7.8); 7.2591 (50.9); 7.2101 (6.8); 7.2075 (7.4); 7.1888 (7.1); 7.1861 (12.5); 7.1834 (8.2); 7.1647 (6.1); 7.1622 (6.5); 7.1423 (8.4); 7.1396 (13.8); 7.1370 (7.5); 7.1216 (7.6); 7.1189 (12.5); 7.1163 (6.7); 7.0868 (11.9); 7.0652 (11.2); 6.7595 (7.8); 6.7586 (7.8); 6.6252 (16.0); 6.6242 (15.8); 6.4908 (8.0); 6.4898 (7.9); 2.0811 (0.5); 0.0080 (2.1); -0.0002 (63.3); -0.0085 (2.3) |
| 1-16: ¹H-NMR(400.0 MHz, CDCl3): |
| δ= 8.1257 (1.9); 8.1208 (2.2); 8.1113 (2.3); 8.1085 (2.2); 7.7510 (1.6); 7.7460 (1.6); 7.7327 (2.0); 7.7304 (2.3); 7.7270 (2.4); 7.7122 (1.8); 7.7072 (1.8); 7.5196 (1.4); 7.5042 (1.6); 7.4987 (3.1); 7.4832 (3.2); 7.4777 (1.9); 7.4623 (1.7); 7.2587 (76.0); 7.1336 (1.8); 7.1311 (1.9); 7.1125 (1.8); 7.1095 (2.7); 7.1063 (2.3); 7.0877 (1.8); 7.0851 (2.0); 7.0793 (2.5); 7.0767 (3.8); 7.0560 (3.4); 7.0353 (2.4); 7.0233 (8.0); 7.0196 (2.7); 7.0172 (2.6); 7.0048 (5.1); 7.0030 (4.8); 6.8814 (2.7); 6.7865 (4.0); 6.7821 (4.2); 6.7469 (5.5); 6.6124 (2.7); 1.5252 (16.0); 1.2586 (1.5); 0.8818 (0.8); 0.0079 (3.4); -0.0002 (95.1); -0.0084 (4.2) |
| 1-17: ¹H-NMR(400.0 MHz, CDCl3): |
| δ= 8.0378 (10.9); 8.0363 (11.5); 8.0311 (12.0); 8.0296 (11.8); 7.7001 (11.4); 7.6934 (11.2); 7.6783 (12.5); 7.6716 (12.0); 7.5321 (4.7); 7.5166 (5.1); 7.5111 (9.7); 7.4957 (9.8); 7.4902 (6.0); 7.4748 (5.6); 7.2586 (38.0); 7.1596 (5.6); 7.1570 (6.2); 7.1385 (5.6); 7.1351 (7.7); 7.1321 (6.8); 7.1136 (5.2); 7.1110 (5.5); 7.0715 (6.6); 7.0688 (11.3); 7.0662 (6.5); 7.0508 (6.2); 7.0481 (10.5); 7.0454 (5.9); 7.0007 (14.1); 6.9992 (14.6); 6.9789 (12.8); 6.9774 (13.3); 6.8902 (7.7); 6.7952 (10.7); 6.7899 (10.8); 6.7558 (16.0); 6.6213 (8.0); 1.5310 (5.8); 1.2576 (1.2); 0.0080 (1.7); -0.0002 (49.3); -0.0085 (2.1) |
| 1-18: ¹H-NMR(400.0 MHz, CDCl3): |
| δ= 8.0595 (2.7); 8.0582 (2.9); 8.0529 (2.9); 8.0516 (2.9); 7.6236 (2.7); 7.6169 (2.6); 7.6018 (2.8); 7.5952 (2.8); 7.4567 (1.6); 7.4368 (3.1); 7.4170 (2.2); 7.2596 (22.2); 7.2183 (2.0); 7.1993 (1.7); 7.0508 (1.9); 7.0312 (1.8); 6.8713 (1.9); 6.8214 (3.2); 6.8200 (3.3); 6.7996 (3.1); 6.7982 (3.1); 6.7368 (4.2); 6.6022 (2.1); 6.4759 (5.3); 2.3822 (16.0); 1.5378 (7.5); 0.0080 (0.8); -0.0002 (29.9); -0.0085 (0.9) |
| 1-19: ¹H-NMR(400.0 MHz, CDCl3): |
| δ= 7.8179 (2.8); 7.8129 (3.3); 7.5537 (2.2); 7.5362 (2.9); 7.5203 (1.7); 7.4998 (0.8); 7.2609 (24.5); 7.1978 (1.2); 7.1760 (1.8); 7.1518 (1.1); 7.1150 (2.2); 7.0943 (1.9); 6.9001 (1.3); 6.8565 (3.0); 6.7662 (2.6); 6.6318 (1.3); 1.5417 (16.0); -0.0002 (28.5) |
| 1-20: ¹H-NMR(400.0 MHz, CDCl3): |
| δ= 7.8296 (4.5); 7.8242 (4.6); 7.4739 (2.6); 7.4685 (2.9); 7.4513 (5.2); 7.4460 (2.6); 7.4307 (2.1); 7.2595 (22.7); 7.2454 (2.0); 7.2262 (1.7); 7.0951 (2.0); 7.0746 (1.7); 6.8799 (1.9); 6.7454 (4.1); 6.6109 (2.0); 6.5590 (5.2); 2.4006 (16.0); 1.5358 (8.7); 0.0080 (0.9); -0.0002 (29.7); -0.0085 (0.9) |
| 1-21: ¹H-NMR(400.0 MHz, CDCl3): |
| δ= 7.8847 (12.7); 7.8789 (14.4); 7.7903 (16.0); 7.7845 (14.1); 7.5566 (3.1); 7.5412 (3.4); 7.5356 (6.6); 7.5203 (6.6); 7.5147 (3.9); 7.4994 (3.6); 7.2584 (48.6); 7.1991 (3.8); 7.1965 (4.0); 7.1779 (3.9); 7.1748 (5.6); 7.1717 (4.4); 7.1531 (3.4); 7.1505 (3.5); 7.1078 (4.6); 7.1052 (7.6); 7.1025 (4.2); 7.0871 (4.2); 7.0844 (6.9); 7.0818 (3.7); 6.8916 (5.6); 6.8710 (8.0); 6.8657 (7.8); 6.7572 (11.6); 6.6228 (5.8); 2.0427 (0.6); 1.5258 (11.9); 1.2587 (2.4); 0.8986 (1.1); 0.8817 (3.5); 0.8641 (1.5); 0.0080 (2.0); -0.0002 (58.7); -0.0085 (2.0) |
| 1-22: ¹H-NMR(400.0 MHz, CDCl3): |
| δ= 8.1872 (5.2); 8.1847 (6.3); 8.1818 (6.5); 8.1794 (5.4); 8.0066 (6.9); 8.0056 (7.2); 8.0012 (6.8); 7.5912 (2.9); 7.5759 (3.1); 7.5702 (6.0); 7.5550 (6.0); 7.5493 (3.7); 7.5340 (3.4); 7.2586 (62.4); 7.2441 (3.6); 7.2416 (3.8); 7.2230 (3.6); 7.2197 (5.1); 7.2167 (4.1); 7.1981 (3.2); 7.1955 (3.2); 7.1534 (4.3); 7.1506 (7.2); 7.1479 (4.0); 7.1326 (3.8); 7.1299 (6.4); 7.1272 (3.5); 6.8805 (11.3); 6.8757 (7.4); 6.7461 (10.6); 6.6118 (5.3); 1.5260 (16.0); 1.2562 (0.7); 0.0079 (2.4); -0.0002 (75.4); -0.0085 (2.6) |
| 1-23: ¹H-NMR(400.0 MHz, CDCl3): |
| δ= 7.8620 (4.1); 7.8582 (4.3); 7.8498 (4.3); 7.8460 (4.4); 7.5418 (2.3); 7.5264 (2.8); 7.5245 (3.3); 7.5208 (7.2); 7.5052 (6.2); 7.5002 (7.2); 7.4963 (3.0); 7.4845 (2.9); 7.4802 (3.0); 7.4764 (2.7); 7.2586 (76.5); 7.1720 (2.7); 7.1693 (3.0); 7.1508 (2.7); 7.1477 (3.9); 7.1446 (3.4); 7.1264 (5.0); 7.1240 (7.2); 7.1061 (3.2); 7.1034 (5.0); 7.1008 (2.6); 7.0421 (2.8); 7.0340 (2.9); 7.0299 (2.9); 7.0220 (5.0); 7.0141 (2.6); 7.0100 (2.6); 7.0019 (2.4); 6.8866 (3.9); 6.8530 (5.6); 6.8482 (5.6); 6.7522 (8.3); 6.6177 (4.2); 1.5262 (16.0); 1.2582 (1.7); 0.8805 (0.6); 0.0079 (2.7); -0.0002 (84.9); -0.0085 (3.0) |
| 1-24: ¹H-NMR(400.0 MHz, CDCl3): |
| δ= 8.3475 (8.0); 8.3439 (8.5); 8.3417 (8.6); 7.9603 (5.7); 7.9541 (5.7); 7.9387 (6.1); 7.9325 (5.9); 7.5680 (4.1); 7.5526 (4.4); 7.5471 (8.8); 7.5317 (8.8); 7.5262 (5.4); 7.5108 (5.0); 7.2582 (36.8); 7.2075 (5.1); 7.2050 (5.7); 7.1863 (5.0); 7.1830 (8.3); 7.1802 (6.3); 7.1615 (4.7); 7.1590 (5.0); 7.1470 (9.9); 7.1254 (9.4); 7.1169 (6.5); 7.1143 (10.8); 7.1117 (6.5); 7.0961 (5.4); 7.0935 (9.6); 7.0910 (5.7); 6.8763 (7.8); 6.8048 (11.4); 6.7987 (11.5); 6.7419 (16.0); 6.6075 (8.1); 1.5288 (6.7); 1.2566 (0.6); 0.0080 (1.4); -0.0002 (42.7); -0.0085 (1.9) |
| 1-25: ¹H-NMR(400.0 MHz, CDCl3): |
| δ= 8.2119 (1.4); 8.2100 (1.5); 8.2069 (1.6); 8.2050 (1.5); 8.1995 (1.5); 8.1976 (1.6); 8.1945 (1.6); 8.1925 (1.5); 7.9450 (3.5); 7.9248 (3.7); 7.7779 (1.5); 7.7729 (1.5); 7.7598 (1.7); 7.7573 (1.8); 7.7548 (1.7); 7.7523 (1.7); 7.7392 (1.8); 7.7341 (1.7); 7.2597 (20.0); 7.1693 (1.4); 7.1675 (1.6); 7.1653 (1.6); 7.1635 (1.4); 7.1491 (1.3); 7.1474 (1.5); 7.1451 (1.6); 7.1434 (1.4); 7.0740 (1.7); 7.0717 (1.8); 7.0615 (1.6); 7.0592 (1.9); 7.0558 (1.7); 7.0535 (1.7); 7.0434 (1.6); 7.0411 (1.7); 7.0217 (1.9); 7.0196 (3.4); 7.0175 (1.7); 7.0010 (1.9); 6.9989 (3.5); 6.9965 (3.0); 6.9933 (2.8); 6.9917 (2.7); 6.8709 (2.1); 6.7524 (5.3); 6.7363 (4.5); 6.6017 (2.3); 2.4002 (16.0); 1.5421 (7.4); 0.0080 (0.8); -0.0002 (27.1); -0.0085 (0.7) |
| 1-26: ¹H-NMR(400.0 MHz, CDCl3): |
| δ= 8.1251 (3.0); 8.1194 (3.1); 7.9325 (3.4); 7.9124 (3.6); 7.7259 (2.7); 7.7192 (2.6); 7.7041 (2.9); 7.6975 (2.8); 7.2594 (13.4); 7.1888 (1.6); 7.1867 (1.6); 7.1687 (1.5); 7.1665 (1.5); 6.9942 (3.5); 6.9932 (3.4); 6.9780 (3.0); 6.9728 (4.4); 6.8770 (2.0); 6.7424 (4.2); 6.7084 (5.5); 6.6078 (2.1); 2.4078 (16.0); 1.5411 (5.9); 0.0080 (0.5); -0.0002 (17.1) |
| 1-27: ¹H-NMR(400.0 MHz, CDCl3): |
| δ= 7.9435 (0.8); 7.9291 (3.5); 7.9234 (0.9); 7.9090 (3.7); 7.8998 (1.1); 7.8943 (1.2); 7.8872 (5.0); 7.8818 (5.0); 7.6258 (0.6); 7.6204 (0.6); 7.6034 (0.6); 7.5979 (0.7); 7.5936 (2.8); 7.5881 (2.7); 7.5712 (2.9); 7.5657 (2.7); 7.2591 (10.6); 7.2106 (1.8); 7.2089 (2.0); 7.2067 (2.0); 7.2050 (1.6); 7.1904 (1.4); 7.1888 (1.6); 7.1865 (1.6); 7.1849 (1.3); 7.0609 (0.6); 7.0417 (2.9); 7.0402 (2.8); 6.8980 (2.2); 6.8877 (2.0); 6.7859 (5.5); 6.7532 (4.3); 6.6187 (2.1); 2.4312 (3.9); 2.4224 (16.0); 1.5404 (3.2); -0.0002 (14.1) |
| 1-28: ¹H-NMR(400.0 MHz, CDCl3): |
| δ= 7.9458 (4.8); 7.9399 (5.3); 7.9311 (3.6); 7.9109 (3.7); 7.8279 (5.7); 7.8221 (5.2); 7.2588 (8.9); 7.2181 (1.6); 7.2159 (1.7); 7.1979 (1.5); 7.1957 (1.6); 7.0156 (3.0); 7.0141 (2.9); 6.8810 (1.9); 6.8211 (5.6); 6.7464 (4.2); 6.6118 (2.1); 2.4262 (16.0); 2.4073 (0.8); 1.5407 (3.9); -0.0002 (11.6) |
| 1-29: ¹H-NMR(400.0 MHz, CDCl3): |
| δ= 10.6247 (0.7); 9.8683 (2.1); 8.1371 (0.6); 8.0449 (6.6); 8.0431 (7.1); 8.0400 (7.4); 8.0381 (7.1); 8.0325 (7.0); 8.0305 (7.3); 8.0274 (7.4); 8.0256 (7.0); 7.9599 (1.8); 7.8753 (5.9); 7.8710 (10.6); 7.8666 (6.2); 7.8532 (6.1); 7.8501 (6.9); 7.8476 (6.1); 7.8445 (6.0); 7.7871 (0.5); 7.7713 (0.5); 7.7484 (0.6); 7.7362 (6.7); 7.7312 (6.4); 7.7183 (7.5); 7.7154 (8.0); 7.7133 (7.7); 7.7104 (7.3); 7.6975 (7.4); 7.6925 (7.1); 7.5191 (1.8); 7.3892 (0.6); 7.3778 (0.8); 7.3690 (0.6); 7.3388 (8.0); 7.3331 (14.9); 7.3268 (7.8); 7.3157 (0.7); 7.3066 (0.9); 7.2949 (1.0); 7.2690 (28.0); 7.2645 (28.2); 7.2602 (318.7); 7.2537 (25.1); 7.2510 (13.7); 7.2477 (11.7); 7.2253 (0.7); 7.1085 (1.0); 7.0880 (1.1); 7.0704 (1.0); 7.0604 (8.3); 7.0583 (15.5); 7.0563 (8.7); 7.0395 (7.5); 7.0375 (13.9); 7.0355 (7.7); 6.9962 (1.9); 6.9716 (7.8); 6.9694 (7.5); 6.9591 (7.8); 6.9569 (7.7); 6.9537 (7.9); 6.9514 (7.2); 6.9412 (7.3); 6.9389 (7.1); 6.7085 (7.8); 6.5738 (16.0); 6.4392 (8.1); 5.2995 (2.6); 3.8102 (0.8); 2.3679 (0.8); 2.3493 (1.2); 2.3303 (0.9); 1.6193 (0.9); 1.5607 (1.8); 1.4744 (2.6); 1.3330 (1.5); 1.2843 (2.4); 1.2552 (12.8); 0.8967 (0.8); 0.8801 (2.3); 0.8626 (1.0); 0.1461 (0.5); 0.0080 (5.4); - 0.0002 (190.1); -0.0085 (5.6); -0.1496 (0.7) |
| 1-32: ¹H-NMR(400.0 MHz, CDCl3): |
| δ= 7.9829 (2.9); 7.9815 (3.1); 7.9763 (3.2); 7.9748 (3.1); 7.5810 (3.1); 7.5744 (2.9); 7.5592 (3.2); 7.5526 (3.1); 7.2603 (26.9); 7.2297 (2.6); 7.2267 (2.9); 7.2239 (5.0); 7.2126 (5.1); 7.2083 (5.4); 7.1160 (1.5); 7.1128 (1.4); 7.1105 (1.5); 7.1073 (1.3); 7.0953 (1.5); 7.0909 (2.5); 7.0866 (1.2); 6.7563 (3.6); 6.7548 (3.6); 6.7345 (3.4); 6.7330 (3.5); 6.7269 (2.0); 6.5888 (4.2); 6.4507 (2.0); 6.3716 (2.4); 6.3691 (4.4); 6.3668 (2.4); 3.7764 (8.7); 3.7742 (16.0); 3.7719 (8.7); 1.5524 (3.8); 0.0080 (0.5); -0.0002 (18.8); -0.0085 (0.5) |
| 1-33: ¹H-NMR(400.0 MHz, CDCl3): |
| δ= 7.9413 (3.5); 7.9323 (2.3); 7.9284 (2.4); 7.9207 (5.2); 7.9163 (2.3); 7.5672 (1.4); 7.5632 (1.3); 7.5472 (1.6); 7.5430 (2.5); 7.5388 (1.4); 7.5228 (1.5); 7.5188 (1.5); 7.2596 (25.7); 7.1922 (1.4); 7.1905 (1.6); 7.1882 (1.7); 7.1866 (1.4); 7.1721 (1.3); 7.1703 (1.5); 7.1681 (1.6); 7.1665 (1.3); 7.0772 (1.5); 7.0691 (1.7); 7.0650 (1.9); 7.0572 (5.3); 7.0491 (1.5); 7.0451 (1.4); 7.0370 (1.3); 6.8825 (2.0); 6.8279 (5.5); 6.7479 (4.4); 6.6133 (2.2); 2.4172 (16.0); 1.5374 (11.0); 0.0079 (1.1); -0.0002 (33.2); -0.0085 (0.9) |
| 1-34: ¹H-NMR(400.6 MHz, CDCl3): |
| δ= 9.8785 (8.4); 7.9860 (2.8); 7.9847 (3.0); 7.9794 (2.9); 7.9782 (3.0); 7.5856 (2.6); 7.5790 (2.6); 7.5638 (2.8); 7.5572 (2.8); 7.5503 (1.2); 7.5343 (1.3); 7.5294 (2.3); 7.5135 (2.4); 7.5085 (1.4); 7.4926 (1.4); 7.2614 (14.2); 7.1472 (1.3); 7.1447 (1.5); 7.1255 (2.2); 7.1232 (2.4); 7.1041 (1.2); 7.1016 (1.3); 7.0663 (1.7); 7.0639 (2.9); 7.0615 (1.7); 7.0457 (1.5); 7.0432 (2.6); 7.0408 (1.6); 6.7742 (3.3); 6.7728 (3.5); 6.7581 (7.6); 6.7525 (3.4); 6.7511 (3.4); 5.2999 (0.7); 3.8406 (15.4); 3.8385 (16.0); 3.7919 (0.7); 3.1832 (0.6); 0.0079 (0.7); -0.0002 (21.1); -0.0084 (0.8) |
| 1-35: ¹H-NMR(400.6 MHz, CDCl3): |
| δ= 10.8079 (0.6); 8.1192 (7.5); 8.1181 (8.0); 8.1126 (8.0); 8.1115 (8.2); 8.0618 (5.8); 8.0576 (6.0); 8.0421 (6.2); 8.0379 (6.3); 7.7356 (8.9); 7.7289 (8.6); 7.7240 (0.7); 7.7170 (0.7); 7.7138 (9.4); 7.7072 (9.0); 7.5346 (3.3); 7.5304 (3.5); 7.5161 (4.9); 7.5141 (4.6); 7.5119 (5.1); 7.5099 (4.6); 7.4956 (5.2); 7.4913 (5.0); 7.3941 (5.0); 7.3912 (5.4); 7.3746 (6.2); 7.3718 (6.4); 7.3560 (3.8); 7.3530 (3.8); 7.2598 (45.0); 7.1964 (6.9); 7.1937 (6.8); 7.1758 (6.2); 7.1731 (5.9); 7.0153 (9.4); 7.0139 (10.1); 6.9936 (9.1); 6.9922 (9.5); 6.8930 (6.1); 6.7822 (16.0); 6.7588 (12.9); 6.6245 (6.5); 5.2987 (0.7); 1.5488 (10.8); 0.0080 (1.1); -0.0002 (41.4); -0.0085 (1.3) |
| 1-36: ¹H-NMR(400.6 MHz, CDCl3): |
| δ= 8.0620 (2.3); 8.0578 (2.5); 8.0423 (2.6); 8.0382 (2.7); 7.8819 (4.7); 7.8765 (5.0); 7.6029 (2.6); 7.5974 (2.7); 7.5804 (2.8); 7.5750 (2.7); 7.5491 (1.2); 7.5450 (1.4); 7.5292 (2.0); 7.5267 (2.1); 7.5185 (0.6); 7.5101 (1.9); 7.5059 (1.8); 7.4173 (1.7); 7.4145 (1.9); 7.3978 (2.7); 7.3950 (2.8); 7.3792 (1.4); 7.3764 (1.5); 7.2717 (3.2); 7.2690 (3.3); 7.2600 (35.2); 7.2512 (4.1); 6.9041 (2.2); 6.8680 (6.7); 6.7699 (4.5); 6.6357 (2.3); 2.5363 (0.8); 1.5407 (16.0); 0.0080 (1.3); -0.0002 (31.3); -0.0083 (2.2); -0.0281 (0.6) |
| 1-37: ¹H-NMR(400.0 MHz, CDCl3): |
| δ= 7.9696 (2.5); 7.9476 (2.6); 7.8952 (2.7); 7.8897 (2.8); 7.6004 (1.5); 7.5949 (1.4); 7.5780 (1.6); 7.5726 (1.4); 7.2598 (15.8); 6.9415 (1.4); 6.9353 (1.5); 6.9195 (1.3); 6.9132 (1.5); 6.8783 (1.1); 6.7711 (2.6); 6.7649 (2.5); 6.7437 (2.4); 6.7210 (3.4); 6.6091 (1.2); 3.8547 (16.0); 3.8333 (0.6); 1.5388 (1.9); -0.0002 (14.6); -0.0084 (0.7) |
| 1-38: ¹H-NMR(400.0 MHz, CDCl3): |
| δ= 8.2061 (4.2); 8.2016 (4.4); 8.1939 (4.5); 8.1893 (4.3); 8.0729 (5.3); 8.0688 (5.3); 8.0532 (5.6); 8.0491 (5.5); 7.7874 (2.6); 7.7824 (2.6); 7.7655 (4.1); 7.7486 (3.0); 7.7437 (2.8); 7.5216 (2.4); 7.5176 (2.6); 7.5019 (4.8); 7.4997 (4.9); 7.4978 (4.6); 7.4824 (3.6); 7.4784 (3.5); 7.3691 (3.8); 7.3674 (3.9); 7.3503 (6.3); 7.3491 (6.4); 7.3309 (2.9); 7.3292 (2.8); 7.2596 (24.8); 7.2091 (6.9); 7.1885 (6.1); 7.0825 (4.0); 7.0699 (4.2); 7.0646 (4.1); 7.0520 (3.8); 7.0396 (7.0); 7.0189 (6.6); 6.8872 (5.0); 6.8301 (16.0); 6.7527 (10.3); 6.6181 (5.2); 0.0080 (0.8); -0.0002 (23.8); -0.0083 (1.0) |
| 1-39: ¹H-NMR(400.0 MHz, CDCl3): |
| δ= 8.1350 (2.1); 8.1284 (2.3); 7.9730 (2.5); 7.9510 (2.6); 7.7336 (1.4); 7.7270 (1.4); 7.7119 (1.5); 7.7053 (1.5); 7.2597 (16.4); 7.0037 (2.4); 6.9820 (2.3); 6.9247 (1.4); 6.9186 (1.5); 6.9026 (1.3); 6.8965 (1.4); 6.8676 (1.2); 6.7329 (2.4); 6.6893 (2.7); 6.6831 (2.6); 6.6412 (3.8); 6.5983 (1.2); 3.8406 (16.0); 1.5350 (4.1); 0.0080 (0.6); -0.0002 (21.2) |
| 1-40: ¹H-NMR(599.7 MHz, d₆-DMSO): |
| δ= 8.2311 (0.7); 8.2272 (0.8); 8.1071 (0.6); 8.1029 (0.5); 8.0926 (0.6); 8.0883 (0.5); 7.7004 (0.4); 7.6896 (0.4); 7.3970 (0.4); 7.3673 (0.3); 7.2788 (0.8); 7.2487 (0.6); 7.2348 (0.5); 7.1903 (0.4); 7.1826 (0.8); 7.1680 (0.8); 7.1098 (0.9); 3.3334 (50.0); 2.5208 (0.4); 2.5177 (0.4); 2.5089 (5.3); 2.5060 (10.6); 2.5029 (14.4); 2.4999 (10.5); 2.4969 (5.0) |
| 1-42: ¹H-NMR(400.6 MHz, CDCl3): |
| δ= 8.1391 (8.4); 8.1378 (8.4); 8.1325 (9.1); 8.1312 (8.5); 7.9212 (12.9); 7.8995 (13.5); 7.7576 (8.3); 7.7510 (8.1); 7.7359 (8.8); 7.7292 (8.6); 7.5187 (1.7); 7.2985 (0.5); 7.2883 (1.1); 7.2713 (0.8); 7.2696 (0.8); 7.2603 (297.3); 7.2542 (2.3); 7.2535 (2.3); 7.0316 (10.0); 7.0302 (9.6); 7.0099 (9.5); 7.0085 (8.9); 6.9967 (1.7); 6.8700 (5.4); 6.8197 (8.1); 6.8135 (8.5); 6.7980 (7.7); 6.7919 (8.2); 6.7356 (12.0); 6.6401 (16.0); 6.6276 (14.3); 6.6215 (13.2); 6.6012 (5.9); 5.6331 (0.9); 1.5631 (2.1); 0.1459 (0.6); 0.0279 (0.6); 0.0080 (4.6); -0.0002 (181.6); -0.0085 (5.3); -0.1492 (0.6) |
| 1-43: ¹H-NMR(400.0 MHz, CDCl3): |
| δ= 7.9736 (4.8); 7.9515 (5.0); 7.8968 (5.4); 7.8913 (5.5); 7.6020 (2.9); 7.5965 (2.8); 7.5797 (3.0); 7.5742 (2.8); 7.2593 (25.1); 7.0036 (2.7); 6.9974 (2.9); 6.9815 (2.6); 6.9753 (2.9); 6.8811 (2.0); 6.8345 (5.0); 6.8283 (4.6); 6.7463 (5.0); 6.7426 (6.3); 6.6119 (2.1); 4.6972 (2.2); 4.6914 (6.6); 4.6855 (6.6); 4.6797 (2.2); 1.8668 (7.3); 1.8610 (16.0); 1.8551 (7.2); 1.5321 (2.5); 0.0080 (1.0); -0.0002 (32.9); -0.0085 (0.9) |
| 1-44: ¹H-NMR(400.0 MHz, CDCl3): |
| δ= 7.9594 (12.6); 7.9373 (13.2); 7.8995 (13.6); 7.8940 (14.0); 7.5986 (7.5); 7.5932 (7.1); 7.5763 (7.5); 7.5708 (7.1); 7.2589 (36.0); 6.9443 (6.9); 6.9381 (7.3); 6.9222 (6.5); 6.9160 (7.1); 6.8782 (5.4); 6.7857 (13.0); 6.7796 (12.0); 6.7435 (11.8); 6.7287 (16.0); 6.6089 (5.8); 6.0824 (1.4); 6.0691 (3.0); 6.0560 (2.9); 6.0428 (3.4); 6.0393 (1.8); 6.0295 (1.8); 6.0260 (3.6); 6.0128 (3.4); 5.9996 (3.7); 5.9863 (1.8); 5.4457 (2.0); 5.4418 (5.4); 5.4382 (5.5); 5.4343 (2.1); 5.4026 (1.8); 5.3987 (4.7); 5.3950 (4.8); 5.3911 (1.8); 5.3406 (2.2); 5.3373 (6.0); 5.3339 (5.8); 5.3306 (2.0); 5.3143 (2.1); 5.3110 (5.6); 5.3076 (5.5); 5.3043 (1.9); 5.2977 (3.8); 4.5849 (7.2); 4.5812 (12.5); 4.5775 (7.3); 4.5716 (7.2); 4.5679 (12.4); 4.5642 (7.0); 2.6028 (0.9); 1.5380 (2.3); 0.0080 (1.2); -0.0002 (36.2); -0.0085 (1.0) |
| 1-45: ¹H-NMR(400.0 MHz, CDCl3): |
| δ= 8.1388 (3.4); 8.1375 (3.6); 8.1322 (3.6); 8.1308 (3.5); 7.9558 (5.2); 7.9337 (5.5); 7.7269 (3.4); 7.7202 (3.2); 7.7052 (3.6); 7.6985 (3.5); 7.2594 (18.6); 6.9946 (4.1); 6.9932 (4.1); 6.9728 (3.8); 6.9714 (3.8); 6.9016 (2.9); 6.8954 (3.0); 6.8795 (2.8); 6.8733 (3.0); 6.8651 (2.2); 6.7305 (4.9); 6.6682 (5.2); 6.6621 (5.0); 6.6395 (6.4); 6.5958 (2.4); 4.0818 (2.1); 4.0643 (7.2); 4.0468 (7.2); 4.0293 (2.2); 3.9474 (0.5); 1.4384 (7.6); 1.4210 (16.0); 1.4035 (7.4); 0.0080 (0.5); -0.0002 (18.9); -0.0085 (0.5) |
| 1-46: ¹H-NMR(400.0 MHz, CDCl3): |
| δ= 7.9527 (5.3); 7.9307 (5.5); 7.8988 (5.8); 7.8933 (5.9); 7.5961 (3.2); 7.5906 (3.0); 7.5737 (3.2); 7.5683 (3.0); 7.2592 (41.5); 6.9181 (2.9); 6.9120 (3.1); 6.8961 (2.8); 6.8899 (3.0); 6.8761 (2.3); 6.7455 (6.0); 6.7412 (6.7); 6.7398 (6.5); 6.7169 (7.2); 6.6069 (2.4); 4.0973 (2.2); 4.0798 (7.3); 4.0623 (7.5); 4.0449 (2.4); 2.5959 (0.6); 1.5323 (3.1); 1.4481 (7.7); 1.4307 (16.0); 1.4132 (7.4); 1.2643 (0.7); 0.8820 (1.0); 0.0080 (2.2); -0.0002 (55.6); -0.0085 (2.0) |
| 1-47: ¹H-NMR(400.0 MHz, CDCl3): |
| δ= 8.0765 (5.8); 8.0723 (5.9); 8.0568 (6.3); 8.0526 (6.1); 7.9300 (6.1); 7.9262 (6.3); 7.9179 (6.3); 7.9140 (6.4); 7.5788 (4.0); 7.5749 (4.0); 7.5589 (4.4); 7.5547 (7.3); 7.5505 (4.1); 7.5427 (3.5); 7.5384 (3.6); 7.5344 (4.5); 7.5305 (4.4); 7.5241 (4.8); 7.5220 (4.7); 7.5198 (5.2); 7.5178 (4.4); 7.5035 (5.1); 7.4992 (4.9); 7.3986 (4.8); 7.3956 (5.2); 7.3790 (6.0); 7.3761 (6.3); 7.3603 (3.8); 7.3574 (3.7); 7.2887 (7.1); 7.2863 (6.8); 7.2680 (6.3); 7.2654 (6.3); 7.2609 (52.9); 7.0900 (4.3); 7.0818 (4.5); 7.0779 (4.3); 7.0699 (7.7); 7.0619 (4.0); 7.0579 (4.0); 7.0497 (3.7); 6.9176 (16.0); 6.9027 (6.1); 6.7682 (12.8); 6.6337 (6.4); 1.5467 (13.7); 0.0080 (2.2); -0.0002 (70.2); -0.0085 (1.8) |
| 1-48: ¹H-NMR(400.0 MHz, CDCl3): |
| δ= 7.9836 (0.5); 7.9794 (0.6); 7.9725 (0.7); 7.9645 (0.6); 7.9599 (0.6); 7.9202 (7.2); 7.9158 (7.2); 7.9007 (9.5); 7.8966 (12.2); 7.8882 (0.9); 7.8777 (9.1); 7.8689 (0.7); 7.8454 (5.7); 7.8445 (5.5); 7.8253 (8.1); 7.8065 (3.9); 7.7978 (15.9); 7.7923 (16.0); 7.6090 (0.6); 7.5915 (0.6); 7.5855 (0.6); 7.5832 (0.6); 7.5594 (8.4); 7.5568 (8.1); 7.5404 (7.4); 7.5378 (7.0); 7.5206 (3.4); 7.5161 (3.7); 7.5020 (5.8); 7.5006 (5.2); 7.4976 (5.9); 7.4961 (5.0); 7.4819 (6.0); 7.4774 (5.8); 7.4578 (8.8); 7.4523 (8.4); 7.4351 (9.1); 7.4296 (8.6); 7.4230 (5.8); 7.4198 (6.1); 7.4139 (0.9); 7.4039 (7.9); 7.4008 (8.2); 7.3851 (3.9); 7.3821 (3.8); 7.2561 (12.2); 7.2424 (8.2); 7.2395 (7.9); 7.2222 (6.9); 7.2193 (6.5); 7.2094 (0.6); 7.2037 (0.5); 7.1885 (0.5); 7.0908 (0.5); 7.0876 (0.6); 5.2938 (5.6); 3.8782 (5.3); 1.5516 (0.8); 0.0080 (0.5); - 0.0002 (17.1) |
| 1-49: ¹H-NMR(400.0 MHz, CDCl3): |
| δ= 8.0688 (12.7); 8.0675 (13.3); 8.0621 (13.3); 8.0609 (13.0); 8.0516 (0.6); 8.0454 (0.5); 7.9380 (8.8); 7.9336 (8.9); 7.9187 (9.8); 7.9143 (9.6); 7.9024 (0.5); 7.8794 (7.0); 7.8602 (11.7); 7.8313 (0.5); 7.8122 (6.6); 7.8111 (6.7); 7.7920 (10.7); 7.7731 (4.6); 7.7719 (4.6); 7.5868 (13.2); 7.5801 (12.2); 7.5650 (13.7); 7.5581 (16.0); 7.5540 (11.4); 7.5372 (9.5); 7.5349 (9.4); 7.5063 (4.6); 7.5018 (4.9); 7.4878 (7.6); 7.4863 (6.6); 7.4833 (7.4); 7.4817 (6.6); 7.4676 (7.8); 7.4631 (7.2); 7.4066 (7.4); 7.4035 (8.1); 7.3875 (10.0); 7.3845 (10.6); 7.3688 (5.0); 7.3657 (4.8); 7.2590 (34.0); 7.1872 (10.4); 7.1845 (10.4); 7.1670 (9.0); 7.1643 (8.8); 6.7924 (15.0); 6.7910 (15.4); 6.7705 (14.2); 6.7692 (14.5); 5.2975 (1.2); 3.8808 (4.5); 1.5512 (6.7); 0.0080 (1.3); -0.0002 (48.0); -0.0085 (1.3) |
| 1-50: ¹H-NMR(400.6 MHz, CDCl3): |
| δ= 13.8235 (2.5); 8.1633 (6.4); 8.1614 (6.9); 8.1583 (7.3); 8.1563 (7.1); 8.1509 (7.0); 8.1490 (7.3); 8.1458 (7.5); 8.1440 (7.1); 8.1315 (0.8); 8.1104 (1.0); 8.0832 (0.7); 8.0631 (0.8); 7.9740 (10.1); 7.9695 (10.4); 7.9546 (11.3); 7.9497 (16.0); 7.9276 (11.8); 7.8082 (1.0); 7.8044 (1.1); 7.7902 (6.4); 7.7887 (7.0); 7.7695 (11.8); 7.7509 (6.0); 7.7494 (6.0); 7.6778 (0.9); 7.6635 (0.9); 7.6594 (1.4); 7.6420 (7.4); 7.6369 (7.2); 7.6240 (8.0); 7.6213 (8.1); 7.6190 (8.2); 7.6162 (8.0); 7.6033 (8.1); 7.5982 (7.8); 7.5381 (12.4); 7.5359 (12.9); 7.5286 (0.7); 7.5188 (10.8); 7.5166 (10.8); 7.4933 (5.7); 7.4889 (6.0); 7.4749 (9.0); 7.4732 (7.6); 7.4704 (8.8); 7.4687 (7.6); 7.4547 (9.6); 7.4502 (9.1); 7.3868 (8.6); 7.3837 (9.6); 7.3679 (11.1); 7.3648 (12.0); 7.3491 (6.2); 7.3460 (6.1); 7.2593 (36.7); 7.1936 (11.6); 7.1906 (12.0); 7.1734 (10.0); 7.1704 (10.2); 6.9619 (7.7); 6.9596 (8.2); 6.9494 (7.6); 6.9472 (8.2); 6.9439 (8.0); 6.9416 (7.8); 6.9315 (7.5); 6.9292 (7.7); 6.8811 (1.1); 6.8613 (2.1); 6.8414 (1.2); 6.8246 (8.3); 6.8225 (15.1); 6.8204 (8.4); 6.8039 (7.8); 6.8018 (14.4); 6.7997 (7.9); 1.5673 (2.5); 1.3342 (0.6); 1.2843 (0.9); 1.2550 (1.7); 0.0712 (2.0); 0.0079 (1.4); -0.0002 (53.4); -0.0085 (1.8) |
| 1-51: ¹H-NMR(400.6 MHz, CDCl3): |
| δ= 13.8220 (0.5); 7.9911 (2.2); 7.9870 (3.6); 7.9829 (4.1); 7.9785 (2.5); 7.9768 (1.9); 7.9711 (2.7); 7.9684 (3.2); 7.9636 (2.4); 7.8862 (0.5); 7.8088 (0.6); 7.7902 (1.5); 7.7856 (0.7); 7.7716 (2.4); 7.7638 (0.6); 7.7523 (1.3); 7.7510 (1.3); 7.6647 (0.7); 7.6614 (0.6); 7.6453 (0.5); 7.6237 (0.5); 7.5494 (0.6); 7.5451 (2.7); 7.5430 (2.7); 7.5258 (2.5); 7.5237 (2.3); 7.4752 (0.6); 7.4675 (1.2); 7.4630 (1.2); 7.4556 (0.6); 7.4491 (3.1); 7.4445 (2.6); 7.4430 (2.8); 7.4288 (3.2); 7.4243 (2.5); 7.4225 (1.7); 7.3626 (1.7); 7.3595 (1.8); 7.3436 (2.2); 7.3405 (2.3); 7.3249 (1.2); 7.3218 (1.2); 7.2598 (20.4); 7.1437 (2.4); 7.1409 (2.4); 7.1350 (0.9); 7.1234 (2.1); 7.1207 (2.1); 6.8630 (0.6); 6.7269 (2.9); 6.7060 (2.7); 2.7713 (0.5); 2.3109 (2.8); 2.2660 (2.0); 2.2643 (1.9); 2.2468 (16.0); 1.5605 (3.6); 1.2546 (0.8); 0.0702 (0.8); 0.0080 (0.7); -0.0002 (27.7); -0.0085 (0.8) |
| 1-52: ¹H-NMR(400.6 MHz, CDCl3): |
| δ= 8.6012 (2.9); 8.5993 (3.1); 8.5948 (3.1); 8.5930 (3.1); 7.9800 (1.5); 7.9781 (2.3); 7.9762 (2.0); 7.9739 (2.0); 7.9719 (2.4); 7.9701 (1.7); 7.8943 (2.1); 7.8900 (2.3); 7.8749 (2.4); 7.8706 (2.4); 7.8191 (0.5); 7.7550 (3.2); 7.7532 (3.2); 7.7338 (4.1); 7.7319 (4.2); 7.5888 (4.1); 7.5824 (4.0); 7.5675 (3.2); 7.5612 (3.2); 7.4474 (1.4); 7.4429 (1.6); 7.4403 (1.6); 7.4387 (1.5); 7.4340 (1.5); 7.4325 (1.5); 7.4290 (2.2); 7.4272 (1.8); 7.4245 (2.1); 7.4227 (1.9); 7.4195 (1.7); 7.4179 (1.6); 7.4132 (1.6); 7.4116 (1.6); 7.4089 (2.6); 7.4043 (2.2); 7.3456 (2.0); 7.3424 (2.3); 7.3264 (2.6); 7.3233 (2.8); 7.3079 (1.5); 7.3048 (1.6); 7.2603 (74.0); 7.2323 (0.5); 7.1353 (2.5); 7.1326 (2.6); 7.1150 (2.2); 7.1124 (2.1); 6.7004 (2.8); 6.6794 (2.6); 2.2453 (16.0); 1.5562 (1.1); 0.0080 (2.8); 0.0047 (0.8); -0.0002 (105.5); -0.0068 (1.4); -0.0085 (3.5); -0.0282 (0.8) |
| 1-53: ¹H-NMR(599.6 MHz, d₆-DMSO): |
| δ= 8.1790 (6.9); 8.1746 (7.2); 7.9425 (4.4); 7.9380 (4.2); 7.9279 (4.5); 7.9234 (4.4); 7.7623 (5.0); 7.7488 (6.1); 7.6246 (4.4); 7.6109 (8.2); 7.5972 (4.2); 7.4087 (5.9); 7.3950 (5.3); 7.3636 (2.8); 7.2751 (6.5); 7.1867 (3.1); 7.0533 (7.5); 7.0386 (7.2); 7.0031 (12.8); 3.3161 (15.2); 2.6144 (0.3); 2.5231 (0.9); 2.5201 (1.1); 2.5170 (1.2); 2.5079 (18.7); 2.5052 (37.1); 2.5022 (50.0); 2.4993 (37.5); 1.2762 (0.4); 1.2651 (0.5); 1.2449 (1.1); 0.8693 (1.0); 0.8579 (2.4); 0.8460 (1.1); 0.0051 (0.6); -0.0001 (14.5); -0.0055 (0.5) |
| 1-54: ¹H-NMR(400.6 MHz, CDCl3): |
| δ= 8.0362 (14.0); 8.0150 (15.2); 7.9833 (10.2); 7.9818 (9.8); 7.9766 (11.1); 7.9751 (10.0); 7.6998 (10.0); 7.6932 (9.6); 7.6780 (10.6); 7.6714 (10.4); 7.5297 (9.5); 7.5249 (10.7); 7.5085 (8.7); 7.5037 (10.4); 7.4500 (16.0); 7.4453 (14.1); 7.4393 (0.5); 7.3349 (5.1); 7.3340 (5.0); 7.3286 (10.3); 7.3276 (9.4); 7.3221 (5.4); 7.2598 (31.1); 7.0412 (12.1); 7.0396 (11.4); 7.0194 (11.3); 7.0178 (10.4); 6.7417 (5.2); 6.7407 (5.0); 6.6074 (11.2); 6.6063 (10.5); 6.4732 (5.6); 6.4722 (5.2); 1.5540 (7.2); 0.8817 (0.8); 0.0079 (1.2); -0.0002 (41.6); -0.0085 (1.2) |
| 1-56: ¹H-NMR(599.6 MHz, d₆-DMSO): |
| δ= 8.1640 (2.2); 8.1602 (2.3); 7.9195 (1.6); 7.9149 (1.6); 7.9048 (1.7); 7.9003 (1.6); 7.6345 (1.3); 7.6210 (2.8); 7.6074 (1.6); 7.3490 (2.0); 7.3448 (1.0); 7.3361 (1.7); 7.2562 (2.2); 7.1677 (1.0); 7.1122 (1.9); 7.1110 (2.0); 7.0985 (1.9); 7.0973 (1.8); 7.0173 (2.4); 7.0168 (2.4); 7.0027 (2.3); 7.0021 (2.3); 6.9031 (4.3); 3.3133 (13.5); 2.5220 (0.8); 2.5189 (1.0); 2.5158 (1.1); 2.5069 (14.1); 2.5040 (29.4); 2.5011 (50.0); 2.4980 (29.7); 2.4952 (14.2); 2.4281 (0.7); 1.2653 (0.3); 1.2454 (0.7); 0.8698 (0.6); 0.8583 (1.7); 0.8464 (0.8); 0.0053 (0.5); -0.0001 (13.1); -0.0056 (0.4) |
| 1-57: ¹H-NMR(400.6 MHz, CDCl3): |
| δ= 8.1722 (7.8); 8.1681 (8.1); 8.1526 (8.4); 8.1483 (8.4); 7.9833 (13.0); 7.9818 (12.1); 7.9766 (13.8); 7.9751 (12.1); 7.6770 (13.0); 7.6704 (12.3); 7.6552 (13.8); 7.6486 (13.2); 7.5912 (4.5); 7.5869 (4.6); 7.5726 (6.8); 7.5708 (6.4); 7.5683 (6.9); 7.5666 (5.8); 7.5523 (6.4); 7.5480 (6.1); 7.4062 (6.8); 7.4032 (7.2); 7.3866 (8.7); 7.3845 (8.7); 7.3839 (8.3); 7.3680 (5.6); 7.3651 (5.4); 7.3382 (6.4); 7.3372 (6.2); 7.3318 (12.9); 7.3307 (11.7); 7.3253 (6.6); 7.2767 (9.7); 7.2743 (9.4); 7.2597 (56.7); 7.2569 (9.6); 7.2562 (9.5); 7.2540 (8.5); 7.0215 (16.0); 7.0199 (14.4); 6.9997 (15.1); 6.9981 (13.5); 6.7481 (6.9); 6.7470 (6.4); 6.6137 (14.6); 6.6126 (13.4); 6.4794 (7.3); 6.4782 (6.6); 1.5522 (11.9); 1.2561 (0.5); 0.0079 (2.2); -0.0002 (76.3); -0.0085 (2.0) |
| 1-58: ¹H-NMR(400.6 MHz, CDCl3): |
| δ= 8.2856 (5.9); 8.2650 (7.0); 8.0817 (5.6); 8.0612 (4.9); 7.9723 (11.7); 7.9617 (9.7); 7.7156 (5.0); 7.7091 (5.1); 7.6939 (5.7); 7.6874 (5.4); 7.5185 (1.1); 7.3994 (5.3); 7.3932 (9.1); 7.3873 (5.7); 7.2601 (139.5); 7.0813 (8.0); 7.0595 (7.5); 7.0160 (0.8); 6.9964 (1.4); 6.7723 (4.4); 6.7297 (0.6); 6.6383 (9.2); 6.5040 (4.5); 5.2999 (1.2); 2.3944 (0.7); 2.3764 (1.2); 2.3570 (0.7); 1.6531 (0.7); 1.3331 (2.3); 1.2841 (3.6); 1.2552 (16.0); 0.8792 (2.6); 0.8619 (1.3); 0.1458 (0.9); 0.0692 (0.6); 0.0079 (9.2); -0.0002 (191.0); -0.0085 (13.2); -0.0318 (1.7); -0.1496 (0.8) |
| 1-59: ¹H-NMR(599.6 MHz, d₆-DMSO): |
| δ= 8.1516 (1.2); 8.1471 (1.2); 7.9111 (0.8); 7.9065 (0.8); 7.8964 (0.9); 7.8919 (0.8); 7.5913 (0.6); 7.5780 (1.3); 7.5647 (0.8); 7.3454 (1.0); 7.3391 (0.6); 7.3326 (0.9); 7.2506 (1.2); 7.1627 (1.4); 7.1498 (0.9); 7.0003 (1.3); 6.9856 (1.3); 6.8860 (2.3); 3.3124 (21.2); 2.6128 (0.3); 2.5713 (0.6); 2.5586 (1.6); 2.5460 (1.7); 2.5334 (0.8); 2.5217 (1.0); 2.5187 (1.2); 2.5155 (1.4); 2.5037 (36.8); 2.5007 (50.0); 2.4977 (37.8); 1.1062 (2.0); 1.0937 (4.2); 1.0810 (1.9); 0.0053 (0.5); -0.0001 (13.4); -0.0056 (0.5) |
| 1-61: ¹H-NMR(400.6 MHz, CDCl3): |
| δ= 7.7885 (4.6); 7.7830 (4.7); 7.4670 (2.6); 7.4615 (2.5); 7.4444 (2.6); 7.4389 (2.5); 7.4039 (1.0); 7.3886 (1.1); 7.3832 (2.1); 7.3679 (2.1); 7.3625 (1.4); 7.3472 (1.3); 7.2598 (26.5); 7.1246 (1.2); 7.1218 (1.4); 7.1036 (1.2); 7.1004 (1.8); 7.0971 (1.5); 7.0789 (1.1); 7.0760 (1.2); 7.0726 (1.6); 7.0698 (2.6); 7.0670 (1.3); 7.0522 (1.3); 7.0493 (2.3); 7.0465 (1.2); 6.8174 (1.8); 6.7001 (0.9); 6.6961 (2.0); 6.6909 (2.0); 6.6870 (1.1); 6.6832 (3.9); 6.5489 (1.9); 3.9548 (0.5); 3.8968 (16.0); 2.6626 (0.5); 2.6447 (0.5); 2.3180 (0.5); 1.5415 (8.7); 0.0080 (0.9); -0.0002 (36.5); -0.0085 (1.1) |
| 1-62: ¹H-NMR(400.6 MHz, CDCl3): |
| δ= 8.1380 (9.0); 8.1365 (9.0); 8.1314 (10.0); 8.1299 (9.4); 7.9194 (13.3); 7.8982 (14.8); 7.7728 (9.9); 7.7661 (9.6); 7.7511 (10.5); 7.7445 (9.9); 7.5182 (0.5); 7.5146 (10.0); 7.5099 (10.4); 7.4935 (8.9); 7.4887 (9.8); 7.3585 (14.2); 7.3539 (13.4); 7.3476 (0.5); 7.2599 (41.0); 7.0551 (11.5); 7.0535 (11.4); 7.0334 (10.7); 7.0318 (10.7); 6.8932 (5.8); 6.8842 (0.6); 6.7777 (16.0); 6.7591 (12.7); 6.6249 (6.3); 1.5425 (0.9); 0.0080 (1.5); -0.0002 (55.6); -0.0058 (0.7); - 0.0085 (1.6) |
| 1-63: ¹H-NMR(400.6 MHz, CDCl3): |
| δ= 8.0627 (2.1); 8.0418 (2.2); 7.9836 (1.5); 7.9821 (1.5); 7.9769 (1.6); 7.9754 (1.5); 7.6775 (1.6); 7.6709 (1.6); 7.6557 (1.7); 7.6491 (1.7); 7.3023 (0.7); 7.3014 (0.8); 7.2959 (1.5); 7.2949 (1.4); 7.2893 (0.8); 7.2600 (7.6); 7.2169 (1.4); 7.2121 (1.4); 7.1960 (1.3); 7.1912 (1.4); 7.0704 (2.1); 7.0657 (2.0); 7.0156 (1.8); 7.0140 (1.9); 6.9938 (1.7); 6.9922 (1.7); 6.7259 (0.8); 6.7248 (0.8); 6.5916 (1.7); 6.5903 (1.7); 6.4572 (0.8); 6.4560 (0.8); 2.5174 (16.0); -0.0002 (10.8) |
| 1-64: ¹H-NMR(400.6 MHz, CDCl3): |
| δ= 8.0475 (2.5); 8.0461 (2.8); 8.0409 (2.7); 8.0394 (2.8); 7.6087 (2.7); 7.6020 (2.6); 7.5869 (2.8); 7.5802 (2.8); 7.3874 (1.0); 7.3720 (1.1); 7.3667 (2.2); 7.3513 (2.2); 7.3460 (1.4); 7.3305 (1.3); 7.2600 (8.4); 7.0954 (1.2); 7.0926 (1.4); 7.0745 (1.2); 7.0714 (2.0); 7.0683 (1.5); 7.0501 (1.1); 7.0473 (1.2); 7.0015 (1.4); 6.9987 (2.5); 6.9960 (1.4); 6.9810 (1.3); 6.9782 (2.3); 6.9755 (1.2); 6.8397 (3.1); 6.8382 (3.2); 6.8179 (3.0); 6.8164 (3.0); 6.8094 (1.9); 6.6751 (3.9); 6.6277 (1.0); 6.6238 (2.6); 6.6198 (2.6); 6.6159 (1.0); 6.5408 (1.9); 5.2980 (2.2); 3.9217 (16.0); 1.5679 (2.7); - 0.0002 (11.2) |
| 1-65: ¹H-NMR(400.6 MHz, CDCl3): |
| δ= 8.0907 (2.7); 8.0893 (2.6); 8.0841 (2.8); 8.0827 (2.6); 7.7593 (1.3); 7.7578 (1.3); 7.7523 (1.4); 7.7508 (1.3); 7.7355 (1.4); 7.7343 (1.4); 7.7282 (1.4); 7.6364 (2.5); 7.6297 (2.5); 7.6146 (2.7); 7.6079 (2.6); 7.2596 (6.8); 7.1041 (0.5); 7.0927 (0.6); 7.0914 (0.7); 7.0834 (2.3); 7.0819 (2.1); 7.0744 (2.1); 7.0704 (2.5); 7.0691 (2.5); 7.0676 (2.2); 7.0565 (1.9); 7.0520 (0.6); 7.0493 (1.7); 7.0452 (0.5); 7.0270 (0.5); 6.8475 (3.2); 6.8461 (3.0); 6.8257 (3.0); 6.8242 (2.8); 6.7857 (1.8); 6.7470 (2.0); 6.7433 (3.7); 6.7397 (2.0); 6.6516 (3.7); 6.5174 (1.8); 5.2980 (3.0); 3.9772 (16.0); 1.5607 (2.0); -0.0002 (9.2) |
| 1-66: ¹H-NMR(400.6 MHz, CDCl3): |
| δ= 8.8992 (4.4); 8.8969 (6.4); 8.8942 (7.3); 8.8919 (6.6); 7.9240 (6.3); 7.9196 (6.5); 7.9046 (7.4); 7.9003 (7.7); 7.8813 (8.4); 7.8754 (10.9); 7.8704 (11.4); 7.8510 (1.6); 7.8065 (15.6); 7.8010 (16.0); 7.5389 (3.3); 7.5344 (3.4); 7.5203 (5.8); 7.5187 (4.5); 7.5159 (5.4); 7.5142 (4.3); 7.5002 (5.6); 7.4957 (5.3); 7.4800 (8.7); 7.4745 (8.3); 7.4574 (8.6); 7.4519 (8.5); 7.4376 (5.3); 7.4345 (5.7); 7.4188 (6.9); 7.4157 (7.2); 7.3998 (3.7); 7.3968 (3.4); 7.2618 (34.5); 7.2370 (7.4); 7.2341 (7.6); 7.2168 (6.4); 7.2138 (6.4); 2.0125 (6.3); 1.5987 (0.7); 0.0079 (1.3); -0.0002 (47.6); - 0.0052 (0.7); -0.0060 (0.6); -0.0085 (1.5) |
| 1-67: ¹H-NMR(400.6 MHz, CDCl3): |
| δ= 8.9083 (5.9); 8.9061 (8.7); 8.9031 (9.0); 8.9006 (9.1); 8.8986 (5.8); 8.0791 (13.3); 8.0777 (13.4); 8.0725 (14.3); 8.0710 (13.4); 7.9164 (8.4); 7.9120 (8.9); 7.8970 (9.4); 7.8926 (9.5); 7.8717 (2.9); 7.8706 (2.7); 7.8660 (2.8); 7.8507 (8.7); 7.8497 (9.0); 7.8450 (9.6); 7.8441 (9.5); 7.8311 (13.7); 7.8104 (4.0); 7.6090 (13.6); 7.6023 (13.4); 7.5872 (14.3); 7.5805 (14.1); 7.5257 (4.8); 7.5213 (5.1); 7.5072 (7.5); 7.5055 (6.4); 7.5028 (7.4); 7.5011 (6.3); 7.4870 (7.8); 7.4825 (7.4); 7.4179 (7.3); 7.4149 (8.0); 7.3989 (9.4); 7.3959 (9.8); 7.3802 (5.3); 7.3771 (5.1); 7.2622 (29.8); 7.1887 (9.8); 7.1857 (10.2); 7.1685 (8.7); 7.1680 (8.7); 7.1655 (8.7); 6.8143 (16.0); 6.8128 (15.7); 6.7925 (15.1); 6.7910 (14.8); 2.0111 (5.4); 1.6208 (0.8); 0.0079 (1.1); -0.0002 (40.8); -0.0085 (1.2) |
| 1-68: ¹H-NMR(400.6 MHz, CDCl3): |
| δ= 8.9081 (11.0); 8.9050 (10.9); 8.9026 (11.2); 8.1736 (6.7); 8.1718 (7.5); 8.1686 (7.9); 8.1668 (7.6); 8.1612 (7.3); 8.1594 (8.0); 8.1562 (7.9); 8.1543 (7.6); 7.9359 (10.5); 7.9315 (11.0); 7.9166 (11.8); 7.9122 (11.7); 7.8970 (6.6); 7.8762 (16.0); 7.8519 (10.0); 7.8507 (10.2); 7.8462 (10.1); 7.8449 (10.0); 7.8298 (4.3); 7.8240 (4.3); 7.6661 (7.3); 7.6611 (7.5); 7.6481 (8.3); 7.6454 (8.7); 7.6431 (8.8); 7.6404 (8.5); 7.6274 (8.5); 7.6223 (8.2); 7.5182 (6.0); 7.5138 (6.2); 7.4998 (8.9); 7.4980 (8.2); 7.4953 (9.2); 7.4936 (8.2); 7.4795 (9.5); 7.4751 (9.2); 7.4008 (9.1); 7.3977 (9.9); 7.3816 (11.7); 7.3787 (12.6); 7.3631 (6.7); 7.3600 (6.7); 7.2624 (176.5); 7.2343 (0.6); 7.2025 (12.6); 7.1998 (12.5); 7.1822 (11.2); 7.1794 (10.7); 6.9987 (1.0); 6.9863 (7.9); 6.9841 (8.5); 6.9739 (7.8); 6.9716 (8.7); 6.9683 (8.4); 6.9660 (8.3); 6.9559 (7.7); 6.9536 (7.8); 6.8423 (8.4); 6.8403 (15.6); 6.8383 (9.2); 6.8216 (8.3); 6.8196 (15.0); 6.8176 (8.6); 3.8884 (2.8); 2.0337 (0.5); 0.1456 (0.8); 0.0080 (5.8); -0.0002 (237.8); -0.0085 (7.6); -0.0283 (0.8); -0.1495 (0.7) |
| 1-69: ¹H-NMR(400.6 MHz, CDCl3): |
| δ= 8.8119 (6.2); 8.7992 (6.3); 7.9962 (7.3); 7.9943 (8.2); 7.9924 (7.4); 7.9346 (5.9); 7.9304 (6.0); 7.9153 (6.5); 7.9111 (6.4); 7.7687 (15.3); 7.7632 (16.0); 7.5461 (3.2); 7.5417 (3.4); 7.5276 (5.4); 7.5260 (4.5); 7.5232 (5.2); 7.5215 (4.7); 7.5075 (5.8); 7.5030 (5.3); 7.4549 (8.9); 7.4494 (8.6); 7.4434 (5.7); 7.4403 (6.2); 7.4324 (9.1); 7.4269 (9.4); 7.4245 (7.5); 7.4213 (7.5); 7.4056 (3.9); 7.4025 (4.0); 7.3991 (4.0); 7.3975 (4.4); 7.3950 (4.4); 7.3935 (3.9); 7.3864 (3.9); 7.3848 (4.3); 7.3823 (4.3); 7.3807 (3.7); 7.2914 (7.1); 7.2887 (7.1); 7.2711 (6.1); 7.2685 (6.0); 7.2618 (33.7); 0.0080 (1.2); -0.0002 (46.5); -0.0085 (1.4) |
| 1-70: ¹H-NMR(400.6 MHz, CDCl3): |
| δ= 8.8156 (8.8); 8.8028 (9.0); 8.0214 (12.7); 8.0199 (13.8); 8.0148 (13.6); 8.0133 (13.7); 7.9348 (6.8); 7.9330 (10.8); 7.9310 (12.4); 7.9291 (11.3); 7.9230 (9.2); 7.9184 (9.0); 7.9036 (9.6); 7.8993 (9.4); 7.5932 (14.3); 7.5865 (13.9); 7.5714 (14.9); 7.5647 (14.4); 7.5303 (4.9); 7.5259 (5.3); 7.5206 (0.6); 7.5119 (7.8); 7.5102 (6.7); 7.5074 (7.7); 7.5057 (7.0); 7.4917 (8.4); 7.4872 (7.9); 7.4206 (7.7); 7.4175 (8.6); 7.4014 (9.6); 7.3984 (10.4); 7.3861 (5.8); 7.3828 (10.8); 7.3799 (10.2); 7.3733 (5.7); 7.3718 (6.4); 7.3693 (6.3); 7.3677 (5.7); 7.2620 (42.4); 7.2564 (0.6); 7.2557 (0.6); 7.2272 (10.3); 7.2244 (9.9); 7.2069 (9.0); 7.2042 (8.4); 6.8312 (15.0); 6.8296 (16.0); 6.8094 (14.3); 6.8079 (14.8); 0.0079 (1.4); -0.0002 (57.9); -0.0052 (0.9); -0.0060 (0.8); -0.0068 (0.7); -0.0085 (1.8) |
| 1-71: ¹H-NMR(400.6 MHz, CDCl3): |
| δ= 8.8141 (9.8); 8.8013 (10.0); 8.1137 (6.8); 8.1118 (7.4); 8.1087 (7.6); 8.1068 (7.2); 8.1013 (7.3); 8.0994 (7.7); 8.0963 (7.7); 8.0944 (7.3); 7.9684 (12.6); 7.9664 (14.2); 7.9644 (12.7); 7.9349 (10.2); 7.9310 (10.5); 7.9155 (11.1); 7.9116 (11.1); 7.6512 (7.6); 7.6461 (7.5); 7.6332 (8.5); 7.6305 (8.8); 7.6281 (8.8); 7.6254 (8.5); 7.6125 (8.6); 7.6074 (8.4); 7.5237 (6.2); 7.5193 (6.4); 7.5053 (8.9); 7.5035 (8.2); 7.5008 (8.9); 7.4991 (8.2); 7.4851 (9.8); 7.4806 (9.4); 7.4015 (9.1); 7.3984 (10.0); 7.3824 (10.9); 7.3794 (11.9); 7.3636 (11.9); 7.3608 (13.0); 7.3575 (7.0); 7.3504 (6.6); 7.3488 (7.3); 7.3463 (7.2); 7.3448 (6.4); 7.2690 (0.8); 7.2673 (1.4); 7.2666 (1.7); 7.2657 (2.3); 7.2623 (156.0); 7.2566 (1.8); 7.2457 (12.2); 7.2432 (11.9); 7.2341 (1.3); 7.2260 (10.0); 7.2254 (10.5); 7.2229 (9.9); 6.9987 (0.8); 6.9441 (8.2); 6.9418 (8.9); 6.9317 (8.1); 6.9294 (9.3); 6.9261 (8.6); 6.9238 (8.7); 6.9137 (7.8); 6.9114 (8.2); 6.8696 (8.7); 6.8675 (16.0); 6.8654 (8.7); 6.8489 (8.6); 6.8468 (15.3); 6.8447 (8.2); 1.6881 (1.1); 0.1456 (0.6); 0.0128 (0.5); 0.0105 (0.6); 0.0080 (5.2); -0.0002 (215.5); -0.0061 (2.6); -0.0085 (6.6); -0.0284 (1.5); -0.1496 (0.6) |
| 1-72: ¹H-NMR(400.6 MHz, CDCl3): |
| δ= 8.9048 (6.5); 8.8998 (6.8); 8.7981 (5.1); 8.7961 (5.9); 8.7930 (6.0); 8.7911 (5.4); 8.1174 (3.4); 8.1158 (3.8); 8.1121 (6.8); 8.1106 (6.9); 8.1068 (3.9); 8.1052 (3.7); 7.7801 (15.2); 7.7746 (16.0); 7.5484 (3.0); 7.5438 (3.7); 7.5301 (4.2); 7.5281 (4.0); 7.5256 (5.8); 7.5235 (5.2); 7.5128 (5.2); 7.5092 (5.9); 7.5056 (7.8); 7.4933 (9.1); 7.4898 (5.5); 7.4837 (0.6); 7.4375 (11.1); 7.4355 (7.6); 7.4320 (8.9); 7.4202 (6.0); 7.4195 (5.6); 7.4175 (6.0); 7.4152 (11.3); 7.4096 (9.1); 7.4011 (3.4); 7.3980 (3.2); 7.2881 (6.7); 7.2857 (6.2); 7.2847 (5.7); 7.2676 (6.2); 7.2658 (6.0); 7.2624 (58.0); 0.0079 (2.0); 0.0047 (0.5); -0.0002 (75.7); -0.0044 (1.8); -0.0052 (1.3); -0.0060 (1.1); -0.0068 (1.0); -0.0085 (2.4); - 0.0282 (0.5) |
| 1-73: ¹H-NMR(400.6 MHz, CDCl3): |
| δ= 8.8965 (9.4); 8.8916 (9.7); 8.7802 (7.5); 8.7782 (8.5); 8.7751 (8.6); 8.7733 (7.7); 8.0517 (5.3); 8.0501 (5.7); 8.0463 (10.3); 8.0448 (10.3); 8.0410 (5.8); 8.0394 (5.2); 8.0086 (13.0); 8.0071 (13.7); 8.0020 (13.8); 8.0005 (13.7); 7.5924 (13.7); 7.5858 (13.0); 7.5707 (14.4); 7.5640 (13.9); 7.5322 (4.4); 7.5277 (5.6); 7.5209 (0.6); 7.5139 (5.9); 7.5120 (5.5); 7.5095 (8.4); 7.5075 (7.7); 7.4983 (7.8); 7.4941 (11.1); 7.4895 (10.4); 7.4796 (11.1); 7.4786 (12.9); 7.4753 (7.7); 7.4743 (6.3); 7.4117 (8.4); 7.4087 (9.1); 7.3931 (8.2); 7.3907 (7.8); 7.3892 (7.0); 7.3742 (4.7); 7.3712 (4.4); 7.2623 (56.6); 7.2413 (9.8); 7.2389 (8.7); 7.2380 (7.9); 7.2336 (0.8); 7.2210 (8.7); 7.2190 (7.6); 7.2179 (7.0); 6.8171 (15.4); 6.8156 (16.0); 6.7954 (14.5); 6.7939 (14.8); 0.0079 (2.0); -0.0002 (78.2); -0.0085 (2.3) |
| 1-74: ¹H-NMR(400.6 MHz, CDCl3): |
| δ= 8.5826 (7.9); 8.5806 (8.6); 8.5764 (8.5); 8.5745 (8.5); 7.8680 (5.6); 7.8638 (5.8); 7.8489 (6.4); 7.8445 (6.3); 7.7945 (0.6); 7.7897 (15.5); 7.7842 (16.0); 7.7609 (0.5); 7.6893 (0.5); 7.6856 (5.4); 7.6837 (5.6); 7.6644 (11.5); 7.6625 (11.4); 7.6296 (11.9); 7.6234 (11.8); 7.6084 (5.7); 7.6022 (5.8); 7.4967 (3.0); 7.4922 (3.2); 7.4782 (5.3); 7.4767 (4.4); 7.4737 (5.2); 7.4721 (4.5); 7.4636 (8.5); 7.4582 (13.6); 7.4536 (5.6); 7.4410 (8.4); 7.4355 (8.1); 7.4089 (5.0); 7.4057 (5.7); 7.4005 (0.6); 7.3900 (6.4); 7.3868 (7.0); 7.3712 (3.4); 7.3681 (3.2); 7.2616 (26.0); 7.2160 (6.6); 7.2130 (6.9); 7.1957 (5.5); 7.1929 (5.7); 2.0112 (6.4); 1.6171 (0.6); 0.0079 (1.0); -0.0002 (35.0); -0.0085 (1.0) |
| 1-75: ¹H-NMR(400.6 MHz, CDCl3): |
| δ= 8.5928 (10.0); 8.5909 (11.0); 8.5866 (10.8); 8.5848 (10.8); 8.0710 (10.8); 8.0698 (11.5); 8.0644 (11.9); 8.0631 (11.7); 7.8665 (7.5); 7.8621 (7.9); 7.8472 (8.6); 7.8428 (8.6); 7.6581 (7.1); 7.6561 (7.5); 7.6369 (16.0); 7.6349 (16.0); 7.6040 (15.0); 7.5979 (14.6); 7.5852 (11.3); 7.5828 (7.8); 7.5785 (11.6); 7.5767 (8.6); 7.5634 (11.4); 7.5567 (11.2); 7.4860 (3.8); 7.4816 (4.1); 7.4675 (6.5); 7.4660 (5.5); 7.4631 (6.5); 7.4615 (5.6); 7.4475 (6.9); 7.4430 (6.5); 7.3932 (6.3); 7.3900 (7.0); 7.3742 (8.5); 7.3711 (9.2); 7.3555 (4.3); 7.3524 (4.2); 7.2623 (73.8); 7.2514 (0.6); 7.1698 (8.9); 7.1669 (9.2); 7.1497 (7.8); 7.1467 (7.8); 6.7749 (13.0); 6.7736 (13.2); 6.7531 (12.4); 6.7517 (12.5); 2.0133 (0.9); 1.5803 (7.0); 0.0079 (2.4); -0.0002 (97.5); -0.0085 (3.4) |
| 1-76: ¹H-NMR(400.6 MHz, CDCl3): |
| δ= 8.5954 (11.8); 8.5937 (12.6); 8.5891 (12.5); 8.5874 (12.2); 8.1656 (5.8); 8.1638 (6.2); 8.1606 (6.6); 8.1588 (6.2); 8.1532 (6.2); 8.1513 (6.5); 8.1482 (6.4); 8.1464 (6.0); 7.8899 (8.7); 7.8855 (8.8); 7.8706 (9.6); 7.8662 (9.5); 7.7193 (11.5); 7.7175 (11.7); 7.6981 (16.0); 7.6962 (15.9); 7.6413 (5.9); 7.6362 (6.0); 7.6233 (6.7); 7.6205 (7.0); 7.6183 (7.0); 7.6155 (6.7); 7.6025 (6.7); 7.5975 (6.6); 7.5900 (14.9); 7.5837 (14.6); 7.5688 (10.9); 7.5625 (10.8); 7.5210 (0.5); 7.4758 (4.7); 7.4713 (4.9); 7.4573 (7.5); 7.4557 (6.5); 7.4529 (7.4); 7.4512 (6.3); 7.4372 (7.9); 7.4327 (7.4); 7.3739 (7.4); 7.3708 (8.0); 7.3549 (9.4); 7.3518 (10.0); 7.3362 (5.1); 7.3331 (5.0); 7.2625 (93.3); 7.1817 (10.3); 7.1788 (10.3); 7.1614 (8.8); 7.1586 (8.5); 6.9988 (0.5); 6.9650 (6.7); 6.9627 (7.0); 6.9526 (6.7); 6.9503 (7.2); 6.9470 (6.8); 6.9447 (6.6); 6.9346 (6.3); 6.9323 (6.3); 6.8047 (7.3); 6.8027 (13.2); 6.8006 (7.4); 6.7840 (7.1); 6.7820 (12.6); 6.7799 (6.8); 1.6280 (3.0); 0.0278 (0.6); 0.0112 (0.6); 0.0080 (3.7); -0.0002 (122.9); -0.0085 (3.4) |
| 1-77: ¹H-NMR(599.6 MHz, d₆-DMSO): |
| δ= 8.1517 (2.1); 8.1473 (2.1); 7.9121 (1.3); 7.9076 (1.3); 7.8975 (1.4); 7.8930 (1.3); 7.5398 (1.1); 7.5264 (2.2); 7.5130 (1.2); 7.3388 (0.8); 7.2502 (2.0); 7.1616 (0.9); 7.1222 (1.7); 7.1089 (1.6); 7.0121 (1.7); 7.0053 (2.4); 6.9989 (1.6); 6.9907 (2.2); 6.8917 (4.0); 3.5096 (0.3); 2.6131 (0.3); 2.5218 (1.0); 2.5187 (1.2); 2.5156 (1.4); 2.5038 (37.4); 2.5009 (50.0); 2.4980 (37.6); 1.7556 (0.5); 1.7502 (0.5); 1.7416 (0.9); 1.7330 (0.5); 1.7277 (0.5); 0.9119 (0.6); 0.9045 (1.6); 0.9009 (1.7); 0.8940 (0.9); 0.8905 (1.6); 0.8870 (1.7); 0.8801 (0.6); 0.7367 (0.7); 0.7296 (1.9); 0.7266 (1.9); 0.7211 (1.8); 0.7180 (1.9); 0.7103 (0.5); 0.0052 (0.8); -0.0001 (17.5); -0.0056 (0.6) |
| 1-78: ¹H-NMR(400.6 MHz, CDCl3): |
| δ= 8.0667 (2.7); 8.0653 (3.0); 8.0601 (3.0); 8.0586 (3.1); 7.6152 (2.9); 7.6085 (2.9); 7.5934 (3.1); 7.5868 (3.1); 7.2590 (6.3); 7.0272 (2.3); 7.0252 (2.7); 7.0235 (2.6); 6.8552 (2.0); 6.8487 (2.4); 6.8466 (2.6); 6.8450 (2.5); 6.8115 (3.4); 6.8100 (3.6); 6.7897 (3.3); 6.7882 (3.5); 6.7208 (4.2); 6.5864 (2.1); 6.4396 (5.3); 5.2974 (1.5); 2.3752 (15.3); 2.3490 (16.0); 2.3369 (0.9); 2.3043 (0.7); 1.5539 (0.7); -0.0002 (8.4) |
| 1-79: ¹H-NMR(400.6 MHz, CDCl3): |
| δ= 7.8357 (5.1); 7.8302 (5.3); 7.4670 (3.0); 7.4615 (2.9); 7.4446 (2.9); 7.4391 (2.9); 7.2595 (22.5); 7.0531 (2.4); 7.0512 (2.7); 7.0493 (2.5); 6.8904 (2.4); 6.8889 (2.5); 6.8867 (2.4); 6.8636 (1.9); 6.7293 (4.2); 6.5950 (2.1); 6.5195 (5.2); 2.3894 (15.3); 2.3767 (0.7); 2.3665 (16.0); 2.3530 (0.6); 2.3442 (0.8); 2.3201 (0.7); 1.5382 (3.5); 0.0080 (0.8); - 0.0002 (29.9); -0.0085 (0.9) |
| 1-80: ¹H-NMR(400.6 MHz, CDCl3): |
| δ= 8.4608 (1.6); 8.4592 (2.3); 8.4573 (2.1); 8.4554 (2.1); 8.4535 (2.3); 8.4518 (1.6); 8.0648 (3.2); 8.0633 (3.1); 8.0581 (3.4); 8.0566 (3.1); 7.8630 (2.0); 7.8588 (2.0); 7.8440 (2.4); 7.8394 (2.2); 7.5697 (2.2); 7.5496 (2.8); 7.5488 (2.7); 7.5374 (3.2); 7.5307 (3.1); 7.5156 (3.3); 7.5089 (3.3); 7.4483 (1.0); 7.4437 (1.1); 7.4298 (2.1); 7.4286 (1.6); 7.4252 (2.5); 7.4235 (2.8); 7.4215 (1.7); 7.4194 (1.2); 7.4175 (1.7); 7.4157 (1.6); 7.4100 (2.2); 7.4052 (2.4); 7.4032 (1.4); 7.4014 (1.3); 7.3993 (0.9); 7.3974 (1.2); 7.3956 (1.2); 7.3756 (2.0); 7.3722 (2.1); 7.3568 (2.5); 7.3534 (2.6); 7.3381 (1.2); 7.3348 (1.1); 7.2604 (7.6); 7.1620 (2.4); 7.1589 (2.5); 7.1418 (2.0); 7.1391 (2.0); 6.7300 (4.0); 6.7285 (3.7); 6.7082 (3.7); 6.7067 (3.4); 5.2965 (1.8); 2.3095 (16.0); -0.0002 (10.9) |
| 1-81: ¹H-NMR(400.6 MHz, CDCl3): |
| δ= 8.4755 (2.2); 8.4741 (2.2); 8.4630 (2.3); 8.4615 (2.2); 8.0576 (3.0); 8.0561 (3.0); 8.0509 (3.2); 8.0494 (3.0); 7.8408 (1.9); 7.8366 (2.0); 7.8217 (2.2); 7.8174 (2.2); 7.5330 (3.1); 7.5263 (3.0); 7.5112 (3.2); 7.5045 (3.2); 7.4698 (1.7); 7.4680 (2.7); 7.4660 (3.0); 7.4640 (2.7); 7.4622 (1.7); 7.4594 (1.4); 7.4548 (1.1); 7.4408 (1.8); 7.4394 (1.4); 7.4363 (1.8); 7.4348 (1.4); 7.4209 (2.0); 7.4163 (1.8); 7.3750 (1.8); 7.3717 (2.0); 7.3561 (2.2); 7.3528 (2.4); 7.3374 (1.2); 7.3341 (1.2); 7.2604 (10.8); 7.1761 (2.3); 7.1730 (2.4); 7.1558 (2.0); 7.1530 (2.0); 6.9801 (1.3); 6.9783 (1.5); 6.9761 (1.5); 6.9743 (1.3); 6.9675 (1.3); 6.9657 (1.5); 6.9635 (1.4); 6.9618 (1.2); 6.7285 (3.6); 6.7270 (3.5); 6.7067 (3.4); 6.7052 (3.3); 2.2864 (16.0); 0.8817 (0.8); -0.0002 (14.7) |
| 1-82: ¹H-NMR(599.6 MHz, CDCl3): |
| δ= 8.1664 (7.6); 8.1630 (7.7); 7.5864 (4.6); 7.5829 (4.6); 7.5718 (4.6); 7.5684 (4.6); 7.3266 (2.1); 7.3157 (2.4); 7.3126 (4.4); 7.3017 (4.3); 7.2986 (2.6); 7.2877 (2.2); 7.2601 (12.0); 6.9030 (3.5); 6.8204 (8.6); 6.8116 (7.4); 6.7774 (0.3); 6.7577 (2.9); 6.7566 (3.0); 6.7426 (5.6); 6.7287 (2.8); 6.7275 (2.8); 6.7201 (3.7); 6.6178 (5.5); 6.6037 (5.3); 3.8962 (1.2); 3.6031 (1.6); 3.5447 (50.0); 2.6424 (5.8); 0.0053 (0.7); -0.0001 (17.5); -0.0056 (0.6) |
| 1-85: ¹H-NMR(400.6 MHz, CDCl3): |
| δ= 9.3774 (0.8); 8.0618 (14.6); 8.0406 (16.0); 7.7554 (12.4); 7.7500 (13.3); 7.5684 (8.1); 7.5637 (9.4); 7.5536 (9.7); 7.5479 (13.7); 7.5425 (9.4); 7.5312 (9.6); 7.5258 (9.2); 7.5186 (0.6); 7.5008 (13.0); 7.4961 (11.2); 7.3318 (4.6); 7.3256 (8.6); 7.3196 (4.8); 7.2601 (54.9); 6.7625 (5.6); 6.6284 (11.4); 6.4942 (5.7); 5.2996 (2.5); 4.8722 (0.9); 3.4676 (5.7); 1.2540 (0.6); 0.0079 (1.7); -0.0002 (77.0); -0.0085 (2.6) |
| 1-86: ¹H-NMR(400.0 MHz, CDCl3): |
| δ= 8.1422 (1.3); 8.1389 (1.3); 8.1297 (1.4); 8.1264 (1.3); 7.6645 (1.1); 7.6594 (1.1); 7.6464 (1.2); 7.6438 (1.4); 7.6415 (1.3); 7.6388 (1.2); 7.6258 (1.2); 7.6207 (1.1); 7.2596 (19.9); 7.0040 (2.6); 6.9753 (1.2); 6.9732 (1.3); 6.9628 (1.3); 6.9607 (1.4); 6.9574 (1.3); 6.9552 (1.3); 6.9448 (1.2); 6.9427 (1.2); 6.8663 (2.6); 6.8389 (3.8); 6.8186 (2.3); 6.7036 (3.6); 6.6612 (0.6); 6.5689 (1.8); 6.4336 (4.9); 2.8457 (0.6); 2.3698 (14.3); 2.3508 (16.0); 2.3107 (1.7); 2.2617 (1.2); 1.5460 (0.8); 0.0080 (0.9); -0.0002 (25.7); -0.0085 (0.8) |
| 1-87: ¹H-NMR(400.6 MHz, CDCl3): |
| δ= 7.7776 (4.9); 7.7722 (5.2); 7.5093 (2.9); 7.5038 (2.8); 7.4868 (2.9); 7.4814 (2.8); 7.3564 (1.5); 7.3501 (2.8); 7.3446 (1.5); 7.2606 (20.8); 7.0122 (1.2); 7.0105 (1.5); 7.0085 (1.6); 7.0068 (1.4); 6.9855 (1.2); 6.9837 (1.4); 6.9817 (1.6); 6.9800 (1.4); 6.9475 (1.6); 6.9433 (2.6); 6.7777 (1.5); 6.7767 (1.6); 6.6434 (3.1); 6.6422 (3.4); 6.5089 (1.6); 6.5077 (1.7); 5.2993 (1.2); 2.4448 (16.0); -0.0002 (15.9) |
| 1-88: ¹H-NMR(400.0 MHz, CDCl3): |
| δ= 8.0442 (2.8); 8.0429 (3.0); 8.0375 (2.9); 8.0363 (3.0); 7.6940 (2.6); 7.6874 (2.6); 7.6723 (2.8); 7.6656 (2.7); 7.2599 (21.9); 6.9910 (3.3); 6.9896 (3.5); 6.9692 (4.5); 6.9678 (4.6); 6.9415 (1.4); 6.9396 (1.5); 6.8755 (1.9); 6.8569 (2.8); 6.7477 (2.8); 6.7414 (6.4); 6.6066 (2.0); 5.2989 (3.3); 2.4171 (16.0); 2.3496 (0.9); 1.5453 (0.6); -0.0002 (13.6) |
| 1-89: ¹H-NMR(400.6 MHz, CDCl3): |
| δ= 7.8210 (4.8); 7.8155 (5.0); 7.5491 (2.8); 7.5436 (2.7); 7.5268 (2.8); 7.5214 (2.7); 7.2598 (13.4); 7.0036 (1.2); 7.0019 (1.4); 6.9999 (1.5); 6.9982 (1.3); 6.9763 (1.2); 6.9746 (1.4); 6.9727 (1.5); 6.9709 (1.2); 6.8989 (2.8); 6.8817 (2.0); 6.8050 (2.6); 6.7993 (2.6); 6.7475 (4.2); 6.6133 (2.1); 2.4380 (1.7); 2.4318 (16.0); 1.5458 (0.7); -0.0002 (10.1) |
| 1-90: ¹H-NMR(400.0 MHz, CDCl3): |
| δ= 8.0031 (2.9); 8.0019 (2.8); 7.9965 (3.1); 7.6530 (2.7); 7.6463 (2.6); 7.6312 (2.8); 7.6245 (2.7); 7.3606 (1.6); 7.3542 (3.0); 7.3481 (1.5); 7.2604 (51.1); 6.9703 (1.6); 6.9437 (1.6); 6.9377 (3.5); 6.9364 (3.3); 6.9158 (3.3); 6.9145 (3.1); 6.8914 (2.8); 6.7612 (1.6); 6.6265 (3.3); 6.4918 (1.6); 2.5684 (0.6); 2.4275 (16.0); 2.3504 (0.8); 1.5514 (0.8); 0.0080 (0.9); -0.0002 (31.6); -0.0085 (0.9) |
| 1-91: ¹H-NMR(400.0 MHz, CDCl3): |
| δ= 8.1308 (1.3); 8.1258 (1.4); 8.1164 (1.4); 8.1133 (1.3); 7.7447 (1.1); 7.7397 (1.1); 7.7264 (1.4); 7.7241 (1.5); 7.7206 (1.5); 7.7059 (1.4); 7.7009 (1.4); 7.2598 (13.4); 7.0259 (1.6); 7.0135 (5.2); 7.0079 (1.5); 6.9950 (3.3); 6.9928 (2.8); 6.9426 (1.6); 6.9153 (1.5); 6.8658 (4.7); 6.7376 (3.0); 6.7316 (5.7); 6.6735 (1.0); 6.6357 (0.9); 6.5967 (2.0); 5.2985 (0.8); 2.4078 (16.0); 2.3452 (2.4); 2.3275 (2.2); 2.3112 (0.5); 2.2759 (0.5); 1.5495 (0.7); 1.2556 (1.4); -0.0002 (13.2) |
| 1-92: ¹H-NMR(400.6 MHz, CDCl3): |
| δ= 7.8192 (0.6); 7.8122 (15.4); 7.8067 (16.0); 7.5552 (4.2); 7.5508 (9.6); 7.5453 (9.2); 7.5400 (4.5); 7.5344 (8.6); 7.5285 (9.7); 7.5230 (9.5); 7.5190 (9.0); 7.5134 (5.2); 7.5091 (0.6); 7.5023 (0.6); 7.4981 (4.8); 7.2602 (75.5); 7.2328 (0.6); 7.1989 (5.5); 7.1964 (19.2); 7.1779 (4.6); 7.1747 (6.4); 7.1716 (5.2); 7.1531 (4.2); 7.1505 (4.3); 7.1240 (5.3); 7.1213 (9.2); 7.1186 (5.0); 7.1033 (5.0); 7.1005 (8.5); 7.0979 (4.4); 7.0738 (14.3); 6.9022 (10.1); 6.8966 (10.2); 5.2992 (4.5); 3.9442 (1.2); 1.5494 (0.8); 1.2550 (0.8); 0.0704 (2.9); 0.0080 (1.4); -0.0002 (50.4); -0.0085 (1.4) |
| 1-93: ¹H-NMR(400.0 MHz, CDCl3): |
| δ= 8.0811 (1.0); 8.0769 (1.0); 8.0614 (1.1); 8.0572 (1.0); 7.8701 (2.4); 7.8646 (2.5); 7.5957 (1.4); 7.5902 (1.3); 7.5733 (1.4); 7.5678 (1.3); 7.5442 (0.5); 7.5400 (0.6); 7.5256 (0.8); 7.5237 (0.8); 7.5214 (0.8); 7.5194 (0.8); 7.5051 (0.8); 7.5008 (0.8); 7.4188 (0.8); 7.4158 (0.8); 7.3993 (1.0); 7.3964 (1.1); 7.3805 (0.6); 7.3776 (0.6); 7.2601 (19.9); 7.2527 (1.2); 7.2502 (1.2); 7.2320 (1.0); 7.2296 (1.0); 7.0466 (5.4); 3.9805 (16.0); 1.5365 (5.4); 0.0079 (0.7); -0.0002 (26.3); -0.0085 (0.8) |
| 1-94: ¹H-NMR(400.0 MHz, CDCl3): |
| δ= 7.8204 (15.3); 7.8150 (16.0); 7.5719 (3.8); 7.5652 (8.6); 7.5597 (8.5); 7.5568 (4.8); 7.5509 (8.1); 7.5429 (8.7); 7.5361 (10.7); 7.5299 (4.8); 7.5146 (4.4); 7.2598 (57.4); 7.2061 (4.3); 7.2038 (5.1); 7.1848 (4.3); 7.1817 (7.2); 7.1789 (5.6); 7.1600 (4.0); 7.1577 (4.5); 7.1260 (9.2); 7.1076 (4.8); 7.1052 (8.4); 6.8791 (13.6); 6.8735 (13.8); 1.5406 (7.5); 1.2544 (1.6); 0.0707 (1.3); 0.0080 (0.8); -0.0002 (33.2); -0.0085 (1.1) |
| 1-95: ¹H-NMR(599.6 MHz, d₆-DMSO): |
| δ= 7.9262 (2.7); 7.7148 (1.5); 7.7112 (1.5); 7.7008 (1.6); 7.6973 (1.5); 7.6737 (0.7); 7.6598 (1.6); 7.6488 (1.6); 7.6349 (0.8); 7.3708 (1.3); 7.3578 (1.3); 7.3423 (1.8); 7.3268 (1.1); 7.2823 (2.8); 7.1938 (1.4); 7.1311 (2.5); 7.1172 (2.3); 7.0702 (4.3); 7.0593 (2.9); 7.0454 (2.7); 3.3145 (21.6); 2.6138 (0.3); 2.5193 (1.3); 2.5043 (39.5); 2.5016 (50.0); 2.4990 (38.1); 2.2281 (14.5); -0.0001 (10.1) |
| 1-96: ¹H-NMR(400.6 MHz, CDCl3): |
| δ= 8.0654 (0.9); 8.0613 (0.9); 8.0457 (1.0); 8.0416 (1.0); 7.8625 (2.4); 7.8571 (2.6); 7.5908 (1.4); 7.5853 (1.3); 7.5684 (1.4); 7.5630 (1.3); 7.5395 (0.5); 7.5352 (0.5); 7.5209 (0.8); 7.5189 (0.8); 7.5167 (0.8); 7.5147 (0.7); 7.5004 (0.8); 7.4961 (0.8); 7.4159 (0.8); 7.4129 (0.8); 7.3964 (0.9); 7.3934 (0.9); 7.3777 (0.6); 7.3747 (0.6); 7.2740 (1.1); 7.2713 (1.1); 7.2596 (36.5); 7.2539 (1.2); 7.2533 (1.2); 7.2510 (1.1); 7.1444 (5.7); 4.2987 (16.0); 1.5345 (6.8); 0.0079 (1.2); -0.0002 (47.8); -0.0061 (0.5); -0.0085 (1.5) |
| 1-97: ¹H-NMR(400.0 MHz, CDCl3): |
| δ= 8.6988 (7.6); 8.6862 (7.8); 7.8845 (0.8); 7.8590 (9.9); 7.8571 (10.2); 7.7693 (15.4); 7.7638 (16.0); 7.6354 (5.5); 7.6321 (5.4); 7.6228 (5.4); 7.6195 (5.3); 7.5578 (3.0); 7.5533 (3.8); 7.5394 (4.4); 7.5375 (4.4); 7.5350 (6.1); 7.5331 (5.6); 7.5218 (6.6); 7.5178 (6.7); 7.5150 (7.7); 7.5021 (9.6); 7.4984 (6.1); 7.4467 (8.7); 7.4412 (8.5); 7.4351 (6.2); 7.4321 (6.6); 7.4243 (8.9); 7.4188 (9.4); 7.4166 (7.6); 7.4140 (6.6); 7.3974 (3.3); 7.3945 (3.2); 7.2794 (7.7); 7.2769 (7.2); 7.2597 (32.5); 0.0080 (0.7); -0.0002 (26.3); -0.0085 (0.8) |
| 1-98: ¹H-NMR(400.0 MHz, CDCl3): |
| δ= 8.6777 (9.2); 8.6651 (9.4); 8.0137 (11.6); 8.0126 (12.1); 8.0071 (12.4); 8.0060 (11.7); 7.8202 (11.9); 7.8182 (12.0); 7.6090 (11.1); 7.6024 (11.2); 7.5981 (6.9); 7.5944 (6.6); 7.5871 (15.6); 7.5807 (16.0); 7.5416 (3.8); 7.5372 (4.7); 7.5232 (5.4); 7.5213 (5.3); 7.5189 (7.8); 7.5170 (6.8); 7.5084 (7.8); 7.5036 (9.1); 7.4989 (8.5); 7.4887 (11.4); 7.4851 (6.6); 7.4082 (7.0); 7.4052 (7.3); 7.3895 (8.2); 7.3872 (7.2); 7.3705 (3.9); 7.3675 (3.8); 7.2599 (78.9); 7.2308 (9.3); 7.2282 (8.2); 7.2104 (8.2); 7.2084 (7.1); 6.8245 (13.7); 6.8232 (13.2); 6.8027 (12.9); 6.8015 (12.5); 2.0069 (1.3); 0.0080 (2.4); -0.0002 (78.8); -0.0085 (2.1) |
| 1-99: ¹H-NMR(400.0 MHz, CDCl3): |
| δ= 8.6758 (2.4); 8.6632 (2.4); 7.8352 (3.1); 7.8334 (3.1); 7.7534 (4.5); 7.7479 (4.6); 7.6143 (1.7); 7.6108 (1.6); 7.6017 (1.6); 7.5982 (1.6); 7.4262 (2.4); 7.4207 (2.4); 7.4037 (2.5); 7.3982 (2.4); 7.3445 (1.0); 7.3403 (1.3); 7.3239 (1.3); 7.3198 (1.8); 7.3036 (3.0); 7.2995 (2.1); 7.2596 (8.3); 7.1657 (3.8); 7.1451 (3.0); 2.4514 (16.0); 2.0063 (1.4); - 0.0002 (8.3) |
| 1-100: ¹H-NMR(400.0 MHz, CDCl3): |
| δ= 8.6625 (2.4); 8.6499 (2.4); 8.0018 (3.2); 7.9960 (3.2); 7.8053 (3.2); 7.8034 (3.1); 7.5908 (2.9); 7.5841 (4.0); 7.5741 (1.8); 7.5691 (3.9); 7.5623 (2.6); 7.3300 (1.1); 7.3257 (1.4); 7.3095 (1.2); 7.3053 (1.8); 7.2908 (3.1); 7.2867 (2.1); 7.2594 (13.3); 7.1173 (3.7); 7.0968 (3.1); 6.8036 (3.4); 6.7819 (3.2); 2.4424 (16.0); -0.0002 (12.8) |
| 1-101: ¹H-NMR(400.0 MHz, CDCl3): |
| δ= 8.1004 (3.0); 8.0991 (3.1); 8.0938 (3.2); 8.0925 (3.1); 7.8465 (2.3); 7.8416 (2.4); 7.7123 (2.8); 7.7057 (2.7); 7.6906 (3.0); 7.6839 (2.9); 7.3291 (1.2); 7.3276 (1.2); 7.3236 (1.2); 7.3221 (1.2); 7.3081 (1.4); 7.3067 (1.5); 7.3026 (1.4); 7.3012 (1.4); 7.2595 (12.7); 7.0902 (3.9); 7.0693 (3.3); 6.9882 (3.5); 6.9869 (3.5); 6.9664 (3.3); 6.9651 (3.4); 6.8812 (2.0); 6.7466 (4.4); 6.7371 (5.7); 6.6121 (2.1); 2.4433 (16.0); -0.0002 (12.8) |
| 1-102: ¹H-NMR(400.0 MHz, CDCl3): |
| δ= 7.9019 (2.1); 7.8980 (2.2); 7.8897 (2.2); 7.8859 (2.2); 7.8581 (2.3); 7.8529 (2.3); 7.5530 (1.3); 7.5491 (1.3); 7.5331 (1.5); 7.5288 (2.2); 7.5245 (1.3); 7.5085 (1.4); 7.5046 (1.4); 7.3365 (1.1); 7.3350 (1.2); 7.3309 (1.2); 7.3294 (1.1); 7.3155 (1.5); 7.3140 (1.5); 7.3100 (1.5); 7.3086 (1.4); 7.2596 (19.6); 7.1815 (4.0); 7.1606 (3.0); 7.0505 (1.4); 7.0424 (1.5); 7.0383 (1.4); 7.0304 (2.5); 7.0225 (1.3); 7.0184 (1.3); 7.0102 (1.2); 6.8868 (2.0); 6.8652 (5.6); 6.7522 (4.3); 6.6177 (2.1); 2.4462 (16.0); 1.5481 (0.6); 0.0080 (0.6); -0.0002 (21.9); -0.0085 (0.6) |
| 1-103: ¹H-NMR(400.0 MHz, CDCl3): |
| δ= 8.1835 (1.3); 8.1800 (1.4); 8.1710 (1.4); 8.1676 (1.3); 7.8600 (2.3); 7.8551 (2.4); 7.7626 (1.2); 7.7576 (1.2); 7.7445 (1.4); 7.7419 (1.5); 7.7395 (1.5); 7.7370 (1.4); 7.7239 (1.4); 7.7188 (1.3); 7.3182 (1.2); 7.3140 (1.2); 7.2973 (1.5); 7.2919 (1.4); 7.2594 (8.8); 7.1079 (3.8); 7.0870 (3.1); 7.0500 (1.3); 7.0479 (1.5); 7.0376 (1.3); 7.0355 (1.6); 7.0320 (1.4); 7.0299 (1.4); 7.0195 (1.3); 7.0173 (1.6); 7.0125 (2.8); 6.9919 (2.5); 6.8744 (2.0); 6.7807 (5.6); 6.7399 (4.1); 6.6053 (2.1); 2.4382 (16.0); 2.0054 (0.6); -0.0002 (11.3) |
| 1-104: ¹H-NMR(400.0 MHz, CDCl3): |
| δ= 8.6945 (11.0); 8.6819 (11.2); 7.8803 (0.6); 7.8615 (14.1); 7.8597 (14.3); 7.7711 (0.6); 7.7168 (15.8); 7.7104 (16.0); 7.6399 (8.0); 7.6361 (7.5); 7.6273 (7.8); 7.6238 (7.2); 7.5485 (4.9); 7.5440 (6.0); 7.5301 (6.9); 7.5282 (6.6); 7.5257 (9.4); 7.5238 (8.5); 7.5158 (9.5); 7.5108 (9.8); 7.5057 (10.8); 7.4960 (14.4); 7.4925 (8.0); 7.4207 (8.8); 7.4177 (9.3); 7.4018 (10.0); 7.3998 (8.8); 7.3983 (7.8); 7.3831 (5.1); 7.3800 (4.6); 7.2752 (6.2); 7.2686 (6.7); 7.2601 (85.2); 7.2525 (7.5); 7.2505 (7.2); 7.2460 (6.7); 7.2413 (10.2); 7.2394 (8.8); 7.2342 (6.4); 7.2277 (5.8); 7.0301 (0.5); 1.5459 (11.0); 1.2554 (2.2); 0.0078 (3.2); -0.0002 (104.8); -0.0085 (3.0) |
| 1-105: ¹H-NMR(400.0 MHz, CDCl3): |
| δ= 8.7434 (2.3); 8.7307 (2.4); 7.8579 (3.0); 7.8559 (3.0); 7.7228 (1.6); 7.7168 (2.9); 7.7109 (1.6); 7.6889 (1.7); 7.6854 (1.6); 7.6763 (1.6); 7.6727 (1.5); 7.3328 (2.1); 7.3274 (2.8); 7.3093 (1.5); 7.3078 (1.5); 7.3038 (1.0); 7.2886 (1.5); 7.2871 (1.6); 7.2832 (1.3); 7.2610 (14.5); 6.9812 (3.5); 6.9746 (1.1); 6.9679 (1.2); 6.9605 (3.2); 6.9554 (1.9); 6.9487 (1.7); 6.9361 (1.1); 6.9294 (1.0); 5.3000 (0.8); 2.4489 (16.0); 1.5541 (0.7); 0.0080 (0.5); -0.0002 (19.3); - 0.0085 (0.6) |
| 1-106: ¹H-NMR(400.6 MHz, CDCl3): |
| δ= 8.4536 (2.2); 8.4517 (2.0); 8.4498 (2.0); 8.4480 (2.2); 7.8633 (2.0); 7.8588 (1.9); 7.8443 (2.3); 7.8397 (2.2); 7.7751 (5.6); 7.7696 (5.9); 7.5849 (1.9); 7.5649 (2.6); 7.4618 (1.1); 7.4571 (1.3); 7.4496 (1.4); 7.4481 (1.4); 7.4433 (3.4); 7.4421 (2.8); 7.4387 (2.3); 7.4372 (1.8); 7.4296 (1.2); 7.4252 (4.5); 7.4235 (3.8); 7.4197 (4.6); 7.4026 (3.5); 7.3971 (3.4); 7.3919 (2.2); 7.3885 (2.4); 7.3730 (2.6); 7.3697 (2.9); 7.3544 (1.3); 7.3511 (1.2); 7.2646 (0.6); 7.2638 (0.7); 7.2605 (48.5); 7.2556 (0.8); 7.2548 (0.7); 7.2054 (2.5); 7.2024 (2.7); 7.1860 (2.0); 7.1853 (2.1); 7.1826 (2.1); 2.3167 (16.0); 1.5992 (2.1); 0.0080 (1.8); -0.0002 (68.8); -0.0085 (2.0) |
| 1-108: ¹H-NMR(400.6 MHz, CDCl3): |
| δ= 9.1093 (2.3); 9.1064 (2.4); 8.0760 (2.3); 8.0746 (2.4); 8.0694 (2.5); 8.0680 (2.3); 7.9786 (1.6); 7.9746 (1.6); 7.9592 (1.7); 7.9551 (1.7); 7.6216 (2.6); 7.6149 (2.7); 7.6079 (2.6); 7.6060 (2.6); 7.5998 (2.9); 7.5931 (2.8); 7.5389 (0.9); 7.5345 (1.0); 7.5205 (1.4); 7.5186 (1.3); 7.5161 (1.4); 7.5142 (1.3); 7.5002 (1.5); 7.4958 (1.4); 7.4103 (1.4); 7.4073 (1.4); 7.3910 (1.7); 7.3881 (1.8); 7.3724 (1.0); 7.3694 (1.0); 7.2641 (5.3); 7.1811 (1.9); 7.1786 (1.8); 7.1607 (1.7); 7.1583 (1.6); 6.8289 (2.9); 6.8274 (2.9); 6.8071 (2.8); 6.8056 (2.8); 2.5001 (16.0); 2.0047 (0.5); -0.0002 (6.8) |
| 1-109: ¹H-NMR(400.6 MHz, CDCl3): |
| δ= 9.0906 (2.7); 9.0874 (2.8); 7.9896 (1.6); 7.9853 (1.6); 7.9702 (1.7); 7.9659 (1.7); 7.8220 (0.5); 7.8164 (0.5); 7.7983 (4.2); 7.7929 (4.4); 7.6709 (2.5); 7.6691 (2.5); 7.5527 (0.9); 7.5483 (1.0); 7.5342 (1.6); 7.5324 (1.5); 7.5298 (1.4); 7.5280 (1.3); 7.5140 (1.5); 7.5095 (1.4); 7.4896 (2.4); 7.4841 (2.3); 7.4669 (2.4); 7.4614 (2.4); 7.4309 (1.3); 7.4279 (1.5); 7.4118 (1.7); 7.4087 (1.8); 7.3931 (1.0); 7.3901 (1.0); 7.2625 (8.7); 7.2583 (0.7); 7.2521 (2.1); 7.2490 (1.9); 7.2312 (1.6); 7.2287 (1.6); 2.5214 (16.0); 2.4440 (1.6); 2.3693 (1.6); 2.0058 (4.6); -0.0002 (11.2) |
| 1-110: ¹H-NMR(400.6 MHz, CDCl3): |
| δ= 9.1079 (2.3); 8.1651 (0.9); 8.1565 (0.9); 8.0116 (1.6); 8.0074 (1.6); 7.9922 (1.7); 7.9879 (1.7); 7.6780 (1.2); 7.6729 (3.3); 7.6600 (1.3); 7.6574 (1.4); 7.6551 (1.3); 7.6524 (1.2); 7.6393 (1.2); 7.6343 (1.1); 7.5320 (0.9); 7.5276 (0.9); 7.5136 (1.3); 7.5118 (1.3); 7.5092 (1.4); 7.5073 (1.2); 7.4933 (1.3); 7.4889 (1.3); 7.3941 (1.3); 7.3911 (1.4); 7.3748 (1.7); 7.3720 (1.8); 7.3563 (1.0); 7.3533 (1.0); 7.2623 (10.7); 7.1949 (1.9); 7.1923 (1.9); 7.1745 (1.7); 7.1720 (1.7); 6.9941 (1.0); 6.9920 (1.1); 6.9817 (1.0); 6.9796 (1.2); 6.9762 (1.1); 6.9740 (1.1); 6.9637 (1.0); 6.9616 (1.0); 6.8536 (1.7); 6.8329 (1.7); 2.4807 (16.0); -0.0002 (13.8) |
| 1-111: ¹H-NMR(400.6 MHz, CDCl3): |
| δ= 9.0818 (2.7); 9.0786 (2.8); 8.0147 (1.6); 8.0103 (1.6); 7.9953 (1.7); 7.9909 (1.7); 7.8390 (1.5); 7.8351 (1.6); 7.8268 (1.6); 7.8230 (1.7); 7.7126 (2.5); 7.7105 (2.6); 7.5404 (0.8); 7.5359 (0.9); 7.5219 (1.2); 7.5201 (1.2); 7.5175 (1.3); 7.5157 (1.2); 7.5016 (1.3); 7.4972 (1.3); 7.4577 (1.0); 7.4538 (1.0); 7.4378 (1.1); 7.4333 (1.6); 7.4290 (1.1); 7.4131 (1.1); 7.4091 (2.3); 7.4061 (1.4); 7.3899 (1.6); 7.3869 (1.8); 7.3713 (0.9); 7.3682 (0.9); 7.2666 (2.4); 7.2641 (2.0); 7.2612 (1.9); 7.2437 (1.6); 7.2409 (1.6); 6.9631 (1.1); 6.9550 (1.1); 6.9510 (1.1); 6.9430 (1.9); 6.9352 (1.0); 6.9311 (1.0); 6.9230 (1.0); 2.4945 (16.0); -0.0002 (2.8) |
| 1-112: ¹H-NMR(400.0 MHz, CDCl3): |
| δ= 8.0767 (4.4); 8.0726 (4.5); 8.0570 (4.8); 8.0528 (4.8); 7.8984 (10.4); 7.8929 (10.7); 7.6137 (6.0); 7.6082 (5.7); 7.5912 (5.9); 7.5858 (5.7); 7.5669 (2.3); 7.5627 (2.5); 7.5483 (3.4); 7.5462 (3.5); 7.5441 (3.7); 7.5421 (3.2); 7.5277 (3.6); 7.5234 (3.5); 7.4260 (3.4); 7.4231 (3.7); 7.4064 (4.7); 7.4038 (4.8); 7.3876 (2.6); 7.3848 (2.7); 7.2854 (5.4); 7.2829 (5.3); 7.2646 (5.0); 7.2594 (24.1); 6.9015 (16.0); 1.5384 (8.3); 0.0080 (0.8); -0.0002 (29.4); -0.0085 (0.8) |
| 1-113: ¹H-NMR(400.0 MHz, CDCl3): |
| δ= 8.1302 (7.0); 8.1236 (7.0); 8.0747 (4.4); 8.0706 (4.5); 8.0550 (4.8); 8.0508 (4.7); 7.7477 (5.8); 7.7410 (5.6); 7.7259 (6.1); 7.7193 (5.9); 7.5529 (2.3); 7.5486 (2.4); 7.5342 (3.4); 7.5323 (3.5); 7.5302 (3.7); 7.5282 (3.3); 7.5137 (3.4); 7.5094 (3.2); 7.4041 (3.3); 7.4012 (3.6); 7.3845 (4.7); 7.3820 (4.8); 7.3658 (2.6); 7.3630 (2.5); 7.2596 (25.6); 7.1951 (5.3); 7.1926 (5.2); 7.1744 (4.8); 7.1720 (4.6); 7.0174 (8.0); 6.9957 (7.6); 6.8030 (16.0); 1.5406 (11.4); 0.0080 (0.9); -0.0002 (31.5); -0.0085 (0.9) |
| 1-114: ¹H-NMR(400.6 MHz, CDCl3): |
| δ= 7.8545 (5.3); 7.8490 (5.6); 7.8413 (2.3); 7.8361 (2.3); 7.5770 (2.9); 7.5716 (2.8); 7.5546 (2.9); 7.5491 (2.9); 7.3383 (1.2); 7.3367 (1.3); 7.3328 (1.2); 7.3311 (1.2); 7.3173 (1.5); 7.3158 (1.5); 7.3138 (1.0); 7.3118 (1.5); 7.3102 (1.5); 7.2564 (5.0); 7.1595 (4.1); 7.1386 (3.2); 6.8911 (2.0); 6.8236 (5.6); 6.7569 (4.4); 6.6226 (2.2); 2.4458 (16.0); - 0.0002 (3.2) |
| 1-115: ¹H-NMR(400.6 MHz, CDCl3): |
| δ= 8.1059 (4.0); 8.1004 (4.1); 8.0254 (2.3); 8.0231 (2.4); 8.0219 (2.2); 7.5149 (3.1); 7.5094 (3.0); 7.4916 (3.0); 7.4862 (2.9); 7.3656 (1.3); 7.3643 (1.3); 7.3602 (1.3); 7.3589 (1.3); 7.3443 (1.7); 7.3430 (1.7); 7.3389 (1.8); 7.3376 (1.7); 7.2619 (21.3); 7.2293 (4.1); 7.2080 (3.0); 7.1433 (4.8); 6.4886 (1.8); 6.3543 (3.8); 6.2199 (1.9); 5.2992 (2.3); 2.4579 (0.6); 2.4437 (1.1); 2.4089 (16.0); -0.0002 (12.9) |
| 1-116: ¹H-NMR(400.6 MHz, CDCl3): |
| δ= 7.7957 (2.7); 7.7902 (2.8); 7.5320 (1.6); 7.5265 (1.6); 7.5096 (1.6); 7.5042 (1.6); 7.4951 (0.7); 7.4798 (0.7); 7.4742 (1.4); 7.4590 (1.4); 7.4534 (0.9); 7.4381 (0.8); 7.2607 (13.1); 7.1626 (0.8); 7.1600 (0.9); 7.1416 (0.8); 7.1381 (1.1); 7.1351 (0.9); 7.1167 (0.7); 7.1140 (0.8); 7.0845 (0.9); 7.0817 (1.6); 7.0790 (0.9); 7.0638 (0.8); 7.0611 (1.5); 7.0584 (0.8); 6.4721 (2.0); 6.4656 (2.0); 2.3079 (16.0); -0.0002 (8.0) |
| 1-117: ¹H-NMR(400.6 MHz, CDCl3): |
| δ= 8.0311 (1.8); 8.0295 (1.8); 8.0244 (2.0); 8.0229 (1.9); 7.6760 (2.1); 7.6694 (2.0); 7.6542 (2.2); 7.6476 (2.1); 7.4784 (0.8); 7.4630 (0.8); 7.4575 (1.6); 7.4422 (1.6); 7.4367 (0.9); 7.4213 (0.9); 7.2609 (12.3); 7.1296 (0.9); 7.1269 (1.0); 7.1085 (0.8); 7.1056 (1.1); 7.1048 (1.1); 7.1020 (1.0); 7.0837 (0.8); 7.0809 (0.8); 7.0449 (1.0); 7.0421 (1.8); 7.0394 (0.9); 7.0242 (1.0); 7.0215 (1.7); 7.0188 (0.8); 6.9808 (2.2); 6.9791 (2.3); 6.9590 (2.2); 6.9574 (2.2); 6.4179 (2.0); 6.4118 (2.1); 2.3013 (16.0); -0.0002 (8.3) |
| 1-118: ¹H-NMR(400.0 MHz, CDCl3): |
| δ= 8.1881 (4.6); 8.1840 (4.7); 8.1684 (5.0); 8.1642 (5.0); 7.9741 (6.9); 7.9686 (7.0); 7.9675 (6.9); 7.6863 (6.5); 7.6797 (6.1); 7.6644 (6.9); 7.6578 (6.6); 7.6180 (2.6); 7.6138 (2.6); 7.5994 (3.7); 7.5977 (3.8); 7.5952 (3.8); 7.5935 (3.5); 7.5791 (3.6); 7.5748 (3.5); 7.5189 (0.7); 7.4525 (3.1); 7.4488 (8.2); 7.4450 (8.0); 7.4414 (2.8); 7.4201 (3.6); 7.4172 (3.8); 7.4006 (5.0); 7.3979 (5.1); 7.3819 (3.0); 7.3790 (2.9); 7.2940 (5.6); 7.2914 (5.6); 7.2735 (5.2); 7.2709 (5.0); 7.2599 (120.9); 7.0268 (7.6); 7.0257 (7.7); 7.0050 (7.2); 7.0038 (7.1); 6.9960 (0.8); 1.5473 (16.0); 0.0080 (2.1); -0.0002 (71.6); -0.0085 (2.0) |
| 1-119: ¹H-NMR(400.0 MHz, CDCl3): |
| δ= 8.2142 (1.8); 8.2101 (1.8); 8.1946 (2.0); 8.1904 (1.9); 7.7416 (2.9); 7.7362 (2.9); 7.6413 (0.9); 7.6370 (0.9); 7.6211 (1.5); 7.6183 (1.4); 7.6023 (1.4); 7.5980 (1.3); 7.5452 (2.4); 7.5397 (2.2); 7.5226 (2.4); 7.5172 (2.2); 7.4562 (1.4); 7.4533 (1.5); 7.4412 (3.0); 7.4373 (4.6); 7.4340 (3.0); 7.4179 (1.1); 7.4150 (1.1); 7.3406 (2.2); 7.3380 (2.1); 7.3202 (2.0); 7.3175 (1.8); 7.2601 (105.3); 6.9960 (0.5); 1.5392 (16.0); 0.0080 (2.3); -0.0002 (63.5); -0.0085 (1.8) |

Gegenstand der vorliegenden Erfindung ist weiterhin die Verwendung einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-119) und/oder deren Salze, jeweils wie oben definiert, als Herbizid und/oder Pflanzenwachstumsregulator, vorzugsweise in Kulturen von Nutz-und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-119) und/oder deren Salze, jeweils wie oben definiert, oder
- eines erfindungsgemäßen Mittels, wie nachstehend definiert,
   auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-119) und/oder deren Salze, jeweils wie oben definiert, oder
- eines erfindungsgemäßen Mittels, wie nachstehend definiert,
   auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch ein Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (1-1) bis (1-119) und/oder deren Salze, jeweils wie oben definiert, oder
- eines erfindungsgemäßen Mittels, wie nachstehend definiert,
   die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen bzw. die erfindungsgemäßen Mittel z.B. im Vorsaat- (gegebenenfalls auch durch Einarbeitung in den Boden), Vorauflauf- und/oder Nachauflaufverfahren ausgebracht werden. Im einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen der allgemeinen Formel (I) und/oder deren Salze eignen sich zur Bekämpfung der folgenden Gattungen von monokotylen und dikotylen Schadpflanzen: Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen der allgemeinen Formel (I) vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen der allgemeinnen Formel (I) eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen der allgemeinen Formel (I) (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe der allgemeinen Formel (I) auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen der allgemeinen Formel (I) und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen der allgemeinen Formel (I) auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Verbindungen der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung von Verbindungen der allgemeinen Formel (I) in Getreide, dabei vorzugsweise Mais, Weizen, Gerste, Roggen, Hafer, Hirse, oder Reis, im Vor- oder Nachauflauf.

Bevorzugt ist auch die Verwendung von Verbindungen der allgemeinene Formel (I) in Soja im Vor- oder Nachauflauf.

Die Verwendung erfindungsgemäßer Verbindungen der Formel (I) zur Bekämpfung von Schadpflanzen oder zur Wachstumsregulierung von Pflanzen schließt auch den Fall ein, bei dem eine Verbindung der allgemeinen Formel (I) oder deren Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der allgemeinen Formel (I) oder deren Salzen auf die Pflanzen (Schadpflanzen, gegebenenfalls zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, dass das Mittel
(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (I-1) bis (I-119) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten allgemeinen Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der allgemeinen Formel (I) und/oder deren Salze enthalten.

Die erfindungsgemäßen Verbindungen der allgemeinen Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der allgemeinen Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die erfindungsgemäßen Verbindungen der allgemeinen Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der allgemeinen Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200: 1 bis 1:200, vorzugsweise 100: 1 bis 1: 100, insbesondere 20:1 bis 1:20. Die Safener können analog den Verbindungen der allgemeinen Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von erfindungsgemäßen Verbindungen der allgemeinen Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der allgemeinen Formel (I) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfaßt, auch wenn diese nicht explizit erwähnt sind.

Beispiele für solche herbiziden Mischungspartner sind:
Acetochlor, acifluorfen, acifluorfen-sodium, aclonifen, alachlor, allidochlor, alloxydim, alloxydimsodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlorpotassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenacsodium, chlorfenprop, chlorflurenol, chlorflurenol-methyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofop-butyl, cyprazine, 2,4-D, 2,4-D-butotyl, -butyl, - dimethylammonium, -diolamin, -ethyl, 2-ethylhexyl, -isobutyl, -isooctyl, -isopropylammonium, - potassium, -triisopropanolammonium und -trolamine, 2,4-DB, 2,4-DB-butyl, -dimethylammonium, isooctyl, -potassium und -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyrsodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-[2-Chlor-4-fluor-5 -[4-(3 -fluorpropyl)-4,5 -dihydro-5 -oxo-1H-tetrazol-1-yl] -phenyl] -ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, - dimethylammonium und -methyl, fluoroglycofen, fluoroglycofen-ethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, - potassium, -sodium und -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuronmethyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(Dimethoxyphosphoryl)-ethyl-(2,4-dichlorphenoxy)acetat, imazamethabenz, Imazamethabenz-methyl, imazamox, imazamox-ammonium, imazapic, imazapic-ammonium, imazapyr, imazapyr-isopropylammonium, imazaquin, imazaquinammonium, imazethapyr, imazethapyr-immonium, imazosulfuron, indanofan, indaziflam, iodosulfuron, iodosulfuron-methyl-sodium, ioxynil, ioxynil-octanoate, -potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl } sulfonyl)-5,5-dimethyl-4,5-dihydro-1,2-oxazol, ketospiradox, lactofen, lenacil, linuron, MCPA, MCPA-butotyl, -dimethylammonium, -2-ethylhexyl, - isopropylammonium, -potassium und -sodium, MCPB, MCPB-methyl, -ethyl und -sodium, mecoprop, mecoprop-sodium, und -butotyl, mecoprop-P, mecoprop-P-butotyl, -dimethylammonium, -2-ethylhexyl und -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, neburon, nicosulfuron, nonanoic acid (Pelargonsäure), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuronethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron,, SYN-523, SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trifluoressigsäure), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, sowie die folgenden Verbindungen:

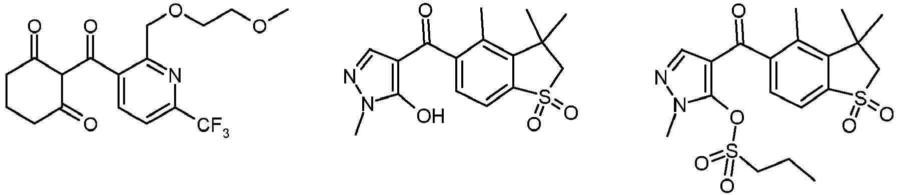

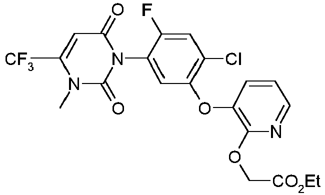

Beispiele für Pflanzenwachstumsregulatoren als mögliche Mischungspartner sind:
Acibenzolar, acibenzolar-S-methyl, 5-Aminolävulinsäure, ancymidol, 6-benzylaminopurine, Brassinolid, Catechin, chlormequat chloride, cloprop, cyclanilide, 3-(Cycloprop-1-enyl)propionsäure, daminozide, dazomet, n-decanol, dikegulac, dikegulac-sodium, endothal, endothaldipotassium, -disodium, und mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, Jasmonsäure, Jasmonsäuremethylester, maleic hydrazide, mepiquat chloride, 1-methylcyclopropene, 2-(1-naphthyl)acetamide, 1-naphthylacetic acid, 2-naphthyloxyacetic acid, nitrophenolate-mixture, 4-Oxo-4[(2-phenylethyl)amino]buttersäure, paclobutrazol, N-phenylphthalamic acid, prohexadione, prohexadione-calcium, prohydrojasmone, Salicylsäure, Strigolacton, tecnazene, thidiazuron, triacontanol, trinexapac, trinexapac-ethyl, tsitodef, uniconazole, uniconazole-P.

Ebenfalls als Kombinationspartner für die erfindungsgemäßen Verbindungen der allgemeinen Formel (I) kommen beispielsweise die folgenden Safener in Frage:
S1) Verbindungen aus der Gruppe heterocyclischer Carbonsäurederivate:
   S1^{a}) Verbindungen vom Typ der Dichlorphenylpyrazolin-3-carbonsäure (S1^{a}), vorzugsweise Verbindungen wie
      1-(2,4-Dichlorphenyl)-5-(ethoxycarbonyl)-5-methyl-2-pyrazolin-3-carbonsäure,
      1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   S1^{b}) Derivate der Dichlorphenylpyrazolcarbonsäure (S1^{b}), vorzugsweise Verbindungen wie
      1-(2,4-Dichlorphenyl)-5-methylpyrazol-3-carbonsäureethylester (S1-2),
      1-(2,4-Dichlorphenyl)-5-isopropylpyrazol-3-carbonsäureethylester (S1-3),
      1-(2,4-Dichlorphenyl)-5-(1,1-dimethyl-ethyl)pyrazol-3-carbonsäureethylester (S1-4) und verwandte Verbindungen, wie sie in EP-A-333131 und EP-A-269806 beschrieben sind;
   S1^{c}) Derivate der 1,5-Diphenylpyrazol-3-carbonsäure (S1^{c}), vorzugsweise Verbindungen wie
      1-(2,4-Dichlorphenyl)-5-phenylpyrazol-3-carbonsäureethylester (S 1-5),
      1-(2-Chlorphenyl)-5-phenylpyrazol-3-carbonsäuremethylester (S1-6) und verwandte Verbindungen wie sie beispielsweise in der EP-A-268554 beschrieben sind;
   S1^{d}) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
   S1^{e}) Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbon-säureethylester (S1-13), wie sie in der Patentanmeldung WO-A-95/07897 beschrieben sind.
S2) Verbindungen aus der Gruppe der 8-Chinolinoxyderivate (S2):
   S2^{a}) Verbindungen vom Typ der 8-Chinolinoxyessigsäure (S2^{a}), vorzugsweise
      (5-Chlor-8-chinolinoxy)essigsäure-(1-methylhexyl)-ester ("Cloquintocet-mexyl") (S2-1),
      (5-Chlor-8-chinolinoxy)essigsäure-(1,3-dimethyl-but-1-yl)-ester (S2-2),
      (5-Chlor-8-chinolinoxy)essigsäure-4-allyl-oxy-butylester (S2-3),
      (5-Chlor-8-chinolinoxy)essigsäure-1-allyloxy-prop-2-ylester (S2-4),
      (5-Chlor-8-chinolinoxy)essigsäureethylester (S2-5),
      (5-Chlor-8-chinolinoxy)essigsäuremethylester (S2-6),
      (5-Chlor-8-chinolinoxy)essigsäureallylester (S2-7),
      (5-Chlor-8-chinolinoxy)essigsäure-2-(2-propyliden-iminoxy)-1-ethylester (S2-8),
      (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   S2^{b}) Verbindungen vom Typ der (5-Chlor-8-chinolinoxy)malonsäure (S2^{b}), vorzugsweise Verbindungen wie (5-Chlor-8-chinolinoxy)malonsäurediethylester,
      (5-Chlor-8-chinolinoxy)malonsäurediallylester,
      (5-Chlor-8-chinolinoxy)malonsäure-methyl-ethylester und verwandte Verbindungen, wie sie in EP-A-0 582 198 beschrieben sind.
S3) Wirkstoffe vom Typ der Dichloracetamide (S3), die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF,
   "Furilazol" oder "MON 13900" ((RS)-3-Dichloracetyl-5-(2-furyl)-2,2-dimethyloxazolidin) (S3-10), sowie dessen (R)-Isomer (S3-11).
S4) Verbindungen aus der Klasse der Acylsulfonamide (S4):
   S4^{a}) N-Acylsulfonamide der Formel (S4^{a}) und deren Salze wie sie in der WO-A-97/45016 beschrieben sind, 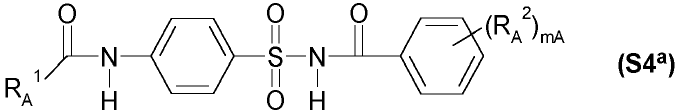 worin
      - R_{A}¹: (C₁-C₆)Alkyl, (C₃-C₆)Cycloalkyl, wobei die 2 letztgenannten Reste durch v_{A} Substituenten aus der Gruppe Halogen, (C₁-C₄)Alkoxy, (C₁-C₆)Haloalkoxy und (C₁-C₄)Alkylthio und im Falle cyclischer Reste auch durch (C₁-C₄)Alkyl und (C₁-C₄)Haloalkyl substituiert sind;
      - R_{A}²: Halogen, (C₁-C₄)Alkyl, (C₁-C₄)Alkoxy, CF₃,
      - m_{A}: 1 oder 2;
      - v_{A}: ist 0, 1, 2 oder 3 bedeuten;
   S4^{b}) Verbindungen vom Typ der 4-(Benzoylsulfamoyl)benzamide der Formel (S4^{b}) und deren Salze, wie sie in der WO-A-99/16744 beschrieben sind, 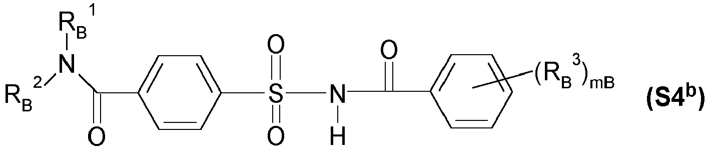 worin
      R_{B}¹, R_{B}² unabhängig voneinander Wasserstoff, (C₁-C₆)Alkyl, (C₃-C₆)Cycloalkyl, (C₃-C₆)Alkenyl, (C₃-C₆)Alkinyl,
      R_{B}³ Halogen, (C₁-C₄)Alkyl, (C₁-C₄)Haloalkyl oder (C₁-C₄)Alkoxy und
      m_{B} 1 oder 2 bedeuten,
      z.B. solche worin
      R_{B}¹ = Cyclopropyl, R_{B}² = Wasserstoff und (R_{B}³) = 2-OMe ist ("Cyprosulfamide", S4-1),
      R_{B}¹= Cyclopropyl, R_{B}² = Wasserstoff und (R_{B}³) = 5-Cl-2-OMe ist (S4-2),
      R_{B}¹ = Ethyl, R_{B}² = Wasserstoff und (R_{B}³) = 2-OMe ist (S4-3),
      R_{B}¹ = Isopropyl, R_{B}² = Wasserstoff und (R_{B}³) = 5-Cl-2-OMe ist (S4-4) und
      R_{B}¹ = Isopropyl, R_{B}² = Wasserstoff und (R_{B}³) = 2-OMe ist (S4-5);
   S4^{c}) Verbindungen aus der Klasse der Benzoylsulfamoylphenylharnstoffe der Formel (S4^{c}), wie sie in der EP-A-365484 beschrieben sind, 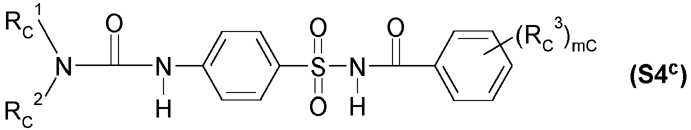 worin
      - R_{C}¹, R_{C}²: unabhängig voneinander Wasserstoff, (Ci-Cs)Alkyl, (C₃-C₈)Cycloalkyl, (C₃-C₆)Alkenyl, (C₃-C₆)Alkinyl,
      - R_{C}³: Halogen, (C₁-C₄)Alkyl, (C₁-C₄)Alkoxy, CF₃ und
      - m_{C}: 1 oder 2 bedeuten;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff;
   S4^{d}) Verbindungen vom Typ der N-Phenylsulfonylterephthalamide der Formel (S4^{d}) und deren Salze, die z.B. bekannt sind aus CN 101838227, 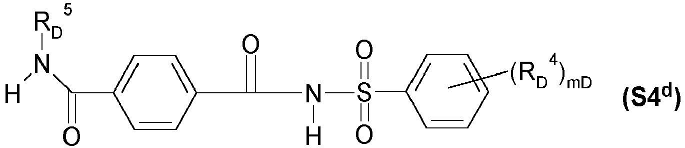 worin
      - R_{D}⁴: Halogen, (C₁-C₄)Alkyl, (C₁-C₄)Alkoxy, CF₃,
      - m_{D}: 1 oder 2;
      - R_{D}⁵: Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl bedeutet.
S5) Wirkstoffe aus der Klasse der Hydroxyaromaten und der aromatisch-aliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 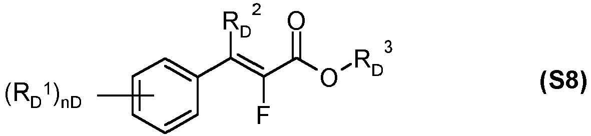 worin die Symbole und Indizes folgende Bedeutungen haben:
   - R_{D}¹: ist Halogen, (C₁-C₄)Alkyl, (C₁-C₄)Haloalkyl, (C₁-C₄)Alkoxy, (C₁-C₄)Haloalkoxy,
   - R_{D}²: ist Wasserstoff oder (C₁-C₄)Alkyl,
   - R_{D}³: ist Wasserstoff, (Ci-Cs)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
   - n_{D}: ist eine ganze Zahl von 0 bis 2.
   - S9): Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
   - S10): Verbindungen der Formeln (S10^{a}) oder (S10^{b}), wie sie in der WO-A-2007/023719 und WO-A-2007/023764 beschrieben sind, 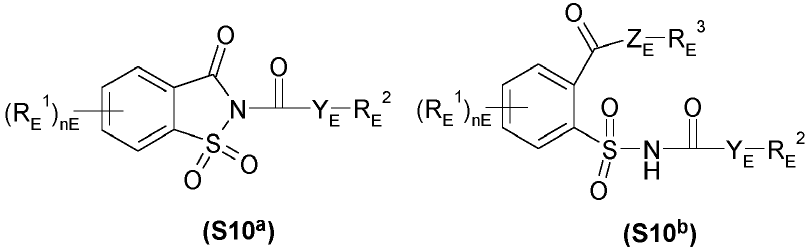
   worin
   - R_{E}¹: Halogen, (C₁-C₄)Alkyl, Methoxy, Nitro, Cyano, CF₃, OCF₃
   - Y_{E}, Z_{E}: unabhängig voneinander O oder S,
   - n_{E}: eine ganze Zahl von 0 bis 4,
   - R_{E}²: (C₁-C₁₆)Alkyl, (C₂-C₆)Alkenyl, (C₃-C₆)Cycloalkyl, Aryl; Benzyl, Halogenbenzyl,
   - R_{E}³: Wasserstoff oder (C₁-C₆)Alkyl bedeuten.
   - S11): Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3 -dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8)
   (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG 838" (CAS-Reg.Nr. 133993-74-5)
   (2-propenyl 1-oxa-4-azaspiro[4.5]decan-4-carbodithioat) (S13-6) der Firma Nitrokemia
   "Disulfoton" (O,O-Diethyl S-2-ethylthioethyl phosphordithioat) (S13-7),
   "Dietholate" (O,O-Diethyl-O-phenylphosphorothioat) (S13-8),
   "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087270), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere, 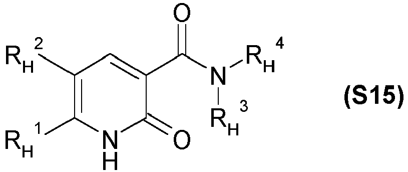 wie sie in der WO-A-2008/131861 und WO-A-2008/131860 beschrieben sind,
   worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl, wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z. B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugte Safener in Kombination mit den erfindungsgemäßen Verbindungen der allgemeinen Formel (I) und/oder deren Salze, insbesondere mit den Verbindungen der Formeln (1-1) bis (1-119) und/oder deren Salze, sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

### Biologische Beispiele

### A. Herbizide Wirkung im frühen Nachauflauf

Samen von mono- bzw. dikotylen Unkrautpflanzen wurden in 96-well Mikrotiterplatten in Quarzsand ausgelegt und in der Klimakammer unter kontrollierten Wachstumsbedingungen angezogen. 5 bis 7 Tage nach der Aussaat wurden die Versuchspflanzen im Keimblattstadium behandelt. Die in Form von Emulsionskonzentraten (EC) formulierten erfindungsgemäßen Verbindungen wurden mit einer Wasseraufwandmenge von umgerechnet 2200 Liter pro Hektar appliziert. Nach 9 bis 12 Tagen Standzeit der Versuchspflanzen in der Klimakammer unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen A1 bis A8 sind die Wirkungen ausgewählter Verbindungen der allgemeinen Formel (I) und Vergleichsverbindungen gemäß Tabelle 1 auf verschiedene Schadpflanzen und einer Aufwandmenge entsprechend 1900 g/ha, die gemäß zuvor genannter Versuchvorschrift erhalten wurden, dargestellt.

**Tabelle A1: Frühe Nachauflaufwirkung gegen Agrostis tenuis (AGSTE)**

| Beispielnummer | Dosierung [g/ha] | AGSTE |
|---|---|---|
| 1-8 | 1900 | 80 |
| 1-12 | 1900 | 80 |
| 1-13 | 1900 | 80 |
| 1-30 | 1900 | 80 |
| 1-32 | 1900 | 80 |
| 1-41 | 1900 | 80 |
| 1-42 | 1900 | 80 |
| 1-50 | 1900 | 80 |
| 1-56 | 1900 | 100 |
| 1-66 | 1900 | 100 |
| 1-69 | 1900 | 100 |
| 1-70 | 1900 | 100 |
| 1-73 | 1900 | 100 |
| 1-78 | 1900 | 100 |
| 1-82 | 1900 | 80 |
| 1-87 | 1900 | 80 |
| 1-88 | 1900 | 100 |
| 1-108 | 1900 | 80 |
| 1-109 | 1900 | 80 |
| 1-112 | 1900 | 100 |
| 1-113 | 1900 | 100 |

**Tabelle A2: Frühe Nachauflaufwirkung gegen Lolium perenne (LOLPE)**

| Beispielnummer | Dosierung [g/ha] | LOLPE |
|---|---|---|
| 1-78 | 1900 | 80 |

**Tabelle A3: Frühe Nachauflaufwirkung gegen Poa annua (POAAN)**

| Beispielnummer | Dosierung [g/ha] | POAAN |
|---|---|---|
| 1-37 | 1900 | 80 |
| 1-41 | 1900 | 100 |
| 1-56 | 1900 | 100 |
| 1-66 | 1900 | 80 |
| 1-69 | 1900 | 100 |
| 1-70 | 1900 | 100 |
| 1-73 | 1900 | 80 |
| 1-78 | 1900 | 100 |
| 1-87 | 1900 | 80 |
| 1-88 | 1900 | 100 |
| 1-106 | 1900 | 80 |
| 1-108 | 1900 | 80 |
| 1-109 | 1900 | 100 |
| 1-112 | 1900 | 100 |
| 1-113 | 1900 | 100 |

**Tabelle A4: Frühe Nachauflaufwirkung gegen Setaria viridis (SETVI)**

| Beispielnummer | Dosierung [g/ha] | SETVI |
|---|---|---|
| 1-49 | 1900 | 100 |
| 1-88 | 1900 | 80 |

**Tabelle A5: Frühe Nachauflaufwirkung gegen Diplotaxis tenuifolia (DIPTE)**

| Beispielnummer | Dosierung [g/ha] | DIPTE |
|---|---|---|
| 1-5 | 1900 | 80 |
| 1-8 | 1900 | 80 |
| 1-14 | 1900 | 80 |
| 1-32 | 1900 | 80 |
| 1-109 | 1900 | 80 |

**Tabelle A6: Frühe Nachauflaufwirkung gegen Matricaria chamonilla (MATCH)**

| Beispielnummer | Dosierung [g/ha] | MATCH |
|---|---|---|
| 1-51 | 1900 | 80 |
| 1-108 | 1900 | 80 |
| 1-112 | 1900 | 80 |
| 1-113 | 1900 | 80 |

**Tabelle A7: Frühe Nachauflaufwirkung gegen Stellaria media (STEME)**

| Beispielnummer | Dosierung [g/ha] | STEME |
|---|---|---|
| 1-49 | 1900 | 80 |

**Tabelle A8: Frühe Nachauflaufwirkung gegen Veronica persica (VERPE)**

| Beispielnummer | Dosierung [g/ha] | VERPE |
|---|---|---|
| 1-84 | 1900 | 80 |

Wie die Ergebnisse der Tabellen A1-A8 zeigen, weisen die Verbindungen Nr. 1-5, 1-8, 1-12, 1-13, 1-14, 1-30, 1-32, 1-37, 1,41, 1-42, 1-49, 1-50, 1-51, 1-56, 1-66, 1-69, 1-70, 1-73, 1-78, 1-82, 1-84, 1-87, 1-88, 1-106, 1-108, 1-109, 1-112 und 1-113 bei Behandlung im frühen Nachauflauf eine sehr gute herbizide Wirksamkeit gegen die Schadpflanzen *Agrostis tenuis* (AGSTE), *Diplotaxis tenuifolia* (DIPTE), *Lolium perenne* (LOLPE), *Matricaria chamonilla* (MATCH), *Poa annua* (POAAN), *Setaria viridis* (SETVI), *Stellaria media* (STEME) und *Veronica persica* (VERPE) bei einer Aufwandmenge von 1900 g Aktivsubstanz pro Hektar auf.

### B. Herbizide Wirkung im Nachauflauf

Samen von mono- bzw. dikotylen Unkrautpflanzen wurden in Kunststofftöpfen in sandigem Lehmboden ausgelegt (Doppelaussaaten mit jeweils einer Spezies mono- bzw. dikotyler Unkrautpflanzen pro Topf), mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden als wässrige Suspension bzw. Emulsion, unter Zusatz von 0,5% Additiv, mit einer Wasseraufwandmenge von umgerechnet 600 Liter pro Hektar, auf die grünen Pflanzenteile appliziert. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert.

Beispielsweise bedeutet 100% Wirkung = Pflanzen sind abgestorben, 0% Wirkung = wie Kontrollpflanzen.

In den nachstehenden Tabellen B1 bis B12 sind die Wirkungen ausgewählter Verbindungen der allgemeinen Formel (I) und Vergleichsverbindungen gemäß Tabelle 1 auf verschiedene Schadpflanzen und einer Aufwandmenge entsprechend 1280 oder 320 g/ha, die gemäß zuvor genannter Versuchvorschrift erhalten wurden, dargestellt.

**Tabelle B1: Nachauflaufwirkung gegen Echinochloa crus-galli (ECHCG)**

| Beispielnummer | Dosierung [g/ha] | ECHCG |
|---|---|---|
| 1-10 | 1280 | 100 |
| 1-16 | 1280 | 100 |
| 1-16 | 320 | 90 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-23 | 1280 | 100 |
| 1-23 | 320 | 100 |
| 1-26 | 1280 | 100 |
| 1-26 | 320 | 100 |
| 1-31 | 1280 | 100 |
| 1-31 | 320 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 90 |
| 1-39 | 1280 | 90 |
| 1-40 | 1280 | 100 |
| 1-40 | 320 | 100 |
| 1-53 | 1280 | 100 |
| 1-53 | 320 | 100 |
| 1-55 | 1280 | 100 |
| 1-75 | 1280 | 100 |
| 1-86 | 1280 | 100 |

**Tabelle B2: Nachauflaufwirkung gegen Lolium rigidum (LOLRI)**

| Beispielnummer | Dosierung [g/ha] | LOLRI |
|---|---|---|
| 1-16 | 1280 | 100 |
| 1-17 | 1280 | 100 |
| 1-17 | 320 | 100 |
| 1-18 | 1280 | 100 |
| 1-18 | 320 | 90 |
| 1-19 | 1280 | 100 |
| 1-19 | 320 | 100 |
| 1-20 | 1280 | 90 |
| 1-20 | 320 | 90 |
| 1-21 | 1280 | 100 |
| 1-23 | 1280 | 100 |
| 1-31 | 1280 | 90 |
| 1-35 | 1280 | 100 |
| 1-36 | 1280 | 100 |
| 1-40 | 1280 | 100 |
| 1-40 | 320 | 100 |
| 1-72 | 1280 | 100 |

**Tabelle B3: Nachauflaufwirkung gegen Poa annua (POAAN)**

| Beispielnummer | Dosierung [g/ha] | POAAN |
|---|---|---|
| 1-3 | 1280 | 100 |
| 1-9 | 1280 | 100 |
| 1-9 | 320 | 100 |
| 1-10 | 1280 | 100 |
| 1-10 | 320 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-22 | 1280 | 100 |
| 1-22 | 320 | 100 |
| 1-23 | 1280 | 100 |
| 1-23 | 320 | 100 |
| 1-26 | 1280 | 100 |
| 1-26 | 320 | 100 |
| 1-28 | 1280 | 90 |
| 1-31 | 1280 | 100 |
| 1-31 | 320 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-60 | 320 | 90 |
| 1-61 | 1280 | 100 |
| 1-61 | 320 | 100 |
| 1-62 | 1280 | 100 |
| 1-62 | 320 | 100 |
| 1-67 | 1280 | 100 |
| 1-67 | 320 | 100 |
| 1-74 | 1280 | 100 |
| 1-74 | 320 | 100 |
| 1-77 | 1280 | 100 |
| 1-77 | 320 | 100 |
| 1-79 | 1280 | 100 |
| 1-79 | 320 | 100 |
| 1-81 | 1280 | 90 |
| 1-86 | 1280 | 100 |
| 1-86 | 320 | 100 |
| 1-89 | 1280 | 100 |
| 1-89 | 320 | 100 |

**Tabelle B4: Nachauflaufwirkung gegen Setaria viridis (SETVI)**

| Beispielnummer | Dosierung [g/ha] | SETVI |
|---|---|---|
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-21 | 1280 | 100 |
| 1-21 | 320 | 90 |
| 1-23 | 1280 | 90 |
| 1-31 | 1280 | 100 |
| 1-35 | 1280 | 100 |
| 1-36 | 1280 | 100 |
| 1-40 | 1280 | 100 |
| 1-40 | 320 | 100 |
| 1-53 | 1280 | 100 |
| 1-55 | 1280 | 100 |
| 1-55 | 320 | 100 |
| 1-72 | 1280 | 90 |

**Tabelle B5: Nachauflaufwirkung gegen Abutilon theophrasti (ABUTH)**

| Beispielnummer | Dosierung [g/ha] | ABUTH |
|---|---|---|
| 1-9 | 1280 | 100 |
| 1-9 | 320 | 100 |
| 1-10 | 1280 | 100 |
| 1-10 | 320 | 90 |
| 1-11 | 1280 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-22 | 1280 | 100 |
| 1-23 | 1280 | 100 |
| 1-23 | 320 | 100 |
| 1-25 | 1280 | 90 |
| 1-26 | 1280 | 100 |
| 1-26 | 320 | 90 |
| 1-27 | 1280 | 90 |
| 1-31 | 1280 | 100 |
| 1-31 | 320 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 90 |
| 1-40 | 1280 | 100 |
| 1-40 | 320 | 100 |
| 1-47 | 1280 | 100 |
| 1-47 | 320 | 90 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 90 |
| 1-61 | 1280 | 100 |
| 1-61 | 320 | 100 |
| 1-62 | 1280 | 100 |
| 1-62 | 320 | 100 |
| 1-67 | 1280 | 100 |
| 1-67 | 320 | 90 |
| 1-72 | 1280 | 100 |
| 1-72 | 320 | 100 |
| 1-74 | 1280 | 100 |
| 1-74 | 320 | 90 |
| 1-77 | 1280 | 90 |
| 1-79 | 1280 | 100 |
| 1-79 | 320 | 100 |
| 1-80 | 1280 | 100 |
| 1-81 | 1280 | 90 |
| 1-86 | 1280 | 100 |
| 1-86 | 320 | 100 |
| 1-89 | 1280 | 100 |
| 1-89 | 320 | 100 |

**Tabelle B6: Nachauflaufwirkung gegen Amaranthus retroflexus (AMARE)**

| Beispielnummer | Dosierung [g/ha] | AMARE |
|---|---|---|
| 1-3 | 1280 | 100 |
| 1-3 | 320 | 100 |
| 1-9 | 1280 | 100 |
| 1-9 | 320 | 100 |
| 1-10 | 1280 | 100 |
| 1-11 | 1280 | 100 |
| 1-11 | 320 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 90 |
| 1-23 | 1280 | 100 |
| 1-23 | 320 | 100 |
| 1-26 | 1280 | 100 |
| 1-28 | 1280 | 100 |
| 1-28 | 320 | 90 |
| 1-31 | 1280 | 100 |
| 1-31 | 320 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 90 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 90 |
| 1-60 | 1280 | 100 |
| 1-60 | 320 | 100 |
| 1-61 | 1280 | 100 |
| 1-61 | 320 | 100 |
| 1-62 | 1280 | 100 |
| 1-62 | 320 | 100 |
| 1-64 | 1280 | 100 |
| 1-64 | 320 | 100 |
| 1-65 | 1280 | 100 |
| 1-65 | 320 | 100 |
| 1-67 | 1280 | 100 |
| 1-72 | 1280 | 100 |
| 1-72 | 320 | 100 |
| 1-74 | 1280 | 100 |
| 1-74 | 320 | 100 |
| 1-77 | 1280 | 100 |
| 1-79 | 1280 | 100 |
| 1-79 | 320 | 100 |
| 1-80 | 1280 | 100 |
| 1-81 | 1280 | 100 |
| 1-86 | 1280 | 100 |
| 1-89 | 1280 | 100 |
| 1-89 | 320 | 100 |

**Tabelle B7: Nachauflaufwirkung gegen Matricaria inodora (MATIN)**

| Beispielnummer | Dosierung [g/ha] | MATIN |
|---|---|---|
| 1-17 | 1280 | 100 |
| 1-17 | 320 | 100 |
| 1-18 | 1280 | 100 |
| 1-19 | 1280 | 100 |
| 1-20 | 1280 | 100 |
| 1-40 | 1280 | 100 |
| 1-40 | 320 | 100 |
| 1-53 | 1280 | 100 |
| 1-59 | 1280 | 100 |
| 1-74 | 1280 | 100 |
| 1-74 | 320 | 100 |
| 1-75 | 1280 | 100 |
| 1-79 | 1280 | 90 |
| 1-86 | 1280 | 100 |
| 1-86 | 320 | 100 |
| 1-89 | 1280 | 100 |
| 1-89 | 320 | 100 |

**Tabelle B8: Nachauflaufwirkung gegen Stellaria media (STEME)**

| Beispielnummer | Dosierung [g/ha] | STEME |
|---|---|---|
| 1-3 | 1280 | 90 |
| 1-4 | 1280 | 100 |
| 1-9 | 1280 | 100 |
| 1-9 | 320 | 100 |
| 1-10 | 1280 | 100 |
| 1-10 | 320 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-23 | 1280 | 100 |
| 1-23 | 320 | 100 |
| 1-25 | 1280 | 100 |
| 1-26 | 1280 | 100 |
| 1-27 | 1280 | 100 |
| 1-27 | 320 | 100 |
| 1-28 | 1280 | 100 |
| 1-31 | 1280 | 100 |
| 1-31 | 320 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-53 | 1280 | 90 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-60 | 320 | 100 |
| 1-61 | 1280 | 100 |
| 1-61 | 320 | 100 |
| 1-62 | 1280 | 100 |
| 1-64 | 1280 | 100 |
| 1-67 | 1280 | 100 |
| 1-67 | 320 | 100 |
| 1-72 | 1280 | 100 |
| 1-72 | 320 | 100 |
| 1-74 | 1280 | 90 |
| 1-77 | 1280 | 100 |
| 1-77 | 320 | 100 |
| 1-79 | 1280 | 100 |
| 1-79 | 320 | 100 |
| 1-80 | 1280 | 100 |
| 1-81 | 1280 | 100 |
| 1-86 | 1280 | 100 |
| 1-86 | 320 | 100 |
| 1-89 | 1280 | 100 |
| 1-89 | 320 | 100 |

**Tabelle B9: Nachauflaufwirkung gegen Alopecurus myosuroides (ALOMY)**

| Beispielnummer | Dosierung [g/ha] | ALOMY |
|---|---|---|
| 1-10 | 1280 | 100 |
| 1-10 | 320 | 100 |
| 1-18 | 1280 | 100 |
| 1-18 | 320 | 100 |
| 1-20 | 1280 | 100 |
| 1-20 | 320 | 100 |
| 1-26 | 1280 | 100 |
| 1-26 | 320 | 100 |
| 1-31 | 1280 | 100 |
| 1-31 | 320 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-40 | 1280 | 100 |
| 1-40 | 320 | 100 |
| 1-53 | 1280 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-60 | 320 | 100 |
| 1-67 | 1280 | 90 |
| 1-72 | 1280 | 100 |
| 1-72 | 320 | 100 |
| 1-75 | 1280 | 90 |
| 1-79 | 1280 | 90 |
| 1-86 | 1280 | 100 |
| 1-86 | 320 | 100 |
| 1-89 | 1280 | 100 |
| 1-89 | 320 | 100 |

**Tabelle B10: Nachauflaufwirkung gegen Digitaria sanguinalis (DIGSA)**

| Beispielnummer | Dosierung [g/ha] | DIGSA |
|---|---|---|
| 1-10 | 1280 | 100 |
| 1-10 | 320 | 100 |
| 1-18 | 1280 | 100 |
| 1-18 | 320 | 100 |
| 1-20 | 1280 | 100 |
| 1-20 | 320 | 100 |
| 1-25 | 1280 | 100 |
| 1-26 | 1280 | 90 |
| 1-31 | 1280 | 100 |
| 1-31 | 320 | 90 |
| 1-35 | 1280 | 100 |
| 1-35 | 320 | 90 |
| 1-36 | 1280 | 100 |
| 1-36 | 320 | 100 |
| 1-38 | 1280 | 100 |
| 1-40 | 1280 | 100 |
| 1-40 | 320 | 100 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-67 | 1280 | 90 |
| 1-72 | 1280 | 100 |
| 1-72 | 320 | 90 |
| 1-77 | 1280 | 100 |
| 1-79 | 1280 | 100 |
| 1-89 | 1280 | 100 |

**Tabelle B11: Nachauflaufwirkung gegen Bassia scoparia (KCHSC)**

| Beispielnummer | Dosierung [g/ha] | KCHSC |
|---|---|---|
| 1-10 | 1280 | 100 |
| 1-18 | 1280 | 100 |
| 1-18 | 320 | 100 |
| 1-20 | 1280 | 100 |
| 1-20 | 320 | 100 |
| 1-26 | 1280 | 100 |
| 1-26 | 320 | 90 |
| 1-27 | 1280 | 90 |
| 1-31 | 1280 | 100 |
| 1-31 | 320 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 90 |
| 1-40 | 1280 | 100 |
| 1-40 | 320 | 100 |
| 1-47 | 1280 | 90 |
| 1-47 | 320 | 90 |
| 1-53 | 1280 | 100 |
| 1-53 | 320 | 90 |
| 1-55 | 1280 | 100 |
| 1-55 | 320 | 100 |
| 1-59 | 1280 | 90 |
| 1-60 | 1280 | 100 |
| 1-61 | 1280 | 100 |
| 1-61 | 320 | 100 |
| 1-64 | 1280 | 90 |
| 1-67 | 1280 | 90 |
| 1-72 | 1280 | 100 |
| 1-72 | 320 | 100 |
| 1-79 | 1280 | 90 |
| 1-79 | 320 | 90 |
| 1-86 | 1280 | 90 |
| 1-89 | 1280 | 100 |
| 1-89 | 320 | 90 |

**Tabelle B12: Nachauflaufwirkung gegen Veronica persica (VERPE)**

| Beispielnummer | Dosierung [g/ha] | VERPE |
|---|---|---|
| 1-10 | 1280 | 100 |
| 1-10 | 320 | 100 |
| 1-18 | 1280 | 100 |
| 1-18 | 320 | 100 |
| 1-20 | 1280 | 100 |
| 1-20 | 320 | 100 |
| 1-26 | 1280 | 100 |
| 1-27 | 1280 | 100 |
| 1-27 | 320 | 100 |
| 1-28 | 1280 | 90 |
| 1-31 | 1280 | 100 |
| 1-31 | 320 | 100 |
| 1-33 | 1280 | 100 |
| 1-33 | 320 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-60 | 320 | 100 |
| 1-61 | 1280 | 100 |
| 1-61 | 320 | 100 |
| 1-62 | 1280 | 100 |
| 1-62 | 320 | 100 |
| 1-64 | 1280 | 90 |
| 1-64 | 320 | 90 |
| 1-65 | 1280 | 100 |
| 1-65 | 320 | 100 |
| 1-67 | 1280 | 100 |
| 1-67 | 320 | 100 |
| 1-72 | 1280 | 100 |
| 1-72 | 320 | 100 |
| 1-74 | 1280 | 100 |
| 1-74 | 320 | 100 |
| 1-75 | 1280 | 100 |
| 1-77 | 1280 | 100 |
| 1-77 | 320 | 100 |
| 1-79 | 1280 | 100 |
| 1-79 | 320 | 100 |
| 1-80 | 1280 | 100 |
| 1-80 | 320 | 100 |
| 1-81 | 1280 | 100 |
| 1-81 | 320 | 100 |
| 1-86 | 1280 | 100 |
| 1-86 | 320 | 100 |
| 1-89 | 1280 | 100 |
| 1-89 | 320 | 100 |

Wie die Ergebnisse der Tabellen B1-B12 zeigen, weisen die Verbindungen Nr. 1-3, 1-4, 1-9, 1-10, 1-11, 1-16, 1-17, 1-18, 1-19, 1-20, 1-21, 1-22, 1-23, 1-25, 1-26, 1-27, 1-28, 1-31, 1-33, 1-35, 1-36, 1-38, 1-39, 1-40, 1-47, 1-53, 1-55, 1-59, 1-60, 1-61, 1-62, 1-64, 1-65, 1-67, 1-72, 1-74, 1-75, 1-77, 1-79, 1-80, 1-81, 1-86 und 1-89 bei Behandlung im Nachauflauf eine sehr gute herbizide Wirksamkeit gegen die Schadpflanzen *Abutilon theophrasti* (ABUTH), *Amaranthus retroflexus* (AMARE), *Echinochloa crus-galli* (ECHCG), *Lolium rigidum* (LOLRI), *Matricaria inodora* (MATIN), *Poa annua* (POAAN), *Setaria viridis* (SETVI), *Stellaria media* (STEME), *Alopecurus myosuroides* (ALOMY), *Digitaria sanguinalis* (DIGSA), *Bassia scoparia* (KCHSC) und *Veronica persica* (VERPE) bei einer Aufwandmenge von 1280 oder 320 g Aktivsubstanz pro Hektar auf.

### C. Herbizide Wirkung im Vorauflauf

Samen von mono- und dikotylen Unkrautpflanzen wurden in Kunststofftöpfen, in sandigem Lehmboden, ausgelegt (Doppelaussaaten mit jeweils einer Spezies mono- bzw. dikotyler Unkrautpflanzen pro Topf) und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion, unter Zusatz von 0,5% Additiv, mit einer Wasseraufwandmenge von umgerechnet 600 Liter pro Hektar auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert.

Beispielsweise bedeutet 100% Wirkung = Pflanzen sind abgestorben, 0% Wirkung = wie Kontrollpflanzen.

In nachstehenden Tabellen C1 bis C12 sind die Wirkungen ausgewählter Verbindungen der allgemeinen Formel (I) und Vergleichsverbindungen gemäß Tabelle 1 auf verschiedene Schadpflanzen und einer Aufwandmenge entsprechend 1280 oder 320 g/ha, die gemäß zuvor genannter Versuchvorschrift erhalten wurden, dargestellt.

**Tabelle C1: Vorauflaufwirkung gegen Echinochloa crus-galli (ECHCG)**

| Beispielnummer | Dosierung [g/ha] | ECHCG |
|---|---|---|
| 1-10 | 1280 | 90 |
| 1-11 | 1280 | 90 |
| 1-16 | 1280 | 90 |
| 1-17 | 1280 | 100 |
| 1-17 | 320 | 90 |
| 1-18 | 1280 | 100 |
| 1-18 | 320 | 90 |
| 1-19 | 1280 | 100 |
| 1-19 | 320 | 100 |
| 1-20 | 1280 | 90 |
| 1-20 | 320 | 90 |
| 1-23 | 1280 | 100 |
| 1-23 | 320 | 100 |
| 1-31 | 1280 | 100 |
| 1-35 | 1280 | 100 |
| 1-36 | 1280 | 100 |
| 1-38 | 1280 | 100 |
| 1-40 | 1280 | 100 |
| 1-40 | 320 | 100 |
| 1-53 | 1280 | 90 |
| 1-55 | 1280 | 100 |
| 1-55 | 320 | 100 |
| 1-59 | 1280 | 90 |
| 1-72 | 1280 | 100 |
| 1-72 | 320 | 100 |
| 1-86 | 1280 | 100 |

**Tabelle C2: Vorauflaufwirkung gegen Lolium rigidum (LOLRI)**

| Beispielnummer | Dosierung [g/ha] | LOLRI |
|---|---|---|
| 1-10 | 1280 | 90 |
| 1-16 | 1280 | 100 |
| 1-17 | 1280 | 100 |
| 1-17 | 320 | 100 |
| 1-18 | 1280 | 90 |
| 1-19 | 1280 | 100 |
| 1-19 | 320 | 100 |
| 1-20 | 1280 | 90 |
| 1-21 | 1280 | 100 |
| 1-23 | 1280 | 100 |
| 1-31 | 1280 | 100 |
| 1-35 | 1280 | 90 |
| 1-36 | 1280 | 90 |
| 1-40 | 1280 | 100 |
| 1-40 | 320 | 90 |
| 1-53 | 1280 | 100 |
| 1-53 | 320 | 90 |
| 1-55 | 1280 | 100 |
| 1-59 | 1280 | 100 |
| 1-64 | 1280 | 90 |
| 1-72 | 1280 | 100 |

**Tabelle C3: Vorauflaufwirkung gegen Poa annua (POAAN)**

| Beispielnummer | Dosierung [g/ha] | POAAN |
|---|---|---|
| 1-3 | 1280 | 90 |
| 1-9 | 1280 | 100 |
| 1-9 | 320 | 100 |
| 1-10 | 1280 | 100 |
| 1-10 | 320 | 100 |
| 1-11 | 1280 | 90 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 90 |
| 1-19 | 1280 | 100 |
| 1-19 | 320 | 100 |
| 1-20 | 1280 | 90 |
| 1-20 | 320 | 90 |
| 1-21 | 1280 | 100 |
| 1-21 | 320 | 100 |
| 1-22 | 1280 | 100 |
| 1-23 | 1280 | 100 |
| 1-23 | 320 | 90 |
| 1-25 | 1280 | 90 |
| 1-26 | 1280 | 100 |
| 1-26 | 320 | 90 |
| 1-31 | 1280 | 100 |
| 1-31 | 320 | 90 |
| 1-33 | 1280 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-53 | 1280 | 100 |
| 1-53 | 320 | 100 |
| 1-60 | 1280 | 100 |
| 1-61 | 1280 | 100 |
| 1-61 | 320 | 90 |
| 1-62 | 1280 | 100 |
| 1-62 | 320 | 90 |
| 1-64 | 1280 | 100 |
| 1-64 | 320 | 90 |
| 1-65 | 1280 | 100 |
| 1-67 | 1280 | 100 |
| 1-72 | 1280 | 100 |
| 1-72 | 320 | 100 |
| 1-74 | 1280 | 100 |
| 1-74 | 320 | 100 |
| 1-77 | 1280 | 100 |
| 1-79 | 1280 | 100 |
| 1-79 | 320 | 90 |
| 1-80 | 1280 | 90 |
| 1-81 | 1280 | 100 |
| 1-86 | 1280 | 100 |
| 1-86 | 320 | 90 |
| 1-89 | 1280 | 100 |
| 1-89 | 320 | 90 |

**Tabelle C4: Vorauflaufwirkung gegen Setaria viridis (SETVI)**

| Beispielnummer | Dosierung [g/ha] | SETVI |
|---|---|---|
| 1-3 | 1280 | 100 |
| 1-3 | 320 | 100 |
| 1-9 | 1280 | 100 |
| 1-10 | 1280 | 100 |
| 1-10 | 320 | 100 |
| 1-11 | 1280 | 90 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-22 | 1280 | 90 |
| 1-23 | 1280 | 100 |
| 1-23 | 320 | 100 |
| 1-25 | 1280 | 100 |
| 1-25 | 320 | 100 |
| 1-26 | 1280 | 90 |
| 1-28 | 1280 | 90 |
| 1-28 | 320 | 90 |
| 1-31 | 1280 | 100 |
| 1-31 | 320 | 100 |
| 1-33 | 1280 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-39 | 1280 | 90 |
| 1-40 | 1280 | 100 |
| 1-40 | 320 | 100 |
| 1-47 | 1280 | 90 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-61 | 1280 | 90 |
| 1-62 | 1280 | 100 |
| 1-64 | 1280 | 100 |
| 1-67 | 1280 | 100 |
| 1-72 | 1280 | 100 |
| 1-72 | 320 | 100 |
| 1-74 | 1280 | 100 |
| 1-77 | 1280 | 100 |
| 1-79 | 1280 | 100 |
| 1-80 | 1280 | 100 |
| 1-86 | 1280 | 100 |
| 1-89 | 1280 | 100 |
| 1-89 | 320 | 100 |

**Tabelle C5: Vorauflaufwirkung gegen Abutilon theophrasti (ABUTH)**

| Beispielnummer | Dosierung [g/ha] | ABUTH |
|---|---|---|
| 1-3 | 1280 | 100 |
| 1-3 | 320 | 90 |
| 1-9 | 1280 | 100 |
| 1-10 | 1280 | 100 |
| 1-10 | 320 | 100 |
| 1-11 | 1280 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 90 |
| 1-21 | 1280 | 90 |
| 1-21 | 320 | 90 |
| 1-23 | 1280 | 100 |
| 1-23 | 320 | 100 |
| 1-25 | 1280 | 100 |
| 1-26 | 1280 | 90 |
| 1-31 | 1280 | 100 |
| 1-31 | 320 | 90 |
| 1-33 | 1280 | 90 |
| 1-35 | 1280 | 100 |
| 1-35 | 320 | 100 |
| 1-36 | 1280 | 100 |
| 1-38 | 1280 | 100 |
| 1-38 | 320 | 100 |
| 1-40 | 1280 | 100 |
| 1-40 | 320 | 100 |
| 1-55 | 1280 | 100 |
| 1-55 | 320 | 100 |
| 1-59 | 1280 | 90 |
| 1-61 | 1280 | 100 |
| 1-62 | 1280 | 90 |
| 1-64 | 1280 | 90 |
| 1-72 | 1280 | 100 |
| 1-72 | 320 | 100 |
| 1-79 | 1280 | 90 |
| 1-81 | 1280 | 100 |
| 1-81 | 320 | 100 |
| 1-86 | 1280 | 100 |
| 1-86 | 320 | 90 |
| 1-89 | 1280 | 90 |

**Tabelle C6: Vorauflaufwirkung gegen Amaranthus retroflexus (AMARE)**

| Beispielnummer | Dosierung [g/ha] | AMARE |
|---|---|---|
| 1-3 | 1280 | 100 |
| 1-3 | 320 | 100 |
| 1-4 | 1280 | 90 |
| 1-9 | 1280 | 100 |
| 1-9 | 320 | 100 |
| 1-10 | 1280 | 100 |
| 1-10 | 320 | 100 |
| 1-11 | 1280 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 100 |
| 1-21 | 320 | 100 |
| 1-22 | 1280 | 100 |
| 1-23 | 1280 | 100 |
| 1-25 | 1280 | 100 |
| 1-25 | 320 | 100 |
| 1-27 | 1280 | 90 |
| 1-31 | 1280 | 100 |
| 1-31 | 320 | 100 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-61 | 1280 | 100 |
| 1-61 | 320 | 100 |
| 1-62 | 1280 | 100 |
| 1-62 | 320 | 90 |
| 1-64 | 1280 | 100 |
| 1-64 | 320 | 100 |
| 1-65 | 1280 | 100 |
| 1-65 | 320 | 90 |
| 1-67 | 1280 | 100 |
| 1-72 | 1280 | 100 |
| 1-72 | 320 | 100 |
| 1-74 | 1280 | 90 |
| 1-74 | 320 | 90 |
| 1-77 | 1280 | 100 |
| 1-77 | 320 | 90 |
| 1-79 | 1280 | 100 |
| 1-79 | 320 | 100 |
| 1-80 | 1280 | 100 |
| 1-80 | 320 | 100 |
| 1-81 | 1280 | 100 |
| 1-86 | 1280 | 100 |
| 1-86 | 320 | 100 |
| 1-89 | 1280 | 100 |
| 1-89 | 320 | 100 |

**Tabelle C7: Vorauflaufwirkung gegen Matricaria inodora (MATIN)**

| Beispielnummer | Dosierung [g/ha] | MATIN |
|---|---|---|
| 1-3 | 1280 | 90 |
| 1-9 | 1280 | 90 |
| 1-10 | 1280 | 100 |
| 1-10 | 320 | 100 |
| 1-11 | 1280 | 90 |
| 1-16 | 1280 | 100 |
| 1-16 | 320 | 90 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-21 | 1280 | 90 |
| 1-23 | 1280 | 100 |
| 1-23 | 320 | 90 |
| 1-25 | 1280 | 90 |
| 1-26 | 1280 | 100 |
| 1-31 | 1280 | 90 |
| 1-35 | 1280 | 100 |
| 1-35 | 320 | 90 |
| 1-38 | 1280 | 100 |
| 1-39 | 1280 | 90 |
| 1-40 | 1280 | 100 |
| 1-40 | 320 | 100 |
| 1-53 | 1280 | 100 |
| 1-53 | 320 | 100 |
| 1-60 | 1280 | 100 |
| 1-64 | 1280 | 90 |
| 1-72 | 1280 | 100 |
| 1-72 | 320 | 90 |
| 1-79 | 1280 | 100 |
| 1-79 | 320 | 100 |
| 1-80 | 1280 | 90 |
| 1-81 | 1280 | 90 |
| 1-86 | 1280 | 100 |
| 1-86 | 320 | 90 |
| 1-89 | 1280 | 90 |
| 1-89 | 320 | 90 |

**Tabelle C8: Vorauflaufwirkung gegen Stellaria media (STEME)**

| Beispielnummer | Dosierung [g/ha] | STEME |
|---|---|---|
| 1-3 | 1280 | 100 |
| 1-3 | 320 | 90 |
| 1-9 | 1280 | 100 |
| 1-9 | 320 | 100 |
| 1-10 | 1280 | 100 |
| 1-10 | 320 | 100 |
| 1-11 | 1280 | 100 |
| 1-16 | 1280 | 100 |
| 1-16 | 320 | 100 |
| 1-17 | 1280 | 100 |
| 1-17 | 320 | 100 |
| 1-18 | 1280 | 100 |
| 1-18 | 320 | 100 |
| 1-19 | 1280 | 100 |
| 1-19 | 320 | 100 |
| 1-20 | 1280 | 100 |
| 1-20 | 320 | 100 |
| 1-22 | 1280 | 100 |
| 1-22 | 320 | 90 |
| 1-23 | 1280 | 100 |
| 1-23 | 320 | 100 |
| 1-25 | 1280 | 100 |
| 1-25 | 320 | 90 |
| 1-26 | 1280 | 100 |
| 1-26 | 320 | 100 |
| 1-27 | 1280 | 90 |
| 1-28 | 1280 | 90 |
| 1-31 | 1280 | 100 |
| 1-31 | 320 | 100 |
| 1-33 | 1280 | 100 |
| 1-33 | 320 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-38 | 320 | 100 |
| 1-39 | 1280 | 100 |
| 1-39 | 320 | 100 |
| 1-40 | 1280 | 100 |
| 1-40 | 320 | 100 |
| 1-47 | 1280 | 100 |
| 1-47 | 320 | 100 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-60 | 320 | 100 |
| 1-61 | 1280 | 100 |
| 1-64 | 1280 | 100 |
| 1-67 | 1280 | 100 |
| 1-67 | 320 | 100 |
| 1-72 | 1280 | 100 |
| 1-72 | 320 | 90 |
| 1-77 | 1280 | 100 |
| 1-77 | 320 | 100 |
| 1-79 | 1280 | 100 |
| 1-79 | 320 | 100 |
| 1-81 | 1280 | 100 |
| 1-86 | 1280 | 100 |
| 1-86 | 320 | 90 |
| 1-89 | 1280 | 90 |
| 1-89 | 320 | 90 |

**Tabelle C9: Vorauflaufwirkung gegen Alopecurus myosuroides (ALOMY)**

| Beispielnummer | Dosierung [g/ha] | ALOMY |
|---|---|---|
| 1-10 | 1280 | 100 |
| 1-10 | 320 | 100 |
| 1-18 | 1280 | 90 |
| 1-20 | 1280 | 100 |
| 1-20 | 320 | 90 |
| 1-26 | 1280 | 90 |
| 1-31 | 1280 | 100 |
| 1-35 | 1280 | 90 |
| 1-36 | 1280 | 100 |
| 1-36 | 320 | 90 |
| 1-38 | 1280 | 100 |
| 1-38 | 320 | 90 |
| 1-40 | 1280 | 100 |
| 1-40 | 320 | 100 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-72 | 1280 | 100 |
| 1-72 | 320 | 100 |
| 1-74 | 1280 | 100 |
| 1-77 | 1280 | 100 |
| 1-79 | 1280 | 90 |
| 1-81 | 1280 | 90 |
| 1-86 | 1280 | 90 |
| 1-86 | 320 | 90 |
| 1-89 | 1280 | 100 |

**Tabelle C10: Vorauflaufwirkung gegen Digitaria sanguinalis (DIGSA)**

| Beispielnummer | Dosierung [g/ha] | DIGSA |
|---|---|---|
| 1-10 | 1280 | 100 |
| 1-10 | 320 | 100 |
| 1-18 | 1280 | 100 |
| 1-18 | 320 | 100 |
| 1-20 | 1280 | 100 |
| 1-20 | 320 | 90 |
| 1-26 | 1280 | 90 |
| 1-31 | 1280 | 100 |
| 1-31 | 320 | 90 |
| 1-35 | 1280 | 100 |
| 1-36 | 1280 | 100 |
| 1-36 | 320 | 100 |
| 1-38 | 1280 | 100 |
| 1-39 | 1280 | 90 |
| 1-40 | 1280 | 100 |
| 1-40 | 320 | 100 |
| 1-47 | 1280 | 90 |
| 1-53 | 1280 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-61 | 1280 | 100 |
| 1-62 | 1280 | 100 |
| 1-64 | 1280 | 90 |
| 1-67 | 1280 | 100 |
| 1-72 | 1280 | 100 |
| 1-72 | 320 | 100 |
| 1-74 | 1280 | 100 |
| 1-74 | 320 | 90 |
| 1-77 | 1280 | 100 |
| 1-79 | 1280 | 100 |
| 1-80 | 1280 | 100 |
| 1-81 | 1280 | 100 |
| 1-86 | 1280 | 100 |
| 1-89 | 1280 | 100 |
| 1-89 | 320 | 90 |

**Tabelle C11: Vorauflaufwirkung gegen Bassia scoparia (KCHSC)**

| Beispielnummer | Dosierung [g/ha] | KCHSC |
|---|---|---|
| 1-18 | 1280 | 90 |
| 1-20 | 1280 | 90 |
| 1-31 | 1280 | 100 |
| 1-31 | 320 | 90 |
| 1-35 | 1280 | 90 |
| 1-36 | 1280 | 90 |
| 1-38 | 1280 | 100 |
| 1-38 | 320 | 100 |
| 1-40 | 1280 | 100 |
| 1-40 | 320 | 100 |
| 1-47 | 1280 | 90 |
| 1-55 | 1280 | 100 |
| 1-55 | 320 | 100 |
| 1-61 | 1280 | 100 |
| 1-72 | 1280 | 100 |
| 1-72 | 320 | 100 |
| 1-86 | 1280 | 100 |
| 1-89 | 1280 | 90 |

**Tabelle C12: Vorauflaufwirkung gegen Veronica persica (VERPE)**

| Beispielnummer | Dosierung [g/ha] | VERPE |
|---|---|---|
| 1-10 | 1280 | 100 |
| 1-10 | 320 | 100 |
| 1-18 | 1280 | 100 |
| 1-18 | 320 | 100 |
| 1-20 | 1280 | 100 |
| 1-20 | 320 | 100 |
| 1-25 | 1280 | 90 |
| 1-26 | 1280 | 90 |
| 1-27 | 1280 | 100 |
| 1-31 | 1280 | 100 |
| 1-31 | 320 | 100 |
| 1-33 | 1280 | 90 |
| 1-35 | 1280 | 100 |
| 1-35 | 320 | 100 |
| 1-36 | 1280 | 100 |
| 1-36 | 320 | 100 |
| 1-38 | 1280 | 100 |
| 1-39 | 1280 | 100 |
| 1-40 | 1280 | 100 |
| 1-40 | 320 | 100 |
| 1-47 | 1280 | 100 |
| 1-47 | 320 | 90 |
| 1-53 | 1280 | 100 |
| 1-53 | 320 | 100 |
| 1-55 | 1280 | 100 |
| 1-55 | 320 | 100 |
| 1-59 | 1280 | 100 |
| 1-59 | 320 | 100 |
| 1-60 | 1280 | 100 |
| 1-61 | 1280 | 100 |
| 1-62 | 1280 | 90 |
| 1-64 | 1280 | 100 |
| 1-64 | 320 | 90 |
| 1-67 | 1280 | 100 |
| 1-72 | 1280 | 100 |
| 1-72 | 320 | 100 |
| 1-74 | 1280 | 90 |
| 1-77 | 1280 | 100 |
| 1-77 | 320 | 90 |
| 1-79 | 1280 | 100 |
| 1-80 | 1280 | 100 |
| 1-81 | 1280 | 100 |
| 1-86 | 1280 | 100 |
| 1-86 | 320 | 90 |
| 1-89 | 1280 | 90 |

Wie die Ergebnisse der Tabellen C1-C12 zeigen, weisen die Verbindungen Nr. 1-3, 1-4, 1-9, 1-10, 1-11, 1-16, 1-17, 1-18, 1-19, 1-20, 1-21, 1-22, 1-23, 1-25, 1-26, 1-27, 1-28, 1-31, 1-33, 1-35, 1-36, 1-38, 1-39, 1-40, 1-47, 1-53, 1-55, 1-59, 1-60, 1-61, 1-62, 1-64, 1-65, 1-67, 1-72, 1-74, 1-77, 1-79, 1-80, 1-81, 1-86 und 1-89 bei Behandlung im Vorauflauf eine sehr gute herbizide Wirksamkeit gegen die Schadpflanzen *Abutilon theophrasti* (ABUTH), *Amaranthus retroflexus* (AMARE), *Echinochloa crus-galli* (ECHCG), *Lolium rigidum* (LOLRI), *Matricaria inodora* (MATIN), *Poa annua* (POAAN), *Setaria viridis* (SETVI), *Stellaria media* (STEME), *Alopecurus myosuroides* (ALOMY), *Digitaria sanguinalis* (DIGSA), *Bassia scoparia* (KCHSC) und *Veronica persica* (VERPE) bei einer Aufwandmenge von 1280 oder 320 g Aktivsubstanz pro Hektar auf.

### D. Herbizide Wirkung im Vorauflauf

Samen von mono- und dikotylen Unkrautpflanzen wurden in Kunststoff- oder organischen Pflanztöpfen ausgelegt (Einzelaussaaten mit einer Spezies mono- oder dikotyler Unkrautpflanze pro Topf) und mit Erde abgedeckt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

In nachstehenden Tabellen D1 bis D8 sind die Wirkungen ausgewählter Verbindungen der allgemeinen Formel (I) und Vergleichsverbindungen gemäß Tabelle 1 auf verschiedene Schadpflanzen und einer Aufwandmenge entsprechend 80 g/ha, die gemäß zuvor genannter Versuchvorschrift erhalten wurden, dargestellt.

**Tabelle D1: Vorauflaufwirkung gegen Alopecurus myosuroides (ALOMY)**

| Beispielnummer | Dosierung [g/ha] | ALOMY |
|---|---|---|
| 1-17 | 80 | 100 |
| 1-19 | 80 | 100 |

**Tabelle D2: Vorauflaufwirkung gegen Digitaria sanguinalis (DIGSA)**

| Beispielnummer | Dosierung [g/ha] | DIGSA |
|---|---|---|
| 1-17 | 80 | 90 |
| 1-19 | 80 | 100 |
| 1-35 | 80 | 100 |
| 1-36 | 80 | 100 |

**Tabelle D3: Vorauflaufwirkung gegen Setaria viridis (SETVI)**

| Beispielnummer | Dosierung [g/ha] | SETVI |
|---|---|---|
| 1-17 | 80 | 100 |
| 1-19 | 80 | 100 |
| 1-35 | 80 | 100 |
| 1-36 | 80 | 80 |

**Tabelle D4: Vorauflaufwirkung gegen Abutilon theophrasti (ABUTH)**

| Beispielnummer | Dosierung [g/ha] | ABUTH |
|---|---|---|
| 1-17 | 80 | 100 |
| 1-19 | 80 | 100 |
| 1-35 | 80 | 80 |
| 1-36 | 80 | 100 |

**Tabelle D5: Vorauflaufwirkung gegen Amaranthus retroflexus (AMARE)**

| Beispielnummer | Dosierung [g/ha] | AMARE |
|---|---|---|
| 1-17 | 80 | 100 |
| 1-19 | 80 | 100 |
| 1-35 | 80 | 100 |
| 1-36 | 80 | 100 |

**Tabelle D6: Vorauflaufwirkung gegen Matricaria inodora (MATIN)**

| Beispielnummer | Dosierung [g/ha] | MATIN |
|---|---|---|
| 1-17 | 80 | 100 |
| 1-19 | 80 | 100 |
| 1-35 | 80 | 80 |
| 1-36 | 80 | 100 |

**Tabelle D7: Vorauflaufwirkung gegen Viola tricolor (VIOTR)**

| Beispielnummer | Dosierung [g/ha] | VIOTR |
|---|---|---|
| 1-17 | 80 | 100 |
| 1-19 | 80 | 100 |
| 1-35 | 80 | 90 |
| 1-36 | 80 | 100 |

**Tabelle D8: Vorauflaufwirkung gegen Veronica persica (VERPE)**

| Beispielnummer | Dosierung [g/ha] | VERPE |
|---|---|---|
| 1-17 | 80 | 100 |
| 1-19 | 80 | 100 |
| 1-35 | 80 | 100 |
| 1-36 | 80 | 100 |

Wie die Ergebnisse der Tabellen D1-D8 zeigen, weisen die Verbindungen Nr. 1-17, 1-19, 1-35 und 1-36 bei Behandlung im Vorauflauf eine sehr gute herbizide Wirksamkeit gegen die Schadpflanzen *Alopecurus myosuroides* (ALOMY), *Digitaria sanguinalis* (DIGSA), *Setaria viridis* (SETVI), *Abutilon theophrasti* (ABUTH), *Amaranthus retroflexus* (AMARE), *Matricaria inodora* (MATIN), *Viola tricolor* (VIOTR) und *Veronica persica* (VERPE) bei einer Aufwandmenge von 80 g Aktivsubstanz pro Hektar auf.

### E. Kulturpflanzenverträglichkeit im Vorauflauf

Samen von mono- bzw. dikotylen Kulturpflanzen wurden in Kunststoff- oder organischen Pflanztöpfen ausgelegt und mit Erde abgedeckt. Die in Form von benetzbaren Pulvern (WP) oder als Emulsionskonzentrate (EC) formulierten Verbindungen wurden dann als wässrige Suspension bzw. Emulsion unter Zusatz von 0,5% Additiv mit einer Wasseraufwandmenge von umgerechnet 600 l/ha auf die Oberfläche der Abdeckerde appliziert. Nach der Behandlung wurden die Töpfe im Gewächshaus aufgestellt und unter guten Wachstumsbedingungen für die Testpflanzen gehalten. Nach ca. 3 Wochen wurde die Wirkung der Präparate visuell im Vergleich zu unbehandelten Kontrollen in Prozentwerten bonitiert. Beispielsweise bedeutet 100% Wirkung = Pflanzen sind abgestorben, 0% Wirkung = wie Kontrollpflanzen.

**Tabelle E1: Kulturpflanzenverträglichkeit im Vorauflauf am Beispiel von Soja (GLXMA, var. Sultana), Weizen (TRZAS, var. Triso) und Mais (ZEAMX, var. Aventura)**

| Struktur | Beispielnummer | Dosierung [g/ha] | GLXMA | TRZAS | ZEAMX |
|---|---|---|---|---|---|
| 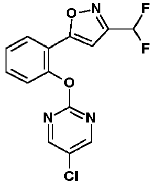 | US 2016/333000 Nr. 53 | 80 | 80 | 20 | 50 |
| 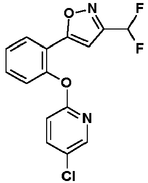 | Verbindung 1-35 | 80 | 20 | 0 | 10 |
| 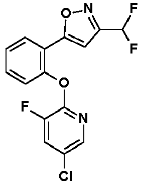 | Verbindung 1-36 | 80 | 10 | 0 | 20 |
| 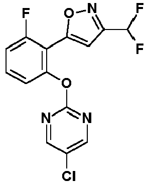 | US 2016/333000 Nr. 144 | 80 | 100 | 70 | 30 |
| 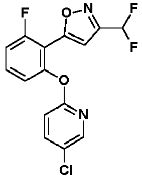 | Verbindung 1-17 | 80 | 20 | 20 | 0 |
| 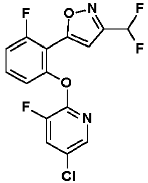 | Verbindung 1-19 | 80 | 20 | 30 | 10 |

Wie die Ergebnisse in der Tabelle E1 zeigen, weisen die Verbindungen Nr. 1-17, 1-19, 1-35 und 1-36 im Vergleich mit aus der Literatur (WO2015/108779; US 2016/333000 (Bsp. Nr. 53 und 144)) bekannten, strukturähnlichen Pyrimidinyloxybenzolen bei Applikation im Vorauflauf auf Soja (GLXMA, var. Sultana), Weizen (TRZAS, var. Triso) und Mais (ZEAMX, var. Aventura) bei einer Aufwandmenge von 80 g Aktivsubstanz pro Hektar eine deutlich verbesserte Kulturpflanzenverträglichkeit auf.

## Patentansprüche

1. Substituierte Pyridinyloxybenzole der allgemeinen Formel (I) oder deren Salze 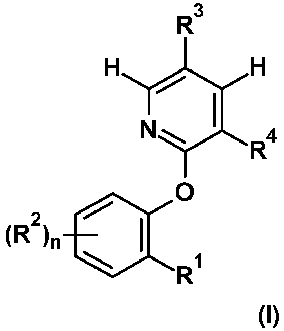 worin
R¹ für ein gegebenenfalls substituiertes Pyridin oder Pyrimidin steht, das optional mit bis zu 4 Substituenten, unabhängig voneinander ausgewählt aus der Gruppe R⁵, substituiert ist,
R² unabhängig voneinander für Halogen, Hydroxy, Amino, Cyano, Nitro, Formyl, Formamid, (C₁-C₄)-Alkyl, (C₁-C₄)-Haloalkyl, (C₃-C₆)-Cycloalkyl, (C₂-C₄)-Alkenyl, (C₂-C₄)-Alkinyl, (C₂-C₄)-Haloalkenyl, (C₂-C₄)-Haloalkinyl, (C₁-C₄)-Alkoxy-(C₁-C₄)-alkyl, (C₁-C₄)-Haloalkoxy-(C₁-C₄)-alkyl, (C₁-C₄)-Alkylthio-(C₁-C₄)-alkyl, (C₁-C₄)-Alkylsulfinyl-(C₁-C₄)-alkyl, (C₁-C₄)-Alkylsulfonyl-(C₁-C₄)-alkyl, (C₁-C₄)-Alkylcarbonyl, (C₁-C₄)-Haloalkylcarbonyl, (C₃-C₆)-Cycloalkylcarbonyl, Carboxyl, (C₁-C₄)-Alkoxycarbonyl, (C₁-C₄)-Haloalkoxycarbonyl, (C₃-C₆)-Cycloalkoxycarbonyl, (C₁-C₄)-Alkylaminocarbonyl, (C₂-C₆)-Dialkylaminocarbonyl, (C₃-C₆)-Cycloalkylaminocarbonyl, (C₁-C₄)-Alkylcarbonylamino, (C₁-C₄)-Haloalkylcarbonylamino, (C₂-C₆)-Cycloalkylcarbonylamino, (C₁-C₄)-Alkoxycarbonylamino, (C₁-C₄)-Alkylaminocarbonylamino, (C₂-C₆)-Dialkylaminocarbonylamino, Carboxy-(C₁-C₄)-alkyl, (C₁-C₄)-Alkoxycarbonyl-(C₁-C₄)-alkyl, (C₁-C₄)-Haloalkoxycarbonyl-(C₁-C₄)-alkyl, (C₃-C₆)-Cycloalkoxycarbonyl-(C₁-C₄)-alkyl, (C₁-C₄)-Alkoxy, (C₁-C₄)-Haloalkoxy, (C₃-C₆)-Cycloalkoxy, (C₁-C₄)-Alkenyloxy, (C₁-C₄)-Alkinyloxy, (C₁-C₄)-Alkylthio, (C₁-C₄)-Haloalkylthio, (C₃-C₆)-Cycloalkylthio, (C₁-C₄)-Alkylsulfinyl, (C₁-C₄)-Haloalkylsulfinyl, (C₃-C₆)-Cycloalkylsulfinyl, (C₁-C₄)-Alkylsulfonyl, (C₁-C₄)-Haloalkylsulfonyl, (C₃-C₆)-Cycloalkylsulfonyl, (C₁-C₄)-Alkylaminosulfonyl, (C₂-C₆)-Dialkylaminosulfonyl oder (C₃-C₆)-Trialkylsilyl steht,
n ist gleich 0, 1, 2, 3, oder 4,
R³ für Wasserstoff, Halogen, Cyano, (C₁-C₄)-Alkyl oder (C₁-C₄)-Haloalkyl steht,
R⁴ für Wasserstoff oder Halogen steht,
und
R⁵ für Wasserstoff, Halogen, Hydroxy, Amino, Cyano, Formyl, (C₁-C₄)-Alkyl, (C₁-C₄)-Haloalkyl, (C₁-C₄)-Alkoxy, (C₁-C₄)-Haloalkoxy, (C₁-C₄)-Alkoxycarbonyl oder (C₁-C₄)-Alkoxythiocarbonyl steht,
ausgenommen 2-Chlor-4-(2-pyridin-2-yloxyphenyl)pyridin.

2. Verbindung der allgemeinen Formel (I) nach Anspruch 1 oder deren Salze, worin
R¹ für ein gegebenenfalls substituiertes Pyridin oder Pyrimidin steht, das optional mit bis zu 4 Substituenten, unabhängig voneinander ausgewählt aus der Gruppe R⁵, substituiert ist,
R² unabhängig voneinander für Halogen, Hydroxy, Amino, Cyano, Nitro, Formyl, (C₁-C₄)-Alkyl, (C₁-C₄)-Haloalkyl, (C₃-C₆)-Cycloalkyl, Carboxyl, (C₁-C₄)-Alkoxycarbonyl, (C₁-C₄)-Haloalkoxycarbonyl, (C₁-C₄)-Alkylaminocarbonyl, (C₂-C₆)-Dialkylaminocarbonyl, (C₁-C₄)-Alkylcarbonylamino, Carboxy-(C₁-C₄)-alkyl, (C₁-C₄)-Alkoxycarbonyl-(C₁-C₄)-alkyl, (C₁-C₄)-Alkoxy, (C₁-C₄)-Haloalkoxy, (C₁-C₄)-Alkenyloxy, (C₁-C₄)-Alkinyloxy oder (C₁-C₄)-Alkylthio steht,
n ist gleich 0, 1, 2, oder 3,
R³ für Wasserstoff, Halogen, Cyano, (C₁-C₄)-Alkyl oder (C₁-C₄)-Haloalkyl steht,
R⁴ für Wasserstoff oder Halogen steht, und R⁵ für Wasserstoff, Halogen, Formyl, (C₁-C₄)-Alkyl, (C₁-C₄)-Haloalkyl, (C₁-C₄)-Alkoxy, (C₁-C₄)-Haloalkoxy, (C₁-C₄)-Alkoxycarbonyl oder (C₁-C₄)-Alkoxythiocarbonyl steht,
ausgenommen 2-Chlor-4-(2-pyridin-2-yloxyphenyl)pyridin.

3. Verbindung der allgemeinen Formel (I) nach Anspruch 1 oder deren Salze, worin
R¹ für ein gegebenenfalls substituiertes 2-Pyridin, 3-Pyridin, 4-Pyridin oder 4-Pyrimidin steht, das optional mit bis zu 3 Substituenten, unabhängig voneinander ausgewählt aus der Gruppe R⁵, substituiert ist,
R² unabhängig voneinander für Fluor, Chlor, Brom, Amino, Hydoxy, Nitro, Cyano, Carboxyl, Methyl, Ethyl, Cyclopropyl, Trifluormethyl, Methoxy, Ethoxy, Allyloxy, But-2-ynoxy oder Methylthio steht,
n ist gleich 0, 1 oder 2,
R³ für Wasserstoff, Fluor, Chlor, Brom, Cyano, Methyl, Ethyl, oder Trifluormethyl steht,
R⁴ für Wasserstoff, Fluor oder Chlor steht,
und
R⁵ für Wasserstoff, Chlor, Formyl, Methyl, Difluormethyl, Trifluormethyl, Chlorfluormethyl, Chlordifluormethyl, Methoxycarbonyl oder Methoxythiocarbonyl steht,
ausgenommen 2-Chlor-4-(2-pyridin-2-yloxyphenyl)pyridin.

4. Herbizide Mittel, **gekennzeichnet durch** einen herbizid wirksamen Gehalt an mindestens einer Verbindung der allgemeinen Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend Cyprosulfamid, Cloquintocet-mexyl, Mefenpyr-diethyl oder Isoxadifen-ethyl.

9. Herbizide Mittel nach einem der Ansprüche 4 bis 8 enthaltend ein weiteres Herbizid.

10. Verfahren zur Bekämpfung unerwünschter Pflanzen, **dadurch gekennzeichnet, dass** man eine wirksame Menge mindestens einer Verbindung der allgemeinen Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der allgemeinen Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, dass** die Verbindungen der allgemeinen Formel (I) zur Bekämpfung unerwünschter Pflanzen in Kulturen von Nutzpflanzen eingesetzt werden.

13. Verwendung nach Anspruch 12, **dadurch gekennzeichnet, dass** die Nutzpflanzen transgene Nutzpflanzen sind.

## Claims

1. Substituted pyridinyloxybenzenes of the general formula (I) or salts thereof 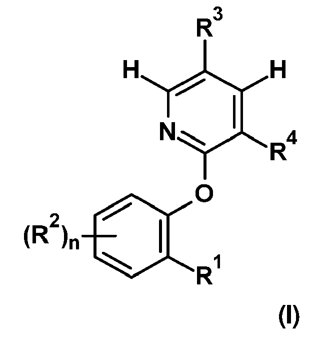 in which
R¹ represents an optionally substituted pyridine or pyrimidine which is optionally substituted by up to 4 substituents independently of one another selected from the group R⁵,
R² independently of the others represents halogen, hydroxy, amino, cyano, nitro, formyl, formamide, (C₁-C₄) -alkyl, (C₁-C₄) -haloalkyl, (C₃-C₆)-cycloalkyl, (C₂-C₄) -alkenyl, (C₂-C₄)-alkynyl, (C₂-C₄) -haloalkenyl, (C₂-C₄)-haloal-kynyl, (C₁-C₄) -alkoxy- (C₁-C₄) -alkyl, (C₁-C₄)-haloalkoxy- (C₁-C₄) -alkyl, (C₁-C₄)-alkylthio-(C₁-C₄) -alkyl, (C₁-C₄)-alkylsulfinyl-(C₁-C₄)-alkyl, (C₁-C₄)-alkylsulfonyl-(C₁-C₄)-alkyl, (C₁-C₄)-alkylcarbonyl, (C₁-C₄)-haloalkylcarbonyl, (C₃-C₆) -cycloalkylcarbonyl, carboxyl, (C₁-C₄)-alkoxycarbonyl, (C₁-C₄)-haloalkoxycarbonyl, (C₃-C₆) -cycloalkoxycarbonyl, (C₁-C₄)-alkyl-aminocarbonyl, (C₂-C₆)-dialkylaminocarbonyl, (C₃-C₆)-cycloalkylaminocarbonyl, (C₁-C₄)-alkyl-carbonylamino, (C₁-C₄)-haloalkylcarbonylamino, (C₂-C₆) -cycloalkylcarbonylamino, (C₁-C₄)-alk-oxycarbonylamino, (C₁-C₄)-alkylaminocarbonylamino, (C₂-C₆)-dialkylaminocarbonylamino, carboxy- (C₁-C₄)-alkyl, (C₁-C₄)-alkoxycarbonyl-(C₁-C₄) -alkyl, (C₁-C₄)-haloalkoxycarbonyl-(C₁-C₄)-alkyl, (C₃-C₆)-cycloalkoxycarbonyl-(C₁-C₄₎-alkyl, (C₁-C₄) -alkoxy, (C₁-C₄) -haloalkoxy, (C₃-C₆)-cycloalkoxy, (C₁-C₄)-alkenyloxy, (C₁-C₄)-alkynyloxy, (C₁-C₄)-alkylthio, (C₁-C₄)-haloalkylthio, (C₃-C₆)-cycloalkylthio, (C₁-C₄)-alkylsulfinyl, (C₁-C₄)-haloalkylsulfinyl, (C₃-C₆)-cycloalkylsulfinyl, (C₁-C₄)-alkylsulfonyl, (C₁-C₄)-haloalkylsulfonyl, (C₃-C₆)-cycloalkyl-sulfonyl, (C₁-C₄)-alkylaminosulfonyl, (C₂-C₆)-dialkylaminosulfonyl or (C₃-C₆)-trialkylsilyl,
n is 0, 1, 2, 3 or 4,
R³ represents hydrogen, halogen, cyano, (C₁-C₄)-alkyl or (C₁-C₄)-haloalkyl,
R⁴ represents hydrogen or halogen,
and R⁵ represents hydrogen, halogen, hydroxy, amino, cyano, formyl, (C₁-C₄)-alkyl, (C₁-C₄)-halo-alkyl, (C₁-C₄) -alkoxy, (C₁-C₄) -haloalkoxy, (C₁-C₄)-alkoxycarbonyl or (C₁-C₄)-alkoxythio-carbonyl,
except for 2-chloro-4-(2-pyridin-2-yloxyphenyl)-pyridine.

2. Compound of the general formula (I) according to Claim 1 or salts thereof, in which
R¹ represents an optionally substituted pyridine or pyrimidine which is optionally substituted by up to 4 substituents independently of one another selected from the group R⁵,
R² independently of the others represents halogen, hydroxy, amino, cyano, nitro, formyl, (C₁-C₄)-alkyl, (C₁-C₄) -haloalkyl, (C₃-C₆)-cycloalkyl, carboxyl, (C₁-C₄)-alkoxycarbonyl, (C₁-C₄)-haloalkoxycarbonyl, (C₁-C₄)-alkylaminocarbonyl, (C₂-C₆)-dialkylaminocarbonyl, (C₁-C₄)-alkylcarbonylamino, carboxy-(C₁-C₄)-alkyl, (C₁-C₄)-alkoxycarbonyl-(C₁-C₄)-alkyl, (C₁-C₄)-alkoxy, (C₁-C₄)-haloalkoxy, (C₁-C₄)-alkenyloxy, (C₁-C₄)-alkynyloxy or (C₁-C₄)-alkylthio,
n is 0, 1, 2 or 3,
R³ represents hydrogen, halogen, cyano, (C₁-C₄)-alkyl or (C₁-C₄)-haloalkyl,
R⁴ represents hydrogen or halogen,
and
R⁵ represents hydrogen, halogen, formyl, (C₁-C₄)-alkyl, (C₁-C₄)-haloalkyl, (C₁-C₄)-alkoxy, (C₁-C₄)-haloalkoxy, (C₁-C₄)-alkoxycarbonyl or (C₁-C₄)-alkoxythiocarbonyl, except for 2-chloro-4-(2-pyridin-2-yloxyphenyl)-pyridine.

3. Compound of the general formula (I) according to Claim 1 or salts thereof, in which
R¹ represents an optionally substituted 2-pyridine, 3-pyridine, 4-pyridine or 4-pyrimidine which is optionally substituted by up to 3 substituents independently of one another selected from the group R⁵,
R² independently of the others represents fluorine, chlorine, bromine, amino, hydroxy, nitro, cyano, carboxyl, methyl, ethyl, cyclopropyl, trifluoromethyl, methoxy, ethoxy, allyloxy, but-2-ynoxy or methylthio,
n is 0, 1 or 2,
R³ represents hydrogen, fluorine, chlorine, bromine, cyano, methyl, ethyl or trifluoromethyl,
R⁴ represents hydrogen, fluorine or chlorine,
and
R⁵ represents hydrogen, chlorine, formyl, methyl, difluoromethyl, trifluoromethyl, chlorofluoromethyl, chlorodifluoromethyl, methoxycarbonyl or methoxythiocarbonyl,
except for 2-chloro-4-(2-pyridin-2-yloxyphenyl)-pyridine.

4. Herbicidal compositions **characterized by** a herbicidally active content of at least one compound of the general formula (I) according to any of Claims 1 to 3.

5. Herbicidal compositions according to Claim 4 in a mixture with formulation auxiliaries.

6. Herbicidal compositions according to Claim 4 or 5, comprising at least one further pesticidally active substance from the group consisting of insecticides, acaricides, herbicides, fungicides, safeners, and growth regulators.

7. Herbicidal compositions according to Claim 6, comprising a safener.

8. Herbicidal compositions according to Claim 7, comprising cyprosulfamide, cloquintocet-mexyl, mefenpyr-diethyl or isoxadifen-ethyl.

9. Herbicidal compositions according to any of Claims 4 to 8, comprising a further herbicide.

10. Method of controlling unwanted plants, **characterized in that** an effective amount of at least one compound of the general formula (I) according to any of Claims 1 to 3 or of a herbicidal composition according to any of Claims 4 to 9 is applied to the plants or to the site of the unwanted vegetation.

11. Use of compounds of the general formula (I) according to any of Claims 1 to 3 or of herbicidal compositions according to any of Claims 4 to 9 for controlling unwanted plants.

12. Use according to Claim 11, **characterized in that** the compounds of the general formula (I) are used for controlling unwanted plants in crops of useful plants.

13. Use according to Claim 12, **characterized in that** the useful plants are transgenic useful plants.

## Revendications

1. Pyridinyloxybenzènes substitués de formule générale (I) ou leurs sels 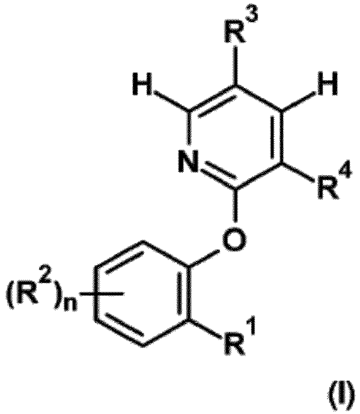 dans lesquels
R¹ représente une pyridine ou pyrimidine éventuellement substituée qui est éventuellement substituée par jusqu'à 4 substituants indépendamment les uns des autres choisis dans le groupe R⁵,
R² représente indépendamment les uns des autres halogène, hydroxy, amino, cyano, nitro, formyle, formamide, (C₁-C₄) -alkyle, (C₁-C₄)-halogénoalkyle, (C₃-C₆)-cycloalkyle, (C₂-C₄)-alcényle, (C₂-C₄)-alcynyle, (C₂-C₄)-halogéno-alcényle, (C₂-C₄)-halogénoalcynyle, (C₁-C₄)-alcoxy-(C₁-C₄)-alkyle, (C₁-C₄)-halogénoalcoxy-(C₁-C₄)-alkyle, (C₁-C₄)-alkylthio-(C₁-C₄)-alkyle, (C₁-C₄)-alkylsulfinyl-(C₁-C₄)-alkyle, (C₁-C₄)-alkylsulfonyl-(C₁-C₄)-alkyle, (C₁-C₄)-alkylcarbonyle, (C₁-C₄)-halogénoalkylcarbonyle, (C₃-C₆)-cycloalkylcarbonyle, carboxyle, (C₁-C₄)-alcoxycarbonyle, (C₁-C₄)-halogénoalcoxycarbonyle, (C₃-C₆)-cycloalcoxycarbonyle, (C₁-C₄)-alkylaminocarbonyle, (C₂-C₆)-dialkylaminocarbonyle, (C₃-C₆)-cycloalkylaminocarbonyle, (C₁-C₄)-alkylcarbonylamino, (C₁-C₄)-halogénoalkylcarbonylamino, (C₂-C₆)-cycloalkylcarbonylamino, (C₁-C₄)-alcoxycarbonylamino, (C₁-C₄)-alkylaminocarbonylamino, (C₂-C₆)-dialkylaminocarbonylamino, carboxy-(C₁-C₄)-alkyle, (C₁-C₄)-alcoxycarbonyl-(C₁-C₄)-alkyle, (C₁-C₄)-halogénoalcoxycarbonyl-(C₁-C₄)-alkyle, (C₃-C₆)-cycloalcoxycarbonyl-(C₁-C₄)-alkyle, (C₁-C₄) -alcoxy, (C₁-C₄)-halogénoalcoxy, (C₃-C₆)-cycloalcoxy, (C₁-C₄) -alcényloxy, (C₁-C₄)-alcynyloxy, (C₁-C₄)-alkylthio, (C₁-C₄)-halogénoalkylthio, (C₃-C₆)-cycloalkylthio, (C₁-C₄)-alkylsulfinyle, (C₁-C₄)-halogénoalkylsulfinyle, (C₃-C₆) -cycloalkylsulfinyle, (C₁-C₄)-alkylsulfonyle, (C₁-C₄)-halogénoalkylsulfonyle, (C₃-C₆) -cycloalkylsulfonyle, (C₁-C₄)-alkylaminosulfonyle, (C₂-C₆)-dialkylaminosulfonyle ou (C₃-C₆)-trialkylsilyle,
n étant 0, 1, 2, 3 ou 4,
R³ représente hydrogène, halogène, cyano, (C₁-C₄)-alkyle ou (C₁-C₄)-halogénoalkyle,
R⁴ représente hydrogène ou halogène,
et R⁵ représente hydrogène, halogène, hydroxy, amino, cyano, formyle, (C₁-C₄)-alkyle, (C₁-C₄)-halogénoalkyle, (C₁-C₄) -alcoxy, (C₁-C₄)-halogénoalcoxy, (C₁-C₄)-alcoxycarbonyle ou (C₁-C₄)-alcoxythiocarbonyle,
à l'exception de la 2-chloro-4-(2-pyridin-2-yloxyphényl)pyridine.

2. Composé de formule générale (I) selon la revendication 1 ou ses sels, dans lequel
R¹ représente une pyridine ou pyrimidine éventuellement substituée qui est éventuellement substituée par jusqu'à 4 substituants indépendamment les uns des autres choisis dans le groupe R⁵,
R² représente indépendamment les uns des autres halogène, hydroxy, amino, cyano, nitro, formyle, (C₁-C₄) -alkyle, (C₁-C₄) -halogéno-alkyle, (C₃-C₆)-cycloalkyle, carboxyle, (C₁-C₄)-alcoxycarbonyle, (C₁-C₄)-halogénoalcoxy-carbonyle, (C₁-C₄)-alkylaminocarbonyle, (C₂-C₆)-dialkylaminocarbonyle, (C₁-C₄) -alkyl-carbonylamino, carboxy-(C₁-C₄)-alkyle, (C₁-C₄)-alcoxycarbonyl-(C₁-C₄) -alkyle, (C₁-C₄) -alcoxy, (C₁-C₄)-halogénoalcoxy, (C₁-C₄) -alcényloxy, (C₁-C₄)-alcynyloxy ou (C₁-C₄) -alkylthio,
n étant 0, 1, 2 ou 3,
R³ représente hydrogène, halogène, cyano, (C₁-C₄)-alkyle ou (C₁-C₄)-halogénoalkyle,
R⁴ représente hydrogène ou halogène,
et R⁵ représente hydrogène, halogène, formyle, (C₁-C₄)-alkyle, (C₁-C₄) -halogénoalkyle, (C₁-C₄) -alcoxy, (C₁-C₄)-halogénoalcoxy, (C₁-C₄)-alcoxycarbonyle ou (C₁-C₄)-alcoxythiocarbonyle,
à l'exception de la 2-chloro-4-(2-pyridin-2-yloxyphényl)pyridine.

3. Composé de formule générale (I) selon la revendication 1 ou leurs sels, dans lequel
R¹ représente une 2-pyridine, 3-pyridine, 4-pyridine ou 4-pyrimidine éventuellement substituée qui est éventuellement substituée par jusqu'à 3 substituants indépendamment les uns des autres choisis parmi le groupe R⁵,
R² représente indépendamment les uns des autres fluor, chlore, brome, amino, hydroxy, nitro, cyano, carboxyle, méthyle, éthyle, cyclopropyle, trifluorométhyle, méthoxy, éthoxy, allyloxy, but-2-ynoxy ou méthylthio,
n étant 0, 1 ou 2,
R³ représente hydrogène, fluor, chlore, brome, cyano, méthyle, éthyle ou trifluorométhyle,
R⁴ représente hydrogène, fluor ou chlore,
et R⁵ représente hydrogène, chlore, formyle, méthyle, difluorométhyle, trifluorométhyle, chloro-fluorométhyle, chlorodifluorométhyle, méthoxy-carbonyle ou méthoxythiocarbonyle,
à l'exception de la 2-chloro-4-(2-pyridin-2-yloxyphényl)pyridine.

4. Agent herbicide **caractérisé par** une teneur efficace herbicide d'au moins un composé de formule générale (I) selon l'une quelconque des revendications 1 à 3.

5. Agent herbicide selon la revendication 4 en mélange avec des auxiliaires de formulation.

6. Agent herbicide selon la revendication 4 ou 5 contenant au moins une substance efficace pesticide supplémentaire du groupe des insecticides, des acaricides, des herbicides, des fongicides, des phytoprotecteurs et des régulateurs de croissance.

7. Agent herbicide selon la revendication 6 contenant un phytoprotecteur.

8. Agent herbicide selon la revendication 7 contenant cyprosulfamide, cloquintocet-mexyl, méfépyrdiéthyle ou isoxadifène-éthyle.

9. Agent herbicide selon l'une quelconque des revendications 4 à 8 contenant un herbicide supplémentaire.

10. Procédé de lutte contre des végétaux indésirables, **caractérisé en ce qu'**on applique une quantité efficace d'au moins un composé de formule générale (I) selon l'une quelconque des revendications 1 à 3 ou d'un agent herbicide selon l'une quelconque des revendications 4 à 9 sur les végétaux ou sur le lieu de la croissance végétale indésirable.

11. Utilisation de composés de formule générale (I) selon l'une quelconque des revendications 1 à 3 ou d'agents herbicides selon l'une quelconque des revendications 4 à 9 pour la lutte contre des végétaux indésirables.

12. Utilisation selon la revendication 11, **caractérisée en ce que** les composés de formule générale (I) sont utilisés dans la lutte contre des végétaux indésirables dans des cultures de végétaux utiles.

13. Utilisation selon la revendication 12, **caractérisée en ce que** les végétaux utiles sont des végétaux utiles transgéniques.
